# EUROPEAN PATENT SPECIFICATION

(11) **EP 1 487 928 B1**
(45) Date of publication and mention of the grant of the patent: **13.08.2008**
(21) Application number: 03745039.2
(22) Date of filing: 20.03.2003
(51) Int. Cl.: C09D 5/16, C09D 133/06, C08F 20/10

(54) **FILM-FORMING POLYMER AND ANTIFOULING PAINT**
FILMBILDENDES POLYMER UND BEWUCHSVERHINDERNDER ANSTRICHSTOFF
POLYMERE FILMOGENE ET PEINTURE ANTISALISSURE

(30) Priority: 26.03.2002 NO 20021491
(43) Date of publication of application: 22.12.2004
(73) Proprietor: Jotun A/S, 3248 Sandefjord (NO)
(72) Inventor: BÖRVE, Anita, N-3280 Tjodalyng (NO); DAHLING, Marit, N-3214 Sandefjord (NO); JOHANSEN, Kristin, N-3213 Sandefjord (NO)
(74) Representative: Hayes, Adrian Chetwynd
(86) International application number: PCT/NO2003/000095
(87) International publication number: WO 2003/080747

(56) References cited:
- EP-A1- 0 775 733
- EP-A1- 1 238 980
- EP-A1- 1 295 888
- EP-A1- 1 310 530
- EP-A2- 1 016 681
- WO-A1-96/41842
- WO-A1-03/027124
- DATABASE WPI Week 198842, Derwent Publications Ltd., London, GB; Class A14, AN 1988-296030, XP002148444 & JP 63 215 780 A (DAINIPPON INK & CHEM KK) 08 September 1988
- 'Group-transfer alternating copolymerization of 2-phenyl-1,3,2-dioxaphosphorinane with trimethylsilyl 3-(acryloyloxy) propionate' MACROMOLECULES vol. 24, 1991, AMERICAN CHEMICAL SOCIETY, pages 4475 - 4478, XP002966572
- DATABASE WPI Week 199632, Derwent Publications Ltd., London, GB; Class A14, AN 1989-209999, XP002966573 & JP 1 146 808 A (TOSHIBA SILICONE KK) 08 June 1989
- DATABASE WPI Week 198338, Derwent Publications Ltd., London, GB; Class A14, AN 1983-767142, XP002966574 & JP 58 047 066 A (NIPPON OILS & FATS CO LTD) 18 March 1983

## Description

Surfaces that are submerged in seawater are subjected to fouling by marine organisms such as green algae, brown algae, barnacles, mussel, tube worms and the like. On marine constructions such as vessels, oil platforms, buoys etc. this fouling may result in increased load and accelerated corrosion. On vessels the fouling will result in reduced manoeuvrability and will also increase the friction which will cause a reduction of the speed and increased fuel consumption.

To prevent attachment and growth of such marine organisms coatings known as antifouling paints are used. These paints usually consist of a film-forming binder with or without biological activity, together with different components such as pigments, fillers, solvents and biologically active substances.

Of the antifouling types of paint that have been used in the recent years, self-polishing antifouling paints containing organotin polymers have been extensively used. This type of paint has e.g. been described in GB1457590 and GB2152947. The success with the self-polishing triorganotin-containing paints is due to the fact that the organotin polymer on the paint surface hydrolyses in seawater to release triorganotin compounds which are very effective biologically active compounds. The remaining polymer on the surface becomes hydrophilic and thereby erodable so that it becomes self-polishing. This self-polishing effect provides controlled release of the biologically active compound and contributes to provide a smooth surface, which results in reduced friction between vessel (marine construction) and water.

In the recent years focus has been put on the environmental effects connected with the use of triorganotin compounds. It has been found that the organotin compounds that are released during the hydrolysis, in particular tributyltin, are slowly degraded, and this will result in an accumulation in the sediments in localised areas. In addition it has been found that some organisms have an extreme sensitivity to these compounds, and this has resulted in several non-lethal effects such as imposex in the snail *Nucella lapillus* and increased thickness of oyster shell such as e.g. with *Crassostrea gigas.*

In view of this increased restrictions have been imposed on the use of these biocidal products, and they will shortly be completely excluded. The International Maritime Organisation, IMO, leads this process, and a total ban against the use of these paints has been planned to enter into force from 1 January 2003.

As a consequence of this several tin-free antifouling systems have been developed and introduced. These paints have e.g. been described in WO8402915, EP646630, EP802243, W09606870 which describe self-polishing antifouling paints where organosilyl groups or organic acids attached to metal atoms have been incorporated in the polymer used instead of incorporating organotin groups. A characteristic feature for these self-polishing tin-free antifouling systems is that they also consist of polymers having hydrolysable groups securing the controlled dissolution of the polymers and thereby the release of antifouling compounds.

In our W09641841 and W09641842 we have described polymers of acrylic acid or methacrylic acid esters, that hydrolyse in contact with seawater and thereby provide a self-polishing system. However, experience has shown that these systems often do not exhibit the desired hydrolysis rate necessary to obtain sufficient polishing and antifouling effect. Monomers that are very similar to the monomers according to the above WO 9641842, are used in EP 0841380 A1 together with tin-containing monomers to prepare polymers for use as binder in marine paint. Our further work has shown that certain modifications in the acrylic acid or methacrylic acid structure will provide polymer systems having a rapid hydrolysis and thereby improved selfpolishing properties.

W09641842 and EP 0841380 A1 describe generally monomers having a certain similarity with the monomers used according to the present invention, but the monomers described specifically in said two applications are considerably different from the essential monomers used according to the present invention. In addition EP 0841380 A1, as mentioned above, relates to tin-containing polymers, while the polymers according to the present invention are tin-free.

Kobayashi S. et al., Macromolecules (1991), 24(15), 4475-4478 describe a group transfer copolymerisation process using trimethylsilyl 3-(acryloyloxy) propionate, which is a monomer of similar structure to the present invention. The polymerisation process proceeds via a trimethylsilyl group transfer process, which differs from the polymerisation processes described in the present invention. No specific use of the synthesised copolymers is mentioned.

Accordingly, the present invention relates to tin-free, hydrolysable polymers for use in antifouling coatings, and a process for the preparation of tin-free, polymerisable monomers which may be used for the preparation of binder systems for antifouling coatings to protect surfaces under water against the growth of marine organisms, the use of the polymers and the monomers, the preparation of the polymers and paint containing these, and constructions provided with antifouling coatings.

The present invention relates to a polymer for use in a paint forming an antifouling coating, and it is characterised in that it comprises units corresponding to the following monomers A and B:
A) one or more monomers of the general formula I 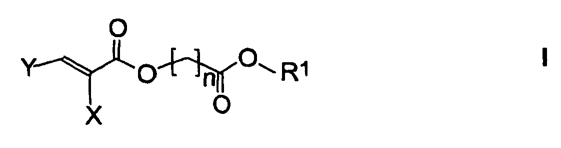 wherein
   n is an integer from 1 to 4,
   X is H or CH₃,
   Y is H or an optionally esterified COOH group,
   R¹ is Ph(R²)ₘ, -C(O)R³ or -Si(R⁴)₃,
   m is an integer from 1 to 3,
   R², which are the same or different, are selected from -C(O)H, C(O)R⁵, COOR⁶, CH₂COOR⁷, C₁₋₄ alkyl, C₁₋₄ alkanoyl, halogen, nitro, OH and OR⁸,
   R³ is selected from substituted or unsubstituted alkyl, aryl, aralkyl and heterocyclyl,
   R⁴, which are the same or different, are selected from substituted or unsubstituted C₁₋₂₀ alkyl, C₅₋₂₀ aryl, C₁₋₂₀ alkoxy and C₅₋₂₀ aryloxy, and
   R⁵, R⁶, R⁷ and R⁸ are independently selected from substituted or unsubstituted C₁₋₂₀ alkyl and C₅₋₂₀ aryl; and
B) other vinyl polymerisable monomers.
   The amount of A-units/A-monomers is normally 2-95 mole%, preferably 5-80 mole%, in particular 10-70 mole%. The amount of B-units/B-monomers is normally 5-98 mole%, preferably 20-95 mole%, in particular 30-90 mole%.
   The extended alkyl chain illustrated by -(CH₂)ₙ- in formula I, contributes to the fact that the hydrolysable group becomes more readily accessible for hydrolysis. The alkyl chain also contributes to an internal flexibilisation of the polymer.
   Another purpose of the invention is a process for the preparation of binders for use in antifouling paints, by polymerisation of polymerisable monomers, in which the process is characterised in that the polymerisable monomers that are polymerised, consist of one or more of component A and one or more of component B.
   Another purpose of the invention is a variation of the process for preparing binders for antifouling paints, in which polymerisable monomers in the form of mixtures of
C) 2 to 95 mole% of one or more compounds of the general formula II 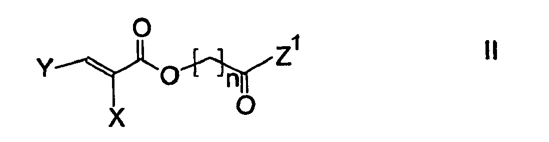 and
D) 5-98 mole% of other vinyl polymerisable monomers,
   are polymerised in step I in the presence of radical or ion-forming initiators, and in step II, in a polymer analogous reaction, the obtained copolymers are reacted with one or more compounds of the general formula III

   R¹-Z² III

   where
   n is an integer from 1 to 4,
   X is H or CH₃,
   Y is H or an optionally esterified COOH group,
   Z¹ is Cl or OH
   Z² is groups that are reactive with Z¹, such as OH or Cl,
   R¹ is Ph(R²)ₘ, -C(O)R³ or -Si(R⁴)₃,
   m is an integer from 1 to 3,
   R², that are the same or different, are selected from -C(O)H, C(O)R⁵, COOR⁶, CH₂COOR⁷, C₁₋₄ alkyl, C₁₋₄ alkanoyl, halogen, nitro, OH and OR⁸,
   R³ is selected from substituted or unsubstituted alkyl, aryl, aralkyl and heterocyclyl,
   R⁴, which are the same or different, are selected from substituted or unsubstituted C₁₋₂₀ alkyl, C₅₋₂₀ aryl, C₁₋₂₀ alkoxy and C₅₋₂₀ aryloxy, and
   R⁵, R⁶, R⁷ and R⁸ are independently selected from substituted or unsubstituted C₁₋₂₀ alkyl and C₅₋₂₀ aryl.
   Another purpose of the invention is a variation of the process for the preparation of binders for antifouling paints, in which polymerisable monomers in the form of mixtures of
E) 2 to 95 mole% of one or more compounds of the general formula IV 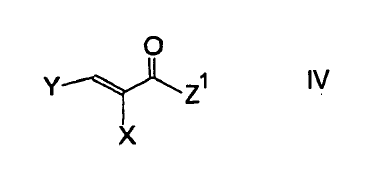 and
F) 5-98 mole-% of other vinyl polymerisable monomers,
   are polymerised in step I in the presence of radical-forming initiators, and in step II in a polymer analogous reaction, the obtained copolymers are reacted with one or more compounds of the general formula V 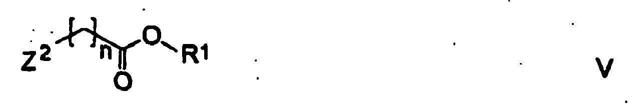
wherein
n is an integer from 1 to 4,
X is H or CH₃,
Y is H or an optionally esterified COOH group,
Z¹ is Cl or OH
Z² is a group that is reactive with Z¹, such as OH or Cl
R¹ is Ph(R²)ₘ,-C(O)R³ or -Si(R⁴)₃,
m is an integer from 1 to 3,
R², that are the same or different, are selected from
-C(O)H, C(O)R⁵, COOR⁶, CH₂COOR₇, C₁₋₄ alkyl, C₁₋₄ alkanoyl, halogen, nitro, OH and OR⁸, R³ is selected from substituted or unsubstituted alkyl, aryl and heterocyclyl, R⁴, that are the same or different, are selected from substituted or usubstituted C₁₋₂₀ alkyl, C₅₋₂₀ aryl, C₁₋₂₀ alkoxy and
C₅₋₂₀ aryloxy, and R⁵, R⁶, R⁷ and R⁸ are independently selected from substituted or unsubstituted C₁₋₂₀ alkyl and C₅₋₂₀ aryl.

The present invention further provides a monomer as defined in claim 19. Preferred features of the monomer are defined in claims 20 to 23.

The alkyl-and alkoxy groups mentioned above are preferably C₁₋₁₂, particularly C₁₋₈, and the aryl-and aryloxy groups are preferably C₅₋₁₂, in particular C₅₋₈.

Examples of monomers which may be used as component A for the preparation of binders for antifouling coatings containing phenyl ester groups defined by the structure I include:
formyl substituted phenyl esters of carboxymethyl acrylate and carboxymethyl methacrylate such as 3-formylphenoxycarbonylmethyl(meth)acrylate, 4-formylphenoxycarbonylmethyl(meth)acrylate, 6-fluoro-2-formylphenoxycarbonylmethyl(meth)acrylate, 2-formylphenoxycarbonylmethyl(meth)acrylate, 2-formyl-6-methoxyphenoxycarbonylmethyl(meth)acrylate, 6-ethoxy-2-formylphenoxycarbonylmethyl(meth)acrylate, 2-formyl-6-hydroxyphenoxycarbonylmethyl(meth)acrylate, 4-formyl-2-methylphenoxycarbonylmethyl (meth)acrylate, 3-chloro-4-formylphenoxycarbonylmethyl (meth)acrylate, 4-bromo-2-formylphenoxycarbonylmethyl (meth)acrylate, 2-formyl-4-methoxyphenoxycarbonylmethyl (meth)acrylate, 4-formyl-2-methoxyphenoxycarbonylmethyl (meth)acrylate, 2-formyl-4-(trifluoromethoxy) phenoxycarbonylmethyl (meth)acrylate, 4-formyl-2-nitrophenoxycarbonylmethyl (meth)acrylate, 4,6-dibromo-2-formyl-phenoxycarbonylmethyl (meth)acrylate, 2,5-dimethyl-4-formylphenoxycarbonylmethyl (meth)acrylate and 4-acetyl-2-formylphenoxycarbonylmethyl (meth)acrylate;
formyl substituted phenyl esters of carboxyethyl acrylate and carboxyethyl methacrylate such as 3-formylphenoxycarbonylethyl (meth)acrylate, 4-formylphenoxycarbonylethyl (meth)acrylate, 6-fluoro-2-formylphenoxycarbonylethyl (meth)acrylate, 2-formylphenoxycarbonylethyl (meth)acrylate, 2-formyl-6-methoxyphenoxycarbonylethyl (meth)acrylate, 6-ethoxy-2-formylphenoxycarbonylethyl (meth)acrylate, 2-formyl-6-hydroxyphenoxycarbonylethyl (meth)acrylate, 4-formyl-2-methylphenoxycarbonylethyl (meth)acrylate, 3-chloro-4-formylphenoxycarbonylethyl (meth)acrylate, 4-bromo-2-formylphenoxycarbonylethyl (meth)acrylate, 2-formyl-4-methoxyphenoxycarbonylethyl (meth)acrylate, 4-formyl-2-methoxyphenoxycarbonylethyl (meth)acrylate, 2-formyl-4-(trifluoromethoxy) phenoxycarbonylethyl (meth)acrylate, 4-formyl-2-nitrophenoxycarbonylethyl (meth)acrylate, 4,6-dibromo-2-formyl-phenoxycarbonylethyl (meth)acrylate, 2,5-dimethyl-4-formylphenoxycarbonylethyl (meth)acrylate and 4-acetyl-2-formylphenoxycarbonylethyl (meth)acrylate;
formyl substituted phenyl esters of carboxypropyl acrylate and carboxypropyl methacrylate such as 3-formylphenoxycarbonylpropyl (meth)acrylate, 4-formylphenoxycarbonylpropyl (meth)acrylate, 6-fluoro-2-formylphenoxycarbonylpropyl (meth)acrylate, 2-formylphenoxycarbonylpropyl (meth)acrylate, 2-formyl-6-methoxyphenoxycarbonylpropyl (meth)acrylate, 6-ethoxy-2-formylphenoxycarbonylpropyl (meth)acrylate, 2-formyl-6-hydroxyphenoxycarbonylpropyl (meth)acrylate, 4-formyl-2-methylphenoxycarbonylpropyl (meth)acrylate, 3-chloro-4-formylphenoxycarbonylpropyl (meth)acrylate, 4-bromo-2-formylphenoxycarbonylpropyl (meth)acrylate, 2-formyl-4-methoxyphenoxycarbonylpropyl (meth)acrylate, 4-formyl-2-methoxyphenoxycarbonylpropyl (meth)acrylate, 2-formyl-4-(trifluoromethoxy) phenoxycarbonylpropyl (meth)acrylate, 4-formyl-2-nitrophenoxycarbonylpropyl (meth)acrylate, 4,6-dibromo-2-formyl-phenoxycarbonylpropyl (meth)acrylate, 2,5-dimethyl-4-formylphenoxycarbonylpropyl (meth)acrylate and 4-acetyl-2-formylphenoxycarbonylpropyl (meth)acrylate;
formyl substituted phenyl esters of carboxybutyl acrylate and carboxybutyl methacrylate such as 3-formylphenoxycarbonylbutyl (meth)acrylate, 4-formylphenoxycarbonylbutyl (meth)acrylate, 6-fluoro-2-formylphenoxycarbonylbutyl (meth)acrylate, 2-formylphenoxycarbonylbutyl (meth)acrylate, 2-formyl-6-methoxyphenoxycarbonylbutyl (meth)acrylate, 6-ethoxy-2-formylphenoxycarbonylbutyl (meth)acrylate, 2-formyl-6-hydroxyphenoxycarbonylbutyl (meth)acrylate, 4-formyl-2-methylphenoxycarbonylbutyl (meth)acrylate, 3-chloro-4-formylphenoxycarbonylbutyl (meth)acrylate, 4-bromo-2-formylphenoxycarbonylbutyl (meth)acrylate, 2-formyl-4-methoxyphenoxycarbonylbutyl (meth)acrylate, 4-formyl-2-methoxyphenoxycarbonylbutyl (meth)acrylate, 2-formyl-4-(trifluoromethoxy) phenoxycarbonylbutyl (meth)acrylate, 4-formyl-2-nitrophenoxycarbonylbutyl (meth)acrylate, 4,6-dibromo-2-formyl-phenoxycarbonylbutyl (meth)acrylate, 2,5-dimethyl-4-formylphenoxycarbonylbutyl (meth)acrylate and 4-acetyl-2-formylphenoxycarbonylbutyl (meth)acrylate;
ester substituted phenyl esters of carboxymethyl acrylate and carboxymethyl methacrylate such as 4-methoxycarbonylphenoxycarbonylmethyl (meth)acrylate, 4-propoxycarbonylphenoxycarbonylmethyl (meth)acrylate, 4-isopropoxycarbonylphenoxycarbonylmethyl (meth)acrylate, 2-methoxycarbonylphenoxycarbonylmethyl (meth)acrylate, 4-methoxycarbonyl-2-nitrophenoxycarbonylmethyl (meth)acrylate, 4-ethoxycarbonyl-2-methoxyphenoxycarbonylmethyl (meth)acrylate, 5-ethoxycarbonyl-2-hydroxyphenoxycarbonylmethyl (meth)acrylate, 3-hydroxy-2 methoxycarbonylphenoxycarbonylmethyl (meth)acrylate, 2-(methoxycarbonylmethyl)phenoxycarbonylmethyl (meth)acrylate and 4-ethoxycarbonyl-2-nitroethylphenoxycarbonylmethyl (meth)acrylate;
ester substituted phenyl esters of carboxyethyl acrylate and carboxyethyl methacrylate such as 4-methoxycarbonylphenoxycarbonylethyl (meth)acrylate, 4-propoxycarbonylphenoxycarbonylethyl (meth)acrylate, 4-isopropoxycarbonylphenoxycarbonylethyl (meth)acrylate, 2-methoxycarbonylphenoxycarbonylethyl (meth)acrylate, 4-methoxycarbonyl-2-nitrophenoxycarbonylethyl (meth)acrylate, 4-ethoxycarbonyl-2-methoxyphenoxycarbonylethyl (meth)acrylate, 5-ethoxycarbonyl-2-hydroxyphenoxycarbonylethyl (meth)acrylate, 3-hydroxy-2 methoxycarbonylphenoxycarbonylethyl (meth)acrylate, 2-(methoxycarbonylmethyl)phenoxycarbonylethyl (meth)acrylate and 4-ethoxycarbonyl-2-nitroethylphenoxycarbonylethyl (meth)acrylate;
ester substituted phenyl esters of carboxypropyl acrylate and carboxypropyl methacrylate such as 4-methoxycarbonylphenoxycarbonylpropyl (meth)acrylate, 4-propoxycarbonylphenoxycarbonylpropyl (meth)acrylate, 4-isopropoxycarbonylphenoxycarbonylpropyl (meth)acrylate, 2-methoxycarbonylphenoxycarbonylpropyl (meth)acrylate, 4-methoxycarbonyl-2-nitrophenoxycarbonylpropyl (meth)acrylate, 4-ethoxycarbonyl-2-methoxyphenoxycarbonylpropyl (meth)acrylate, 5-ethoxycarbonyl-2-hydroxyphenoxycarbonylpropyl (meth)acrylate, 3-hydroxy-2 methoxycarbonylphenoxycarbonylpropyl (meth)acrylate, 2-(methoxycarbonylmethyl)phenoxycarbonylpropyl (meth)acrylate and 4-ethoxycarbonyl-2-nitroethylphenoxycarbonylpropyl (meth)acrylate;
ester substituted phenyl esters of carboxybutyl acrylate and carboxybutyl methacrylate such as 4-methoxycarbonylphenoxycarbonylbutyl (meth)acrylate, 4-propoxycarbonylphenoxycarbonylbutyl (meth)acrylate, 4-isopropoxycarbonylphenoxycarbonylbutyl (meth)acrylate, 2-methoxycarbonylphenoxycarbonylbutyl (meth)acrylate, 4-methoxycarbonyl-2-nitrophenoxycarbonylbutyl (meth)acrylate, 4-ethoxycarbonyl-2-methoxyphenoxycarbonylbutyl (meth)acrylate, 5-ethoxycarbonyl-2-hydroxyphenoxycarbonylbutyl (meth)acrylate, 3-hydroxy-2 methoxycarbonylphenoxycarbonylbutyl (meth)acrylate, 2-(methoxycarbonylmethyl)phenoxycarbonylbutyl (meth)acrylate and 4-ethoxycarbonyl-2-nitroethylphenoxycarbonylbutyl (meth)acrylate;
alkoxy substituted phenyl esters of carboxymethyl acrylate and carboxymethyl methacrylate such as 2-methoxyphenoxycarbonylmethyl (meth)acrylate, 4-methoxyphenoxycarbonylmethyl (meth)acrylate, 4-ethoxyphenoxycarbonylmethyl (meth)acrylate, 1,4-dimethoxyphenoxycarbonylmethyl (meth)acrylate, 2,4-dimethoxyphenoxycarbonylmethyl (meth)acrylate, 2-chloro-5-methoxyphenoxycarbonylmethyl (meth)acrylate, 3,5-diethoxyphenoxycarbonylbutyl (meth)acrylate and 2,5-dimethoxy-3-hydroxyphenoxycarbonylmethyl (meth)acrylate;
alkoxy substituted phenyl esters of carboxyethyl acrylate and carboxyethyl methacrylate such as 2-methoxyphenoxycarbonylethyl (meth)acrylate, 4-methoxyphenoxycarbonylethyl (meth)acrylate, 4-ethoxyphenoxycarbonylethyl (meth)acrylate, 1,4-dimethoxyphenoxycarbonylethyl (meth)acrylate, 2,4-dimethoxyphenoxycarbonylethyl (meth)acrylate, 2-chloro-5-methoxyphenoxycarbonylethyl (meth)acrylate, 3,5-diethoxyphenoxycarbonylethyl (meth)acrylate and 2,5-dimethoxy-3-hydroxyphenoxycarbonylethyl (meth)acrylate;
alkoxy substituted phenyl esters of carboxypropyl acrylate and carboxypropyl methacrylate such as 2-methoxyphenoxycarbonylpropyl (meth)acrylate, 4-methoxyphenoxycarbonylpropyl (meth)acrylate, 4-ethoxyphenoxycarbonylpropyl (meth)acrylate, 1,4-dimethoxyphenoxycarbonylpropyl (meth)acrylate, 2,4-dimethoxyphenoxycarbonylpropyl (meth)acrylate, 2-chloro-5-methoxyphenoxycarbonylpropyl (meth)acrylate, 3,5-diethoxyphenoxycarbonylpropyl (meth)acrylate and 2,5-dimethoxy-3-hydroxyphenoxycarbonylpropyl (meth)acrylate;
alkoxy substituted phenyl esters of carboxybutyl acrylate and carboxybutyl methacrylate such as 2-methoxyphenoxycarbonylbutyl (meth)acrylate, 4-methoxyphenoxycarbonylbutyl (meth)acrylate, 4-ethoxyphenoxycarbonylbutyl (meth)acrylate, 1,4-dimethoxyphenoxycarbonylbutyl (meth)acrylate, 2,4-dimethoxyphenoxycarbonylbutyl (meth)acrylate, 2-chloro-5-methoxyphenoxycarbonylbutyl (meth)acrylate, 3,5-diethoxyphenoxycarbonylbutyl (meth)acrylate and 2,5-dimethoxy-3-hydroxyphenoxycarbonylbutyl (meth)acrylate;
hydroxy substituted phenyl esters of carboxymethyl acrylate and carboxymethyl methacrylate such as 2-hydroxyphenoxycarbonylmethyl (meth)acrylate, 3-hydroxyphenoxycarbonylmethyl (meth)acrylate, 4-hydroxyphenoxycarbonylmethyl (meth)acrylate, 3,5-dihydroxyphenoxycarbonylmethyl (meth)acrylate;
hydroxy substituted phenyl esters of carboxyethyl acrylate and carboxyethyl methacrylate such as 2-hydroxyphenoxycarbonylethyl (meth)acrylate, 3-hydroxyphenoxycarbonylethyl (meth)acrylate, 4-hydroxyphenoxycarbonylethyl (meth)acrylate, 3,5-dihydroxyphenoxycarbonylethyl (meth)acrylate;
hydroxy substituted phenyl esters of carboxypropyl acrylate and carboxypropyl methacrylate such as 2-hydroxyphenoxycarbonylpropyl (meth)acrylate, 3-hydroxyphenoxycarbonylpropyl (meth)acrylate, 4-hydroxyphenoxycarbonylpropyl (meth)acrylate, 3,5-dihydroxyphenoxycarbonylpropyl (meth)acrylate;
hydroxy substituted phenyl esters of carboxybutyl acrylate and carboxybutyl methacrylate such as 2-hydroxyphenoxycarbonylbutyl (meth)acrylate, 3-hydroxyphenoxycarbonylbutyl (meth)acrylate, 4-hydroxyphenoxycarbonylbutyl (meth)acrylate, 3,5-dihydroxyphenoxycarbonylbutyl (meth)acrylate;
halogen substituted phenyl esters of carboxymethyl acrylate and carboxymethyl methacrylate such as 4-bromophenoxycarbonylmethyl (meth)acrylate, 2,4,6-tribromophenoxycarbonylmethyl (meth)acrylate, 2,4,6-trichlorophenoxycarbonylmethyl (meth)acrylate, 4-bromo-2,6-dichlorophenoxycarbonylmethyl (meth)acrylate, 2,4-dichloro-6-iodophenoxycarbonylmethyl (meth)acrylate, 2-chloro-4,6-dibromophenoxycarbonylmethyl (meth)acrylate;
halogen substituted phenyl esters of carboxyethyl acrylate and carboxyethyl methacrylate such as 4-bromophenoxycarbonylethyl (meth)acrylate, 2,4,6-tribromophenoxycarbonylethyl (meth)acrylate, 2,4,6-trichlorophenoxycarbonylethyl (meth)acrylate, 4-bromo-2,6-dichlorophenoxycarbonylethyl (meth)acrylate, 2,4-dichloro-6-iodophenoxycarbonylethyl (meth)acrylate, 2-chloro-4,6-dibromophenoxycarbonylethyl (meth)acrylate;
halogen substituted phenyl esters of carboxypropyl acrylate and carboxypropyl methacrylate such as 4-bromophenoxycarbonylpropyl (meth)acrylate, 2,4,6-tribromophenoxycarbonylpropyl (meth)acrylate, 2,4,6-trichlorophenoxycarbonylpropyl (meth)acrylate, 4-bromo-2,6-dichlorophenoxycarbonylpropyl (meth)acrylate, 2,4-dichloro-6-iodophenoxycarbonylpropyl (meth)acrylate, 2-chloro-4,6-dibromophenoxycarbonylpropyl (meth)acrylate;
halogen substituted phenyl esters of carboxybutyl acrylate and carboxybutyl methacrylate such as 4-bromophenoxycarbonylbutyl (meth)acrylate, 2,4,6-tribromophenoxycarbonylbutyl (meth)acrylate, 2,4,6-trichlorophenoxycarbonylbutyl (meth)acrylate, 4-bromo-2,6-dichlorophenoxycarbonylbutyl (meth)acrylate, 2,4-dichloro-6-iodophenoxycarbonylbutyl (meth)acrylate, 2-chloro-4,6-dibromophenoxycarbonylbutyl (meth)acrylate;
and other multisubstituted phenyl esters of carboxyethyl acrylate and carboxyethyl methacrylate;
derivatives of maleic acid esters such as 4-formylphenoxycarbonylmethyl methyl maleate, 2-formylphenoxycarbonylmethyl methyl maleate, 4-bromo-2-formylphenoxycarbonylmethyl methyl maleate, 4-bromo-3-formylphenoxycarbonylmethyl methyl maleate, 2-formyl-6-methoxyphenoxycarbonylmethyl methyl maleate, 4-formyl-6-methoxyphenoxycarbonylmethyl methyl maleate, 4-formylphenoxycarbonylpropyl methyl maleate, 2-formylphenoxycarbonylpropyl methyl maleate, 4-bromo-2-formylphenoxycarbonylpropyl methyl maleate, 4-bromo-3-formylphenoxycarbonylpropyl methyl maleate, 2-formyl-6-methoxyphenoxycarbonylpropyl methyl maleate, 4-formyl-6-methoxyphenoxycarbonylpropyl methyl maleate, 4-methoxycarbonylphenoxycarbonylmethyl methyl maleate, 4-methoxycarbonyl-2-nitrophenoxycarbonylmethyl methyl maleate, 2-methoxyphenoxycarbonylmethyl methyl maleate, 4-methoxyphenoxycarbonylmethyl methyl maleate, 4-ethoxyphenoxycarbonylmethyl methyl maleate, 4-methoxyphenoxycarbonylethyl methyl maleate, 2-methoxyphenoxycarbonylpropyl methyl maleate, 4-methoxyphenoxycarbonylpropyl methyl maleate, 2-hydroxyphenoxycarbonylmethyl methyl maleate, 3-hydroxyphenoxycarbonylmethyl methyl maleate, 4-hydroxyphenoxycarbonylmethyl methyl maleate, 3,5-dihydroxyphenoxycarbonylmethyl methyl maleate, 3-hydroxyphenoxycarbonylethyl methyl maleate, 4-hydroxyphenoxycarbonylpropyl methyl maleate, 3-hydroxyphenoxycarbonylpropyl methyl maleate, 3,5-dihydroxyphenoxycarbonylpropyl methyl maleate, 4-hydroxyphenoxycarbonylbutyl methyl maleate, 4-bromophenoxycarbonylmethyl methyl maleate, 2-4-6-tribromophenoxycarbonylmethyl methyl maleate, 2-4-6-trichlorophenoxycarbonylmethyl methyl maleate, 4-bromophenoxycarbonylethyl methyl maleate, 4-bromophenoxycarbonylpropyl methyl maleate, 2-formylphenoxycarbonylmethyl ethyl maleate, 4-bromo-2-formylphenoxycarbonylmethyl ethyl maleate, 2-formylphenoxycarbonylpropyl ethyl maleate, 4-bromo-3-formylphenoxycarbonylpropyl ethyl maleate, 4-methoxycarbonylphenoxycarbonylmethyl ethyl maleate, 4-methoxyphenoxycarbonylmethyl ethyl maleate, 4-methoxyphenoxycarbonylpropyl ethyl maleate, 3-hydroxyphenoxycarbonylmethyl ethyl maleate, 4-hydroxyphenoxycarbonylmethyl ethyl maleate, 3-hydroxyphenoxycarbonylpropyl ethyl maleate, 4-hydroxyphenoxycarbonylpropyl ethyl maleate, 3,5-dihydroxyphenoxycarbonylpropyl ethyl maleate, 4-hydroxyphenoxycarbonylbutyl ethyl maleate, 4-bromophenoxycarbonylmethyl ethyl maleate, 4-bromophenoxycarbonylmethyl ethyl maleate, 4-bromophenoxycarbonylethyl ethyl maleate, 4-bromophenoxycarbonylpropyl ethyl maleate;
derivatives of fumaric acid esters such as 4-formylphenoxycarbonylmethyl methyl fumarate, 2-formylphenoxycarbonylmethyl methyl fumarate, 4-bromo-2-formylphenoxycarbonylmethyl methyl fumarate, 4-bromo-3-formylphenoxycarbonylmethyl methyl fumarate, 2-formyl-6-methoxyphenoxycarbonylmethyl methyl fumarate, 4-formyl-6-methoxyphenoxycarbonylmethyl methyl fumarate, 4-formylphenoxycarbonylpropyl methyl fumarate, 2-formylphenoxycarbonylpropyl methyl fumarate, 4-bromo-2-formylphenoxycarbonylpropyl methyl fumarate, 4-bromo-3-formylphenoxycarbonylpropyl methyl fumarate, 2-formyl-6-methoxyphenoxycarbonylpropyl methyl fumarate, 4-formyl-6-methoxyphenoxycarbonylpropyl methyl fumarate, 4-methoxycarbonylphenoxycarbonylmethyl methyl fumarate, 4-methoxycarbonyl-2-nitrophenoxycarbonylmethyl methyl fumarate, 2-methoxyphenoxycarbonylmethyl methyl fumarate, 4-methoxyphenoxycarbonylmethyl methyl fumarate, 4-ethoxyphenoxycarbonylmethyl methyl fumarate, 4-methoxyphenoxycarbonylethyl methyl fumarate, 2-methoxyphenoxycarbonylpropyl methyl fumarate, 4-methoxyphenoxycarbonylpropyl methyl fumarate, 2-hydroxyphenoxycarbonylmethyl methyl fumarate, 3-hydroxyphenoxycarbonylmethyl methyl fumarate, 4-hydroxyphenoxycarbonylmethyl methyl fumarate, 3,5-dihydroxyphenoxycarbonylmethyl methyl fumarate, 3-hydroxyphenoxycarbonylethyl methyl fumarate, 4-hydroxyphenoxycarbonylpropyl methyl fumarate, 3-hydroxyphenoxycarbonylpropyl methyl fumarate, 3,5-dihydroxyphenoxycarbonylpropyl methyl fumarate, 4-hydroxyphenoxycarbonylbutyl methyl fumarate, 4-bromophenoxycarbonylmethyl methyl fumarate, 2-4-6-tribromophenoxycarbonylmethyl methyl fumarate, 2-4-6-trichlorophenoxycarbonylmethyl methyl fumarate, 4-bromophenoxycarbonylethyl methyl fumarate, 4-bromophenoxycarbonylpropyl methyl fumarate, 2-formylphenoxycarbonylmethyl ethyl fumarate, 4-bromo-2-formylphenoxycarbonylmethyl ethyl fumarate, 2-formylphenoxycarbonylpropyl ethyl fumarate, 4-bromo-3-formylphenoxycarbonylpropyl ethyl fumarate, 4-methoxycarbonylphenoxycarbonylmethyl ethyl fumarate, 4-methoxyphenoxycarbonylmethyl ethyl fumarate, 4-methoxyphenoxycarbonylpropyl ethyl fumarate, 3-hydroxyphenoxycarbonylmethyl ethyl fumarate, 4-hydroxyphenoxycarbonylmethyl ethyl fumarate, 3-hydroxyphenoxycarbonylpropyl ethyl fumarate, 4-hydroxyphenoxycarbonylpropyl ethyl fumarate, 3,5-dihydroxyphenoxycarbonylpropyl ethyl fumarate, 4-hydroxyphenoxycarbonylbutyl ethyl fumarate, 4-bromophenoxycarbonylmethyl ethyl fumarate, 4-bromophenoxycarbonylmethyl ethyl fumarate, 4-bromophenoxycarbonylethyl ethyl fumarate, 4-bromophenoxycarbonylpropyl ethyl fumarate;

Preferred examples of monomers which may be used as component A for the preparation of binders for antifouling coatings containing phenyl ester groups defined by the structure I include 4-formylphenoxycarbonylmethyl (meth)acrylate, 2-formylphenoxycarbonylmethyl (meth)acrylate, 4-bromo-2-formylphenoxycarbonylmethyl (meth)acrylate, 4-bromo-3-formylphenoxycarbonylmethyl (meth)acrylate, 2-formyl-6-methoxyphenoxycarbonylmethyl (meth)acrylate, 4-formyl-6-methoxyphenoxycarbonylmethyl (meth)acrylate, 4-formylphenoxycarbonylpropyl (meth)acrylate, 2-formylphenoxycarbonylpropyl (meth)acrylate, 4-bromo-2-formylphenoxycarbonylpropyl (meth)acrylate, 4-bromo-3-formylphenoxycarbonylpropyl (meth)acrylate, 2-formyl-6-methoxyphenoxycarbonylpropyl (meth)acrylate, 4-formyl-6-methoxyphenoxycarbonylpropyl (meth)acrylate, 4-methoxycarbonylphenoxycarbonylmethyl (meth)acrylate, 4-methoxycarbonyl-2-nitrophenoxycarbonylmethyl (meth)acrylate, 2-methoxyphenoxycarbonylmethyl (meth)acrylate, 4-methoxyphenoxycarbonylmethyl (meth)acrylate, 4-ethoxyphenoxycarbonylmethyl (meth)acrylate, 4-methoxyphenoxycarbonylethyl (meth)acrylate, 2-methoxyphenoxycarbonylpropyl (meth)acrylate, 4-methoxyphenoxycarbonylpropyl (meth)acrylate, 2-hydroxyphenoxycarbonylmethyl (meth)acrylate, 3-hydroxyphenoxycarbonylmethyl (meth)acrylate, 4-hydroxyphenoxycarbonylmethyl (meth)acrylate, 3,5-dihydroxyphenoxycarbonylmethyl (meth)acrylate, 3-hydroxyphenoxycarbonylethyl (meth)acrylate, 4-hydroxyphenoxycarbonylpropyl (meth)acrylate, 3-hydroxyphenoxycarbonylpropyl (meth)acrylate, 3,5-dihydroxyphenoxycarbonylpropyl (meth)acrylate, 4-hydroxyphenoxycarbonylbutyl (meth)acrylate, 4-bromophenoxycarbonylmethyl (meth)acrylate, 2-4-6-tribromophenoxycarbonylmethyl (meth)acrylate, 2-4-6-trichlorophenoxycarbonylmethyl (meth)acrylate, 4-bromophenoxycarbonylethyl (meth)acrylate and 4-bromophenoxycarbonylpropyl (meth)acrylate.

Particularly preferred examples of monomers which may be used as component A for the preparation of binders for antifouling coatings containing phenyl ester groups defined by the structure I include 2-formylphenoxycarbonylmethyl (meth)acrylate, 4-bromo-2-formylphenoxycarbonylmethyl (meth)acrylate, 2-formylphenoxycarbonylpropyl (meth)acrylate, 4-bromo-3-formylphenoxycarbonylpropyl (meth)acrylate, 4-methoxycarbonylphenoxycarbonylmethyl (meth)acrylate, 4-methoxyphenoxycarbonylmethyl (meth)acrylate, 4-methoxyphenoxycarbonylpropyl (meth)acrylate, 3-hydroxyphenoxycarbonylmethyl (meth)acrylate, 4-hydroxyphenoxycarbonylmethyl (meth)acrylate, 3-hydroxyphenoxycarbonylpropyl (meth)acrylate, 4-hydroxyphenoxycarbonylpropyl (meth)acrylate, 3,5-dihydroxyphenoxycarbonylpropyl (meth)acrylate, 4-hydroxyphenoxycarbonylbutyl (meth)acrylate, 4-bromophenoxycarbonylmethyl (meth)acrylate, 4-bromophenoxycarbonylmethyl (meth)acrylate, 4-bromophenoxycarbonylethyl (meth)acrylate and 4-bromophenoxycarbonylpropyl (meth)acrylate.

Examples of monomers which may be used as component A for the preparation of binders for antifouling coatings containing anhydride groups defined by the structure I include:
non-aromatic anhydrides of carboxymethyl acrylate and carboxymethyl methacrylate such as trimethylacetoxycarbonylmethyl (meth)acrylate, methoxyacetoxycarbonylmethyl (meth)acrylate, *tert*-butylacetoxycarbonylmethyl (meth)acrylate, acetoxyacetylcarbonylmethyl (meth)acrylate, bromoacetoxycarbonylmethyl (meth)acrylate, chloroacetoxycarbonylmethyl (meth)acrylate, cyclopentylacetoxycarbonylmethyl (meth)acrylate, dichloroacetoxycarbonylmethyl (meth)acrylate, tribromoacetoxycarbonylmethyl (meth)acrylate, trichloroacetoxycarbonylmethyl (meth)acrylate and trifluoroacetoxycarbonylmethyl (meth)acrylate;
non-aromatic anhydrides of carboxyethyl acrylate and carboxyethyl methacrylate such as trimethylacetoxycarbonylethyl (meth)acrylate, methoxyacetoxycarbonylethyl (meth)acrylate, *tert*-butylacetoxycarbonylethyl (meth)acrylate, acetoxyacetylcarbonylethyl (meth)acrylate, bromoacetoxycarbonylethyl (meth)acrylate, chloroacetoxycarbonylethyl (meth)acrylate, cyclopentylacetoxycarbonylethyl (meth)acrylate, dichloroacetoxycarbonylethyl (meth)acrylate, tribromoacetoxycarbonylethyl (meth)acrylate, trichloroacetoxycarbonylethyl (meth)acrylate and trifluoroacetoxycarbonylethyl (meth)acrylate;
non-aromatic anhydrides of carboxypropyl acrylate and carboxypropyl methacrylate such as trimethylacetoxycarbonylpropyl (meth)acrylate, methoxyacetoxycarbonylpropyl (meth)acrylate, *tert*-butylacetoxycarbonylpropyl (meth)acrylate, acetoxyacetylcarbonylpropyl (meth)acrylate, bromoacetoxycarbonylpropyl (meth)acrylate, chloroacetoxycarbonylpropyl (meth)acrylate, cyclopentylacetoxycarbonylpropyl (meth)acrylate, dichloroacetoxycarbonylpropyl (meth)acrylate, tribromoacetoxycarbonylpropyl (meth)acrylate, trichloroacetoxycarbonylpropyl (meth)acrylate and trifluoroacetoxycarbonylpropyl (meth)acrylate;
non-aromatic anhydrides of carboxybutyl acrylate and carboxybutyl methacrylate such as trimethylacetoxycarbonylbutyl (meth)acrylate, methoxyacetoxycarbonylbutyl (meth)acrylate, *tert*-butylacetoxycarbonylbutyl (meth)acrylate, acetoxyacetylcarbonylbutyl (meth)acrylate, bromoacetoxycarbonylbutyl (meth)acrylate, chloroacetoxycarbonylbutyl (meth)acrylate, cyclopentylacetoxycarbonylbutyl (meth)acrylate, dichloroacetoxycarbonylbutyl (meth)acrylate, tribromoacetoxycarbonylbutyl (meth)acrylate, trichloroacetoxycarbonylbutyl (meth)acrylate and trifluoroacetoxycarbonylbutyl (meth)acrylate;
aromatic anhydrides of carboxymethyl acrylate and carboxymethyl methacrylate such as phenylacetoxycarbonylmethyl (meth)acrylate, 2,2-diphenylacetoxycarbonylmethyl (meth)acrylate, 2,2,2-triphenylacetoxycarbonylmethyl (meth)acrylate, 4-methoxyphenylacetoxycarbonylmethyl (meth)acrylate, 3,4-dimethoxyphenylacetoxycarbonylmethyl (meth)acrylate, 2,5-dimethoxyphenylacetoxycarbonylmethyl (meth)acrylate, 2-bromophenylacetoxycarbonylmethyl (meth)acrylate, 2-bromo-4,5-dimethoxyphenylacetoxycarbonylmethyl (meth)acrylate, 4-chlorophenylacetoxycarbonylmethyl (meth)acrylate, 2-chloro-2-phenylacetoxycarbonylmethyl (meth)acrylate, 2-chloro-2,2-diphenylacetoxycarbonylmethyl (meth)acrylate, 4-fluorophenylacetoxycarbonylmethyl (meth)acrylate, 4-methoxyphenyl acetoxycarbonylmethyl(meth)acrylate, phenoxyacetoxycarbonylmethyl (meth)acrylate, 4-fluorophenoxyacetoxycarbonylmethyl (meth)acrylate, benzyloxyacetoxycarbonylmethyl (meth)acrylate, 2-chloro-2-phenyl acetoxycarbonylmethyl(meth)acrylate, 2,2-diphenyl -2-chloroacetoxycarbonylmethyl(meth)acrylate, 2-(2,4,5-trichlorophenoxy)-acetoxycarbonylmethyl (meth)acrylate, benzylacetoxycarbonylmethyl (meth)acrylate, benzoyloxycarbonylmethyl (meth)acrylate, and nicotinoyloxycarbonylmethyl (meth)acrylate;
aromatic anhydrides of carboxyethyl acrylate and carboxyethyl methacrylate such as phenylacetoxycarbonylethyl (meth)acrylate, 2,2-diphenylacetoxycarbonylethyl (meth)acrylate, 2,2,2-triphenylacetoxycarbonylethyl (meth)acrylate, 4-methoxyphenylacetoxycarbonylethyl (meth)acrylate, 3,4-dimethoxyphenylacetoxycarbonylethyl (meth)acrylate, 2,5-dimethoxyphenylacetoxycarbonylethyl (meth)acrylate, 2-bromophenylacetoxycarbonylethyl (meth)acrylate, 2-bromo-4,5-dimethoxyphenylacetoxycarbonylethyl (meth)acrylate, 4-chlorophenylacetoxycarbonylethyl (meth)acrylate, 2-chloro-2-phenylacetoxycarbonylethyl (meth)acrylate, 2-chloro-2,2-diphenylacetoxycarbonylethyl (meth)acrylate, 4-fluorophenylacetoxycarbonylethyl (meth)acrylate, 4-methoxyphenyl acetoxycarbonylethyl(meth)acrylate, phenoxyacetoxycarbonylethyl (meth)acrylate, 4-fluorophenoxyacetoxycarbonylethyl (meth)acrylate, benzyloxyacetoxycarbonylethyl (meth)acrylate, 2-phenyl -2-chloroacetoxycarbonylethyl(meth)acrylate, 2-chloro-2,2-diphenyl acetoxycarbonylethyl(meth)acrylate, 2-(2,4,5-trichlorophenoxy) acetoxycarbonylethyl (meth)acrylate, benzylacetoxycarbonylethyl (meth)acrylate, benzoyloxycarbonylethyl (meth)acrylate, and nicotinoyloxycarbonylethyl (meth)acrylate;
aromatic anhydrides of carboxypropyl acrylate and carboxypropyl methacrylate such as phenylacetoxycarbonylpropyl (meth)acrylate, 2,2-diphenylacetoxycarbonylpropyl (meth)acrylate, 2,2,2-triphenylacetoxycarbonylpropyl (meth)acrylate, 4-methoxyphenylacetoxycarbonylpropyl (meth)acrylate, 3,4-dimethoxyphenylacetoxycarbonylpropyl (meth)acrylate, 2,5-dimethoxyphenylacetoxycarbonylpropyl (meth)acrylate, 2-bromophenylacetoxycarbonylpropyl (meth)acrylate, 2-bromo-4,5-dimethoxyphenylacetoxycarbonylpropyl (meth)acrylate, 4-chlorophenylacetoxycarbonylpropyl (meth)acrylate, 2-chloro-2-phenyl acetoxycarbonylpropyl (meth)acrylate, 2-chloro-2,2-diphenylacetoxycarbonylpropyl (meth)acrylate, 4-fluorophenylacetoxycarbonylpropyl (meth)acrylate, 4-methoxyphenyl acetoxycarbonylpropyl(meth)acrylate, phenoxyacetoxycarbonylpropyl (meth)acrylate, 4-fluorophenoxyacetoxycarbonylpropyl (meth)acrylate, benzyloxyacetoxycarbonylpropyl (meth)acrylate, 2-phenyl -2-chloroacetoxycarbonylpropyl(meth)acrylate, 2-chloro-2,2-diphenyl acetoxycarbonylpropyl(meth)acrylate, 2-(2,4,5-trichlorophenoxy)-acetoxycarbonylpropyl (meth)acrylate, benzylacetoxycarbonylpropyl (meth)acrylate, benzoyloxycarbonylpropyl (meth)acrylate, and nicotinoyloxycarbonylpropyl (meth)acrylate;
aromatic anhydrides of carboxybutyl acrylate and carboxybutyl methacrylate such as phenylacetoxycarbonylbutyl (meth)acrylate, 2,2-diphenylacetoxycarbonylbutyl (meth)acrylate, 2,2,2-triphenylacetoxycarbonylbutyl (meth)acrylate, 4-methoxyphenylacetoxycarbonylbutyl (meth)acrylate, 3,4-dimethoxyphenylacetoxycarbonylbutyl (meth)acrylate, 2,5-dimethoxyphenylacetoxycarbonylbutyl (meth)acrylate, 2-bromophenylacetoxycarbonylbutyl (meth)acrylate, 2-bromo-4,5-dimethoxyphenylacetoxycarbonylbutyl (meth)acrylate, 4-chlorophenylacetoxycarbonylbutyl (meth)acrylate, 2-chloro-2-phenylacetoxycarbonylbutyl (meth)acrylate, 2-chloro-2,2-diphenylacetoxycarbonylbutyl (meth)acrylate, 4-fluorophenylacetoxycarbonylbutyl (meth)acrylate, 4-methoxyphenyl acetoxycarbonylbutyl(meth)acrylate, phenoxyacetoxycarbonylbutyl (meth)acrylate, 4-fluorophenoxyacetoxycarbonylbutyl (meth)acrylate, benzyloxyacetoxycarbonylbutyl (meth)acrylate, 2-chloro-2-phenyl acetoxycarbonylbutyl(meth)acrylate, 2,2-diphenyl -2-chloroacetoxycarbonylbutyl(meth)acrylate, 2-(2,4,5-trichlorophenoxy)acetoxycarbonylbutyl (meth)acrylate, benzylacetoxycarbonylbutyl (meth)acrylate, benzoyloxycarbonylbutyl (meth)acrylate, and nicotinoyloxycarbonylbutyl (meth)acrylate;
derivatives of maleic acid esters such as trimethylacetoxycarbonylmethyl methyl maleate, *tert*-butylacetoxycarbonylmethyl methyl maleate, cyclopentylacetoxycarbonylmethyl methyl maleate, bromoacetoxycarbonylmethyl methyl maleate, trimethylacetoxycarbonylethyl methyl maleate, *tert-*butylacetoxycarbonylethyl methyl maleate, cyclopentylacetoxycarbonylethyl methyl maleate, bromoacetoxycarbonylethyl methyl maleate, trimethylacetoxycarbonylbutyl methyl maleate, *tert*-butylacetoxycarbonylbutyl methyl maleate, methoxyacetoxycarbonylpropyl methyl maleate, phenylacetoxycarbonylmethyl methyl maleate, 2,2-diphenylacetoxycarbonylmethyl methyl maleate, 4-methoxyphenylacetoxycarbonylmethyl methyl maleate, 2-bromophenylacetoxycarbonylbutyl methyl maleate, 2-chloro-2,2-diphenylacetoxycarbonylmethyl methyl maleate, phenylacetoxycarbonylethyl methyl maleate, 2,2-diphenylacetoxycarbonylethyl methyl maleate, 4-methoxyphenylacetoxycarbonylethyl methyl maleate, phenylacetoxycarbonylpropyl methyl maleate, 2,2-diphenylacetoxycarbonylpropyl methyl maleate, phenylacetoxycarbonylbutyl methyl maleate, trimethylacetoxycarbonylmethyl ethyl maleate, *tert*-butylacetoxycarbonylmethyl ethyl maleate, cyclopentylacetoxycarbonylmethyl ethyl maleate, bromoacetoxycarbonylmethyl ethyl maleate, trimethylacetoxycarbonylethyl ethyl maleate, *tert*-butylacetoxycarbonylethyl ethyl maleate, cyclopentylacetoxycarbonylethyl ethyl maleate, bromoacetoxycarbonylethyl ethyl maleate, trimethylacetoxycarbonylbutyl ethyl maleate, *tert-*butylacetoxycarbonylbutyl ethyl maleate, methoxyacetoxycarbonylpropyl ethyl maleate, phenylacetoxycarbonylmethyl ethyl maleate, 2,2-diphenylacetoxycarbonylmethyl ethyl maleate, 4-methoxyphenylacetoxycarbonylmethyl ethyl maleate, 2-bromophenylacetoxycarbonylbutyl ethyl maleate, 2-chloro-2,2-diphenylacetoxycarbonylmethyl ethyl maleate, phenylacetoxycarbonylethyl ethyl maleate, 2,2-diphenylacetoxycarbonylethyl ethyl maleate, 4-methoxyphenylacetoxycarbonylethyl ethyl maleate, phenylacetoxycarbonylpropyl ethyl maleate, 2,2-diphenylacetoxycarbonylpropyl ethyl maleate, phenylacetoxycarbonylbutyl ethyl maleate, benzylacetoxycarbonylmethyl ethyl maleate, benzylacetoxycarbonylethyl ethyl maleate and benzylacetoxycarbonylpropyl methyl maleate;
derivatives of fumaric acid esters such as trimethylacetoxycarbonylmethyl methyl fumarate, *tert*-butylacetoxycarbonylmethyl methyl fumarate, cyclopentylacetoxycarbonylmethyl methyl fumarate, bromoacetoxycarbonylmethyl methyl fumarate, trimethylacetoxycarbonylethyl methyl fumarate, *tert-*butylacetoxycarbonylethyl methyl fumarate, cyclopentylacetoxycarbonylethyl methyl fumarate, bromoacetoxycarbonylethyl methyl fumarate, trimethylacetoxycarbonylbutyl methyl fumarate, *tert*-butylacetoxycarbonylbutyl methyl fumarate, methoxyacetoxycarbonylpropyl methyl fumarate, phenylacetoxycarbonylmethyl methyl fumarate, 2,2-diphenylacetoxycarbonylmethyl methyl fumarate, 4-methoxyphenylacetoxycarbonylmethyl methyl fumarate, 2-bromophenylacetoxycarbonylbutyl methyl fumarate, 2-chloro-2, 2-diphenylacetoxycarbonylmethyl methyl fumarate, phenylacetoxycarbonylethyl methyl fumarate, 2,2-diphenylacetoxycarbonylethyl methyl fumarate, 4-methoxyphenylacetoxycarbonylethyl methyl fumarate, phenylacetoxycarbonylpropyl methyl fumarate, 2,2-diphenylacetoxycarbonylpropyl methyl fumarate, phenylacetoxycarbonylbutyl methyl fumarate, trimethylacetoxycarbonylmethyl ethyl fumarate, *tert-*butylacetoxycarbonylmethyl ethyl fumarate, cyclopentylacetoxycarbonylmethyl ethyl fumarate, bromoacetoxycarbonylmethyl ethyl fumarate, trimethylacetoxycarbonylethyl ethyl fumarate, *tert*-butylacetoxycarbonylethyl ethyl fumarate, cyclopentylacetoxycarbonylethyl ethyl fumarate, bromoacetoxycarbonylethyl ethyl fumarate, trimethylacetoxycarbonylbutyl ethyl fumarate, *tert*-butylacetoxycarbonylbutyl ethyl fumarate, methoxyacetoxycarbonylpropyl ethyl fumarate, phenylacetoxycarbonylmethyl ethyl fumarate, 2,2-diphenylacetoxycarbonylmethyl ethyl fumarate, 4-methoxyphenylacetoxycarbonylmethyl ethyl fumarate, 2-bromophenylacetoxycarbonylbutyl ethyl fumarate, 2-chloro-2,2-diphenylacetoxycarbonylmethyl ethyl fumarate, phenylacetoxycarbonylethyl ethyl fumarate, 2,2-diphenylacetoxycarbonylethyl ethyl fumarate, 4-methoxyphenylacetoxycarbonylethyl ethyl fumarate, phenylacetoxycarbonylpropyl ethyl fumarate, 2,2-diphenylacetoxycarbonylpropyl ethyl fumarate, benzylacetoxycarbonylmethyl ethyl fumarate, benzylacetoxycarbonylethyl ethyl fumarate, benzylacetoxycarbonylpropyl methyl fumarate and phenylacetoxycarbonylbutyl ethyl fumarate.

Preferred examples of monomers which may be used as component A for the preparation of binders for antifouling coatings containing anhydride-groups defined by the structure I include trimethylacetoxycarbonylmethyl (meth)acrylate, *tert*-butylacetoxycarbonylmethyl (meth)acrylate, cyclopentylacetoxycarbonylmethyl (meth)acrylate, bromoacetoxycarbonylmethyl (meth)acrylate, trimethylacetoxycarbonylethyl (meth)acrylate, *tert-*butylacetoxycarbonylethyl (meth)acrylate, cyclopentylacetoxycarbonylethyl (meth)acrylate, bromoacetoxycarbonylethyl (meth)acrylate, trimethylacetoxycarbonylbutyl (meth)acrylate, *tert*-butylacetoxycarbonylbutyl (meth)acrylate, methoxyacetoxycarbonylpropyl (meth)acrylate, phenylacetoxycarbonylmethyl (meth)acrylate, benzylacetoxycarbonylmethyl (meth)acrylate, benzylacetoxycarbonylethyl ethyl maleate and benzylacetoxycarbonylpropylmethylmaleate;2,2-diphenylacetoxycarbonylmethyl (meth)acrylate, 4-methoxyphenylacetoxycarbonylmethyl (meth)acrylate, 2-bromophenylacetoxycarbonylbutyl (meth)acrylate, 2-chloro-2,2-diphenylacetoxycarbonylmethyl (meth)acrylate, phenylacetoxycarbonylethyl (meth)acrylate, 2,2-diphenylacetoxycarbonylethyl (meth)acrylate, 4-methoxyphenylacetoxycarbonylethyl (meth)acrylate, benzylacetoxycarbonylethyl (meth)acrylate, phenylacetoxycarbonylpropyl (meth)acrylate, 2,2-diphenylacetoxycarbonylpropyl (meth)acrylate, benzylacetoxycarbonylbutyl (meth)acrylate and phenylacetoxycarbonylbutyl (meth)acrylate.

Particularly preferred examples of monomers which may be used as component A for the preparation of binders for antifouling coatings containing anhydride groups defined by the structure I include trimethylacetoxycarbonylmethyl (meth)acrylate, *tert*-butylacetoxycarbonylmethyl (meth)acrylate, trimethylacetoxycarbonylethyl (meth)acrylate, *tert-*butylacetoxycarbonylethyl (meth)acrylate, trimethylacetoxycarbonylbutyl (meth)acrylate, *tert*-butylacetoxycarbonylbutyl (meth)acrylate, benzylacetoxycarbonylmethyl (meth)acrylate, phenylacetoxycarbonylmethyl (meth)acrylate, 2,2-diphenylacetoxycarbonylmethyl (meth)acrylate, 4-methoxyphenylacetoxycarbonylmethyl (meth)acrylate, phenylacetoxycarbonylethyl (meth)acrylate, 2,2-diphenylacetoxycarbonylethyl (meth)acrylate, 4-methoxyphenylacetoxycarbonylethyl (meth)acrylate, phenylacetoxycarbonylpropyl (meth)acrylate and benzylacetoxycarbonylpropyl (meth)acrylate.

For monomers that may be used as component A for the preparation of binders for antifouling coatings containing the organosilyl group consisting of -Si(R₄)₃ defined in I, the substituents R₄ may be the same or different, with alkyl groups having up to 20 C atoms, preferably up to 12 C atoms, in particular up to 8 C atoms, such as methyl, ethyl, propyl, butyl, pentyl, hexyl, heptyl, octyl, nonyl, decyl, isopropyl, *tert*-butyl, isobutyl, *sec*-butyl, cyclohexyl and substituted cyclohexyl, aryl groups having up to 20 C atoms, preferably up to 12 C atoms, such as aryl and naphthyl and substituted aryl groups in which at least one substituent is selected from the group of fluoro, chloro, bromo, iodo, nitro, cyano, amino, hydroxy, etc.

Examples of triorganosilyl groups -Si(R₄)₃, are trimethylsilyl, triethylsilyl, tri-n-propylsilyl, triisopropylsilyl, tri-n-butylsilyl, tri-*tert*-butylsilyl, triisobutylsilyl, tri-s-butylsilyl, tri-n-pentylsilyl, tri-n-hexylsilyl, triphenylsilyl, tribenzylsilyl, ethyldimethylsilyl, n-butyldimethylsilyl, diisopropyl-n-butylsilyl, dicyclohexylphenyl silyl, t-butyldiphenylsilyl, t-butyldimethylsilyl, dimethylphenylsilyl, biphenyldimethylsilyl, biphenyldiisopropylsilyl, diphenylmethylsilyl, dimethyloctadecylsilyl, dimethyloctylsilyl, dimethylcyclohexylsilyl, dimethylhexylsilyl, chloromethyldimethylsilyl and pentafluorophenyldimethylsilyl.

Examples of monomers which may be used as component A for the preparation of binders for antifouling coatings containing organosilyl ester groups defined by the structure I include:
silyl esters of carboxymethyl acrylate and carboxymethyl methacrylate such as trimethylsilyloxycarbonylmethyl (meth)acrylate, triethylsilyloxycarbonylmethyl (meth)acrylate, tri-n-propylsilyloxycarbonylmethyl (meth)acrylate, triisopropylsilyloxycarbonylmethyl (meth)acrylate, tri-n-butylsilyloxycarbonylmethyl (meth)acrylate, triisobutylsilyloxycarbonylmethyl (meth)acrylate, tri-*tert-*butylsilyloxycarbonylmethyl (meth)acrylate, tri-s-butylsilyloxycarbonylmethyl (meth)acrylate, tri-n-pentylsilyloxycarbonylmethyl (meth)acrylate, triisopentylsilyloxycarbonylmethyl (meth)acrylate, tri-n-hexylsilyloxycarbonylmethyl (meth)acrylate, tri-n-octylsilyloxycarbonylmethyl (meth)acrylate, triphenylsilyloxycarbonylmethyl (meth)acrylate, tri-p-methylphenylsilyloxycarbonylmethyl (meth)acrylate, tribenzylsilyloxycarbonylmethyl (meth)acrylate, ethyldimethylsilyloxycarbonylmethyl (meth)acrylate, *n-*butyldimethylsilyloxycarbonylmethyl (meth)acrylate, diisopropylsilyloxycarbonylmethyl (meth)acrylate and t-butyldiphenylsilyloxycarbonylmethyl (meth)acrylate;
silyl esters of carboxyethyl acrylate and carboxyethyl methacrylate such as trimethylsilyloxycarbonylethyl (meth)acrylate, triethylsilyloxycarbonylethyl (meth)acrylate, tri-n-propylsilyloxycarbonylethyl (meth)acrylate, triisopropylsilyloxycarbonylethyl (meth)acrylate, tri-n-butylsilyloxycarbonylethyl (meth)acrylate, triisobutylsilyloxycarbonylethyl (meth)acrylate, tri-*tert-*butylsilyloxycarbonylethyl (meth)acrylate, tri-s-butylsilyloxycarbonylethyl (meth)acrylate, tri-n-pentylsilyloxycarbonylethyl (meth)acrylate, triisopentylsilyloxycarbonylethyl (meth)acrylate, tri-n-hexylsilyloxycarbonylethyl (meth)acrylate, *tri-n*-octylsilyloxycarbonylethyl (*meth*)acrylate, triphenylsilyloxycarbonylethyl (meth)acrylate, tri-p-methylphenylsilyloxycarbonylethyl (meth)acrylate, tribenzylsilyloxycarbonylethyl (meth)acrylate, ethyldimethylsilyloxycarbonylethyl (meth)acrylate, *n-*butyldimethylsilyloxycarbonylethyl (meth)acrylate, diisopropylsilyloxycarbonylethyl (meth)acrylate and t-butyldiphenylsilyloxycarbonylethyl (meth)acrylate;
silyl esters of carboxypropyl acrylate and carboxypropyl methacrylate such as trimethylsilyloxycarbonylpropyl (meth)acrylate, triethylsilyloxycarbonylpropyl (meth)acrylate, tri-n-propylsilyloxycarbonylpropyl (meth)acrylate, triisopropylsilyloxycarbonylpropyl (meth)acrylate, tri-n-butylsilyloxycarbonylpropyl (meth)acrylate, triisobutylsilyloxycarbonylpropyl (meth)acrylate, tri-*tert-*butylsilyloxycarbonylpropyl (meth)acrylate, tri-s-butylsilyloxycarbonylpropyl (meth)acrylate, tri-n-pentylsilyloxycarbonylpropyl (meth)acrylate, triisopentylsilyloxycarbonylpropyl (meth)acrylate, tri-n-hexylsilyloxycarbonylpropyl (meth)acrylate, tri-n-octylsilyloxycarbonylpropyl (meth)acrylate, triphenylsilyloxycarbonylpropyl (meth)acrylate, tri-p-methylphenylsilyloxycarbonylpropyl (meth)acrylate, tribenzylsilyloxycarbonylpropyl (meth)acrylate, ethyldimethylsilyloxycarbonylpropyl (meth)acrylate, *n-*butyldimethylsilyloxycarbonylpropyl (meth)acrylate, diisopropylsilyloxycarbonylpropyl (meth)acrylate and t-butyldiphenylsilyloxycarbonylpropyl (meth)acrylate;
silyl esters of carboxybutyl acrylate and carboxybutyl methacrylate such as trimethylsilyloxycarbonylbutyl (meth)acrylate, triethylsilyloxycarbonylbutyl (meth)acrylate, tri-n-propylsilyloxycarbonylbutyl (meth)acrylate, triisopropylsilyloxycarbonylbutyl (meth)acrylate, tri-n-butylsilyloxycarbonylbutyl (meth)acrylate, triisobutylsilyloxycarbonylbutyl (meth)acrylate, tri-*tert-*butylsilyloxycarbonylbutyl (meth)acrylate, tri-s-butylsilyloxycarbonylbutyl (meth)acrylate, tri-n-pentylsilyloxycarbonylbutyl (meth)acrylate, triisopentylsilyloxycarbonylbutyl (meth)acrylate, tri-n-hexylsilyloxycarbonylbutyl (meth)acrylate, tri-n-octylsilyloxycarbonylbutyl (meth)acrylate, triphenylsilyloxycarbonylbutyl (meth)acrylate, tri-p-methylphenylsilyloxycarbonylbutyl (meth)acrylate, tribenzylsilyloxycarbonylbutyl (meth)acrylate, ethyldimethylsilyloxycarbonylbutyl (meth)acrylate, *n-*butyldimethylsilyloxycarbonylbutyl (meth)acrylate, diisopropylsilyloxycarbonylbutyl (meth)acrylate and t-butyldiphenylsilyloxycarbonylbutyl (meth)acrylate;
silyl esters of carboxymethyl maleic acid esters such as trimethylsilyloxycarbonylmethyl methyl maleate, triethylsilyloxycarbonylmethyl ethyl maleate, tri-n-propylsilyloxycarbonylmethyl n-propyl maleate, triisopropylsilyloxycarbonylmethyl methyl maleate, tri-n-butylsilyloxycarbonylmethyl n-butyl maleate and tri-n-hexylsilyloxycarbonylmethyl n-hexyl maleate;
silyl esters of carboxyethyl maleic acid esters such as trimethylsilyloxycarbonylethyl methyl maleate, triethylsilyloxycarbonylethyl ethyl maleate, tri-n-propylsilyloxycarbonylethyl n-propyl maleate, triisopropylsilyloxycarbonylethyl methyl maleate, tri-n-butylsilyloxycarbonylethyl n-butyl maleate and tri-n-hexylsilyloxycarbonylethyl n-hexyl maleate;
silyl esters of carboxypropyl maleic acid esters such as trimethylsilyloxycarbonylpropyl methyl maleate, triethylsilyloxycarbonylpropyl ethyl maleate, tri-n-propylsilyloxycarbonylpropyl n-propyl maleate, triisopropylsilyloxycarbonylpropyl methyl maleate, tri-n-butylsilyloxycarbonylpropyl n-butyl maleate and tri-n-hexylsilyloxycarbonylpropyl n-hexyl maleate;
silyl esters of carboxybutyl maleic acid esters such as trimethylsilyloxycarbonylbutyl methyl maleate, triethylsilyloxycarbonylbutyl ethyl maleate, tri-n-propylsilyloxycarbonylbutyl n-propyl maleate, triisopropylsilyloxycarbonylbutyl methyl maleate, tri-n-butylsilyloxycarbonylbutyl n-butyl maleate and tri-n-hexylsilyloxycarbonylbutyl n-hexyl maleate;
silyl esters of carboxymethyl fumaric acid esters such as trimethylsilyloxycarbonylmethyl methyl fumarate, triethylsilyloxycarbonylmethyl ethyl fumarate, tri-n-propylsilyloxycarbonylmethyl n-propyl fumarate, triisopropylsilyloxycarbonylmethyl methyl fumarate, tri-n-butylsilyloxycarbonylmethyl n-butyl fumarate and tri-n-hexylsilyloxycarbonylmethyl n-hexyl fumarate;
silyl esters of carboxyethyl fumaric acid esters such as trimethylsilyloxycarbonylethyl methyl fumarate, triethylsilyloxycarbonylethyl ethyl fumarate, tri-n-propylsilyloxycarbonylethyl n-propyl fumarate, triisopropylsilyloxycarbonylethyl methyl fumarate, tri-n-butylsilyloxycarbonylethyl n-butyl fumarate and tri-n-hexylsilyloxycarbonylethyl n-hexyl fumarate;
silyl esters of carboxypropyl fumaric acid esters such as trimethylsilyloxycarbonylpropyl methyl fumarate, triethylsilyloxycarbonylpropyl ethyl fumarate, tri-n-propylsilyloxycarbonylpropyl n-propyl fumarate, triisopropylsilyloxycarbonylpropyl methyl fumarate, tri-n-butylsilyloxycarbonylpropyl n-butyl fumarate and tri-n-hexylsilyloxycarbonylpropyl n-hexyl fumarate;
silyl esters of carboxybutyl fumaric acid esters such as trimethylsilyloxycarbonylbutyl methyl fumarate, triethylsilyloxycarbonylbutyl ethyl fumarate, tri-n-propylsilyloxycarbonylbutyl n-propyl fumarate, triisopropylsilyloxycarbonylbutyl methyl fumarate, tri-n-butylsilyloxycarbonylbutyl n-butyl fumarate and tri-n-hexylsilyloxycarbonylbutyl n-hexyl fumarate;

In the present invention at least one monomer A, C or E, defined by the structure I, II or IV, is copolymerised with one or more other vinyl polymerisable monomers B, D or F.

Examples of such vinyl polymerisable monomers B, D and F are acrylic acid and esters of acrylic acid such as methyl acrylate, ethyl acrylate, n-propyl acrylate, isopropyl acrylate, n-butyl acrylate, isobutyl acrylate, *tert*-butyl acrylate, butylethyl acrylate, pentyl acrylate, hexyl acrylate, cyclohexyl acrylate, phenyl acrylate, n-octyl acrylate, isooctyl acrylate, 2-ethylhexyl acrylate, 3,5,5-trimethylhexyl acrylate, lauryl acrylate, cetyl acrylate, stearyl acrylate, 2-hydroxyethyl acrylate, 2-hydroxypropyl acrylate, 4-hydroxybutyl acrylate, 2-hydroxy-3-phenoxypropyl acrylate, 2-methoxyethyl acrylate, methoxypropyl acrylate, 2-ethoxyethyl acrylate, ethoxypropyl acrylate, propoxyethyl acrylate, butoxyethyl acrylate, isobutoxyethyl acrylate, phenoxyethyl acrylate;
methacrylic acid and esters of methacrylic acid such as methyl methacrylate, ethyl methacrylate, propyl methacrylate, isopropyl methacrylate, n-butyl methacrylate, iso-butyl methacrylate, *tert*-butyl methacrylate, pentyl methacrylate, hexyl methacrylate, cyclohexyl methacrylate, phenyl methacrylate, benzyl methacrylate, tridecyl methacrylate, lauryl methacrylate, stearyl methacrylate, 2-ethylhexyl methacrylate, 2-hydroxyethyl methacrylate, 2-hydroxypropyl methacrylate, methoxyethyl methacrylate, 2-ethoxyethyl methacrylate, ethoxypropyl methacrylate, propoxyethyl methacrylate, 2-butoxyethyl methacrylate, isobutoxyethyl methacrylate, isobutoxybutyl diglycol methacrylate, phenoxyethyl methacrylate;
amides such as acrylamide, methacrylamide, N-methyl acrylamide, N-ethyl acrylamide, N-propyl acrylamide, maleimide;
aminofunctional acrylates and methacrylates such as 2-aminoethyl (meth)acrylate, dimethylaminoethyl (meth)acrylate, 2-diethylaminoethyl (meth)acrylate, 3-aminopropyl (meth)acrylate, 2-butylaminoethyl (meth)acrylate, *tert*-butylaminoethyl (meth)acrylate;
N-vinylamides such as N-vinylformamide , N-vinylethylamide , N-vinylpropylamide and others as described in WO0162811;
N-vinyllactams such as N-vinylpyrrolidone, N-vinylpiperidine, N-vinylcaprolactam and others as described in EP1127902;
heterocyclic substituted acrylates and methacrylates such as furfuryl (meth)acrylate and tetrahydrofurfuryl (meth)acrylate;
heterocyclic substituted vinyl monomers such as vinylimidazole, 2-vinylpyridine, 4-vinylpyridine, 1-vinyl-1,2,4-triazole, 2-vinyl-1,3-dioxolane;
functional carbonyloxyethyl (meth)acrylates such as 2-(methacryloyloxy)ethyl acetoacetate, 2-(((butylamino)carbonyl)oxy)ethyl acrylate;
aliphatic and heterocyclic substituted acrylates and methacrylates such as 1-*iso*butoxyethyl (meth)acrylate, 1-ethoxyethyl (meth)acrylate, 1-propoxyethyl (meth)acrylate, 1-(2-ethylhexyloxy)ethyl (meth)acrylate, 2-tetrahydrofuranyl (meth)acrylate, 2-tetrahydropyranyl (meth)acrylate and others as described in JP4103671;
multifunctional acrylates and methacrylates such as tetra(ethylene glycol) diacrylate, tri(propylene glycol) diacrylate, 1,3-butanediol diacrylate, 1,4-butanediol diacrylate, 1,3-butanediol dimethacrylate, 1,4-butanediol dimethacrylate, hexanediol diacrylate, ethylene glycol dimethacrylate, trimethylolpropane triacrylate, trimethylolpropane trimethacrylate, di(trimethylolpropane) tetraacrylate, pentaerythritol triacrylate, pentaerythritol tetraacrylate, dipentaerythritol pentaacrylate, vinyl acrylate.

Examples of compounds which may be used as component C for the preparation of binders for antifouling paint according to the invention, include:
acrylates and methacrylates such as carboxymethyl (meth)acrylate, carboxyethyl (meth)acrylate, carboxypropyl (meth)acrylate, carboxybutyl (meth)acrylate;
acid chlorides such as carboxylic acid substituted methyl esters of (meth)acrylic acid, 2-carboxylic acid chloride substituted ethyl esters of (meth)acrylic acid, 3-carboxylic acid chloride substituted propyl esters of (meth)acrylic acid and 4-carboxylic acid chloride substituted butyl esters of (meth)acrylic acid.

Examples of components of the general formula III reactive with component C include:
phenyl substituted aromatic alcohols such as 3-formylphenol, 4-formylphenol, 6-fluoro-2-formylphenol, 2-formylphenol, 2-formyl-6-methoxyphenol, 6-ethoxy-2-formylphenol, 2-formyl-6-hydroxyphenol, 4-formyl-2-methylphenol, 3-chloro-4-formylphenol, 4-bromo-2-formylphenol, 2-formyl-4-methoxyphenol, 4-formyl-2-methoxyphenol, 2-formyl-4 -(trifluoromethoxy)phenol, 4-formyl-2-nitrophenol, 4,6-dibromo-2-formylphenol and 2,5-dimethyl-4-formylphenol;
ester substituted aromatic alcohols such as 4-methoxycarbonylphenol, 4-propoxycarbonylphenol, 4-isopropoxycarbonylphenol, 2-methoxycarbonylphenol, 4-methoxycarbonyl-2-nitrophenol, 4-ethoxycarbonyl-2-methoxyphenol, 5-ethoxycarbonyl-2-hydroxyphenol, 3-hydroxy-2-methoxycarbonylphenol, 2-methoxycarbonylmethylphenol and 4-ethoxycarbonylethyl-2-nitrophenol;
alkoxy substituted aromatic alcohols such as 2-methoxyphenol, 4-methoxyphenol, 4-ethoxyphenol, 1,4-dimethoxyphenol, 2,4-dimethoxyphenol, 2-chloro-5-methoxyphenol, 3,5-diethoxyphenol and 3-hydroxy-2,5-dimethoxyphenol;
hydroxy substituted aromatic alcohols such as 2-hydroxyphenol, 3-hydroxyphenol, 4-hydroxyphenol, 3,5-dihydroxyphenol;
halogen substituted aromatic alcohols such as 4-bromophenol, 2,4,6-tribromophenol, 2,4,6-trichlorophenol, 4-bromo-2,6-dichlorophenol, 2,4-dichloro-6-iodophenol, 2-chloro-4,6-dibromophenol;
aromatic acetyl chlorides such as benzoyl chloride, phenylacetyl chloride, 2,2-diphenylacetyl chloride, 2,2,2-triphenylacetyl chloride, 4-methoxyphenylacetyl chloride, 3,4-dimethoxyphenylacetyl chloride, 2,5-dimethoxyphenylacetyl chloride, 2-bromophenylacetyl chloride, 2-bromo-4,5-dimethoxyphenylacetyl chloride, 4-chlorophenylacetyl chloride, 2-chloro-2-phenylacetyl chloride, 2-chloro-2,2-diphenylacetyl chloride, 4-fluorophenylacetyl chloride, 4-methoxyphenyl acetylchloride, phenoxyacetylcarbonyl chloride, 4-fluorophenoxyacetyl chloride, benzyloxyacetyl chloride 2-phenyl -2-chloroacetylchloride, 2,2-diphenyl -2-chloroacetylchloride and 2-(2,4,5-trichlorophenoxy)acetyl chloride;
aliphatic acetyl chlorides such as trimethylacetyl chloride, methoxyacetyl chloride, *tert*-butylacetyl chloride, acetoxyacetyl chloride, bromoacetyl chloride, chloroacetyl chloride, cyclopentylacetyl chloride, dichloroacetyl chloride, tribromoacetyl chloride, trichloroacetyl chloride and trifluoroacetyl chloride;
aromatic or aliphatic silyl chlorides such as trimethylsilyl chloride, triethylsilyl chloride, tri-n-propylsilyl chloride, triisopropylsilyl chloride, tri-n-butylsilyl chloride, tri-*tert*-butylsilyl chloride, triisobutylsilyl chloride, tri-s-butylsilyl chloride, tri-n-pentylsilyl chloride, tri-n-hexylsilyl chloride, triphenylsilyl chloride, tribenzylsilyl chloride, ethyldimethylsilyl chloride, n-butyldimethylsilyl chloride, diisopropyl-n-butylsilyl chloride, dicyclohexylphenylsilyl chloride, t-butyldiphenylsilyl chloride, t-butyldimethylsilyl chloride, dimethylphenylsilyl chloride, biphenyldimethylsilyl chloride, biphenyldiisopropylsilyl chloride, diphenylmethylsilyl chloride, dimethyloctadecylsilyl chloride, dimethyloctylsilyl chloride, dimethylcyclohexylsilyl chloride, dimethylhexylsilyl chloride, chloromethyldimethylsilyl chloride and pentafluorophenyldimethylsilyl chloride.

Examples of compounds which may be used as component E for the preparation of binders for antifouling paint according to the invention, include acrylic acid, methacrylic acid, acryloyl chloride and methacryloyl chloride.

Examples of components of the general formula V reactive with component E include:
formyl substituted methyl alcohol compounds such as 3-formylphenoxycarbonylmethyl alcohol, 4-formylphenoxycarbonylmethyl alcohol, 2-formylphenoxycarbonylmethyl alcohol, 6-fluoro-2-formylphenoxycarbonylmethyl alcohol, 2-formyl-6-methoxyphenoxycarbonylmethyl alcohol, 6-ethoxy-2-formylphenoxycarbonylmethyl alcohol, 2-formyl-6-hydroxyphenoxycarbonylmethyl alcohol, 4-formyl-2-methylphenoxycarbonylmethyl alcohol, 3-chloro-4-formylphenoxycarbonylmethyl alcohol, 4-bromo-2-formylphenoxycarbonylmethyl alcohol, 2-formyl-4-methoxyphenoxycarbonylmethyl alcohol, 4-formyl-2-methoxyphenoxycarbonylmethyl alcohol, 2-formyl-4-(trifluoromethoxy) phenoxycarbonylmethyl alcohol, 4-formyl-2-nitrophenoxycarbonylmethyl alcohol, 4,6-dibromo-2-formylphenoxycarbonylmethyl alcohol and 2,5-dimethyl-4-formylphenoxycarbonylmethyl alcohol;
formyl substituted ethyl alcohol compounds such as 2-(3-formylphenoxycarbonyl)ethyl alcohol, 2-(4-formylphenoxycarbonyl)ethyl alcohol, 2-(2-formylphenoxycarbonyl)ethyl alcohol, 2-(6-fluoro-2-formylphenoxycarbonyl)ethyl alcohol, 2-(2-formyl-6-methoxyphenoxycarbonyl)ethyl alcohol, 2-(6-ethoxy-2-formylphenoxycarbonyl)ethyl alcohol, 2-(2-formyl-6-hydroxyphenoxycarbonyl)ethyl alcohol, 2-(4-formyl-2-methylphenoxycarbonyl)ethyl alcohol, 2-(3-chloro-4-formylphenoxycarbonyl)ethyl alcohol, 2-(4-bromo-2-formylphenoxycarbonyl)ethyl alcohol, 2-(2-formyl-4-methoxyphenoxycarbonyl)ethyl alcohol, 2-(4-formyl-2-methoxyphenoxycarbonyl)ethyl alcohol, 2-(2-formyl-4-(trifluoromethoxy) phenoxycarbonyl)ethyl alcohol, 2-(4-formyl-2-nitrophenoxycarbonyl)ethyl alcohol, 2-(4,6-dibromo-2-formylphenoxycarbonyl)ethyl alcohol and 2-(2,5-dimethyl-4-formylphenoxycarbonyl)ethyl alcohol;
formyl substituted propyl alcohol compounds such as 3-(3-formylphenoxycarbonyl)propyl alcohol, 3-(4-formylphenoxycarbonyl)propyl alcohol, 3-(2-formylphenoxycarbonyl)propyl alcohol, 3-(6-fluoro-2-formylphenoxycarbonyl)propyl alcohol, 3-(2-formyl-6-methoxyphenoxycarbonyl)propyl alcohol, 3-(6-ethoxy-2-formylphenoxycarbonyl)propyl alcohol, 3-(2-formyl-6-hydroxyphenoxycarbonyl)propyl alcohol, 3-(4-formyl-2-methylphenoxycarbonyl)propyl alcohol, 3-(3-chloro-4-formylphenoxycarbonyl)propyl alcohol, 3-(4-bromo-2-formylphenoxycarbonyl)propyl alcohol, 3-(2-formyl-4-methoxyphenoxycarbonyl)propyl alcohol, 3-(4-formyl-2-methoxyphenoxycarbonyl)propyl alcohol, 3-(2-formyl-4-(trifluoromethoxy) phenoxycarbonyl)propyl alcohol, 3-(4-formyl-2-nitrophenoxycarbonyl)propyl alcohol, 3-(4,6-dibromo-2-formylphenoxycarbonyl)propyl alcohol and 3-(2,5-dimethyl-4-formylphenoxycarbonyl)propyl alcohol;
formyl substituted butyl alcohol compounds such as 4-(3-formylphenoxycarbonyl)butyl alcohol, 4-(4-formylphenoxycarbonyl)butyl alcohol, 4-(2-formylphenoxycarbonyl)butyl alcohol, 4-(6-fluoro-2-formylphenoxycarbonyl)butyl alcohol, 4-(2-formyl-6-methoxyphenoxycarbonyl)butyl alcohol, 4-(6-ethoxy-2-formylphenoxycarbonyl)butyl alcohol, 4-(2-formyl-6-hydroxyphenoxycarbonyl)butyl alcohol, 4-(4-formyl-2-methylphenoxycarbonyl)butyl alcohol, 4-(3-chloro-4-formylphenoxycarbonyl)butyl alcohol, 4-(4-bromo-2-formylphenoxycarbonyl)butyl alcohol, 4-(2-formyl-4-methoxyphenoxycarbonyl)butyl alcohol, 4-(4-formyl-2-methoxyphenoxycarbonyl)butyl alcohol, 4-(2-formyl-4-(trifluoromethoxy) phenoxycarbonyl)butyl alcohol, 4-(4-formyl-2-nitrophenoxycarbonyl)butyl alcohol, 4-(4,6-dibromo-2-formylphenoxycarbonyl)butyl alcohol and 4-(2,5-dimethyl-4-formylphenoxycarbonyl)butyl alcohol;
ester substituted methyl alcohol compounds such as 4-methoxycarbonylphenoxycarbonylmethyl alcohol, 4-propoxycarbonylphenoxycarbonylmethyl alcohol, 4-isopropoxycarbonylphenoxycarbonylmethyl alcohol, 2-methoxycarbonylphenoxycarbonylmethyl alcohol, 2-nitro-4-methoxycarbonylphenoxycarbonylmethyl alcohol, 4-ethoxycarbonyl-2-methoxyphenoxycarbonylmethyl alcohol, 5-ethoxycarbonyl-2-hydroxyphenoxycarbonylmethyl alcohol, 3-hydroxy-2-methoxycarbonylphenoxycarbonylmethyl alcohol, 2-methoxycarbonylmethylphenoxycarbonylmethyl alcohol and 4-ethoxycarbonylethyl-2-nitrophenoxycarbonylmethyl alcohol;
ester substituted ethyl alcohol compounds such as 2-(4-methoxycarbonylphenoxycarbonyl)ethyl alcohol, 2-(4-propoxycarbonylphenoxycarbonyl)ethyl alcohol, 2-(4-isopropoxycarbonylphenoxycarbonyl)ethyl alcohol, 2-(2-methoxycarbonylphenoxycarbonyl)ethyl alcohol, 2-(2-nitro-4-methoxycarbonylphenoxycarbonyl)ethyl alcohol, 2-(4-ethoxycarbonyl-2-methoxyphenoxycarbonyl)ethyl alcohol, 2-(5-ethoxycarbonyl-2-hydroxyphenoxycarbonyl)ethyl alcohol, 2-(3-hydroxy-2 methoxycarbonylphenoxycarbonyl)ethyl alcohol, 2-(2-methoxycarbonylmethylphenoxycarbonyl)ethyl alcohol and 2-(4-ethoxycarbonylethyl-2-nitrophenoxycarbonyl)ethyl alcohol;
ester substituted propyl alcohol compounds such as 3-(4-methoxycarbonylphenoxycarbonyl)propyl alcohol, 3-(4-propoxycarbonylphenoxycarbonyl)propyl alcohol, 3-(4-isopropoxycarbonylphenoxycarbonyl)propyl alcohol, 3-(2-methoxycarbonylphenoxycarbonyl)propyl alcohol, 3-(2-nitro-4-methoxycarbonylphenoxycarbonyl)propyl alcohol, 3-(4-ethoxycarbonyl-2-methoxyphenoxycarbonyl)propyl alcohol, 3-(5-ethoxycarbonyl-2-hydroxyphenoxycarbonyl)propyl alcohol, 3-(3-hydroxy-2 methoxycarbonylphenoxycarbonyl)propyl alcohol, 3-(2-methoxycarbonylmethylphenoxycarbonyl)propyl alcohol and 3-(4-ethoxycarbonylethyl-2-nitrophenoxycarbonyl)propyl alcohol;
ester substituted butyl alcohol compounds such as 4-(4-methoxycarbonylphenoxycarbonyl)butyl alcohol, 4-(4-propoxycarbonylphenoxycarbonyl)butyl alcohol, 4-(4-isopropoxycarbonylphenoxycarbonyl)butyl alcohol, 4-(2-methoxycarbonylphenoxycarbonyl)butyl alcohol, 4-(2-nitro-4-methoxycarbonylphenoxycarbonyl)butyl alcohol, 4-(4-ethoxycarbonyl-2-methoxyphenoxycarbonyl)butyl alcohol, 4-(5-ethoxycarbonyl-2-hydroxyphenoxycarbonyl)butyl alcohol, 4-(3-hydroxy-2 methoxycarbonylphenoxycarbonyl)butyl alcohol, 4-(2-methoxycarbonylmethylphenoxycarbonyl)butyl alcohol and 4-(4-ethoxycarbonylethyl-2-nitrophenoxycarbonyl)butyl alcohol;
alkoxy substituted methyl alcohol compounds such as 2-methoxyphenoxycarbonylmethyl alcohol, 4-methoxyphenoxycarbonylmethyl alcohol, 4-ethoxyphenoxycarbonylmethyl alcohol, 1,4-dimethoxyphenoxycarbonylmethyl alcohol, 2,4-dimethoxyphenoxycarbonylmethyl alcohol, 2-chloro-5-methoxyphenoxycarbonylmethyl alcohol, 3,5-diethoxyphenoxycarbonylmethyl alcohol and 2,5-dimethoxy-3-hydroxyphenoxycarbonylmethyl alcohol;
alkoxy substituted ethyl alcohol compounds such as 2-(2-methoxyphenoxycarbonyl)ethyl alcohol, 2-(4-methoxyphenoxycarbonyl)ethyl alcohol, 2-(4-ethoxyphenoxycarbonyl)ethyl alcohol, 2-(1,4-dimethoxyphenoxycarbonyl)ethyl alcohol, 2-(2,4-dimethoxyphenoxycarbonyl)ethyl alcohol, 2-(2-chloro-5-methoxyphenoxycarbonyl)ethyl alcohol, 2-(3,5-diethoxyphenoxycarbonyl)ethyl alcohol and 2-(2,5-dimethoxy-3-hydroxyphenoxycarbonyl)ethyl alcohol;
alkoxy substituted propyl alcohol compounds such as 3-(2-methoxyphenoxycarbonyl)propyl alcohol, 3-(4-methoxyphenoxycarbonyl)propyl alcohol, 3-(4-ethoxyphenoxycarbonyl)propyl alcohol, 3-(1,4-dimethoxyphenoxycarbonyl)propyl alcohol, 3-(2,4-dimethoxyphenoxycarbonyl)propyl alcohol, 3-(2-chloro-5-methoxyphenoxycarbonyl)propyl alcohol, 3-(3,5-diethoxyphenoxycarbonyl)propyl alcohol and 3-(2,5-dimethoxy-3-hydroxyphenoxycarbonyl)propyl alcohol;
alkoxy substituted butyl alcohol compounds such as 4-(2-methoxyphenoxycarbonyl)butyl alcohol, 4-(4-methoxyphenoxycarbonyl)butyl alcohol, 4-(4-ethoxyphenoxycarbonyl)butyl alcohol, 4-(1,4-dimethoxyphenoxycarbonyl)butyl alcohol, 4-(2,4-dimethoxyphenoxycarbonyl)butyl alcohol, 4-(2-chloro-5-methoxyphenoxycarbonyl)butyl alcohol, 4-(3,5-diethoxyphenoxycarbonyl)butyl alcohol and 4-(2,5-dimethoxy-3-hydroxyphenoxycarbonyl)butyl alcohol;
hydroxy substituted methyl alcohol compounds such as 2-hydroxyphenoxycarbonylmethyl alcohol, 3-hydroxyphenoxycarbonylmethyl alcohol, 4-hydroxyphenoxycarbonylmethyl alcohol and 3,5-dihydroxyphenoxycarbonylmethyl alcohol;
hydroxy substituted ethyl alcohol compounds such as 2-(2-hydroxyphenoxycarbonyl)ethyl alcohol, 2-(3-hydroxyphenoxycarbonyl)ethyl alcohol, 2-(4-hydroxyphenoxycarbonyl)ethyl alcohol and 2-(3,5-dihydroxyphenoxycarbonyl)ethyl alcohol;
hydroxy substituted propyl alcohol compounds such as 3-(2-hydroxyphenoxycarbonyl)propyl alcohol, 3-(3-hydroxyphenoxycarbonyl)propyl alcohol, 3-(4-hydroxyphenoxycarbonyl)propyl alcohol and 3-(3,5-dihydroxyphenoxycarbonyl)propyl alcohol;
hydroxy substituted butyl alcohol compounds such as 4-(2-hydroxyphenoxycarbonyl)butyl alcohol, 4-(3-hydroxyphenoxycarbonyl)butyl alcohol, 4-(4-hydroxyphenoxycarbonyl)butyl alcohol and 4-(3,5-dihydroxyphenoxycarbonyl)butyl alcohol;
halogen substituted methyl alcohol compounds such as 4-bromophenoxycarbonylmethyl alcohol, 2,4,6-tribromophenoxycarbonylmethyl alcohol, 2,4,6-trichlorophenoxycarbonylmethyl alcohol, 4-bromo-2,6-dichlorophenoxycarbonylmethyl alcohol, 2,4-dichloro-6-iodophenoxycarbonylmethyl alcohol, 2-chloro-4,6-dibromophenoxycarbonylmethyl alcohol;
halogen substituted ethyl alcohol compounds such as 2-(4-bromophenoxycarbonyl)ethyl alcohol, 2-(2,4,6-tribromophenoxycarbonyl)ethyl alcohol, 2-(2,4,6-trichlorophenoxycarbonyl)ethyl alcohol, 2-(4-bromo-2,6-dichlorophenoxycarbonyl)ethyl alcohol, 2-(2,4-dichloro-6-iodophenoxycarbonyl)ethyl alcohol, 2-(2-chloro-4,6-dibromophenoxycarbonyl)ethyl alcohol;
halogen substituted propyl alcohol compounds such as 3-(4-bromophenoxycarbonyl)propyl alcohol, 3-(2,4,6-tribromophenoxycarbonyl)propyl alcohol, 3-(2,4,6-trichlorophenoxycarbonyl)propyl alcohol, 3-(4-bromo-2,6-dichlorophenoxycarbonyl)propyl alcohol, 3-(2,4-dichloro-6-iodophenoxycarbonyl)propyl alcohol, 3-(2-chloro-4,6-dibromophenoxycarbonyl)propyl alcohol;
halogen substituted butyl alcohol compounds such as 4-(4-bromophenoxycarbonyl)butyl alcohol, 4-(2,4,6-tribromophenoxycarbonyl)butyl alcohol, 4-(2,4,6-trichlorophenoxycarbonyl)butyl alcohol, 4-(4-bromo-2,6-dichlorophenoxycarbonyl)butyl alcohol, 4-(2,4-dichloro-6-iodophenoxycarbonyl)butyl alcohol, 4-(2-chloro-4,6-dibromophenoxycarbonyl)butyl alcohol;
substituted acetylcarbonylmethyl alcohol compounds such as trimethylacetylcarbonylmethyl alcohol, methoxyacetylcarbonylmethyl alcohol, *tert-*butylacetylcarbonylmethyl alcohol, acetoxyacetylcarbonylmethyl alcohol, bromoacetylcarbonylmethyl alcohol, chloroacetylcarbonylmethyl alcohol, cyclopentylacetylcarbonylmethyl alcohol, dichloroacetylcarbonylmethyl alcohol, tribromoacetylcarbonylmethyl alcohol, trichloroacetylcarbonylmethyl alcohol and trifluoroacetylcarbonylmethyl alcohol,
substituted acetylcarbonylethyl alcohol compounds such as 2-(trimethylacetylcarbonyl)ethyl alcohol, methoxyacetylcarbonyl)ethyl alcohol, 2-(*tert*-butylacetylcarbonyl)ethyl alcohol, 2-(acetoxyacetylcarbonyl)ethyl alcohol, 2-(bromoacetylcarbonyl)ethyl alcohol, 2-(chloroacetylcarbonyl)ethyl alcohol, 2-(cyclopentylacetylcarbonyl)ethyl alcohol, 2-(dichloroacetylcarbonyl)ethyl alcohol, 2-(tribromoacetylcarbonyl)ethyl alcohol, 2-(trichloroacetylcarbonyl)ethyl alcohol and 2-(trifluoroacetylcarbonyl)ethyl alcohol;
substituted acetylcarbonylpropyl alcohol compounds such as 3-(trimethylacetylcarbonyl)propyl alcohol, methoxyacetylcarbonyl)propyl alcohol, 3-(*tert*-butylacetylcarbonyl)propyl alcohol, 3-(acetoxyacetylcarbonyl)propyl alcohol, 3-(bromoacetylcarbonyl)propyl alcohol, 3-(chloroacetylcarbonyl)propyl alcohol, 3-(cyclopentylacetylcarbonyl)propyl alcohol, 3-(dichloroacetylcarbonyl)propyl alcohol, 3-(tribromoacetylcarbonyl)propyl alcohol, 3-(trichloroacetylcarbonyl)propyl alcohol and 3-(trifluoroacetylcarbonyl)propyl alcohol;
substituted acetylcarbonylbutyl alcohol compounds such as 4-(trimethylacetylcarbonyl)butyl alcohol, methoxyacetylcarbonyl)butyl alcohol, 4-(*tert*-butylacetylcarbonyl)butyl alcohol, 4-(acetoxyacetylcarbonyl)butyl alcohol, 4-(bromoacetylcarbonyl)butyl alcohol, 4-(chloroacetylcarbonyl)butyl alcohol, 4-(cyclopentylacetylcarbonyl)butyl alcohol, 4-(dichloroacetylcarbonyl)butyl alcohol, 4-(tribromoacetylcarbonyl)butyl alcohol, 4-(trichloroacetylcarbonyl)butyl alcohol and 4-(trifluoroacetylcarbonyl)butyl alcohol;
substituted acetylcarbonylmethyl alcohol compounds such as phenylacetylcarbonylmethyl alcohol, 2,2-diphenylacetylcarbonylmethyl alcohol, 2,2,2-triphenyl acetylcarbonylmethyl alcohol, 4-methoxyphenylacetylcarbonylmethyl alcohol, 3,4-dimethoxyphenylacetylcarbonylmethyl alcohol, 2,5-dimethoxyphenylacetylcarbonylmethyl alcohol, 2-bromophenylacetylcarbonylmethyl alcohol, 2-bromo-4,5-dimethoxyphenylacetylcarbonylmethyl alcohol, 4-chlorophenylacetylcarbonylmethyl alcohol, 2-chloro-2-phenylacetylcarbonylmethyl alcohol, 2-chloro-2,2-diphenylacetylcarbonylmethyl alcohol, 4-fluorophenylacetylcarbonylmethyl alcohol, 4-methoxyphenyl acetylcarbonylmethylalcohol, phenoxyacetylcarbonylmethyl alcohol, 4-fluorophenoxyacetylcarbonylmethyl alcohol, benzyloxyacetylcarbonylmethyl alcohol 2-phenyl -2-chloroacetylcarbonylmethylalcohol, 2,2-diphenyl -2-chloroacetylcarbonylmethylalcohol and 2-(2,4,5-trichlorophenoxy)acetylcarbonylmethyl alcohol;
substituted acetylcarbonylethyl alcohol compounds such as 2-(phenylacetylcarbonyl)ethyl alcohol, 2-(2,2-diphenylacetylcarbonyl)ethyl alcohol, 2-(2,2,2-triphenylacetylcarbonyl)ethyl alcohol, 2-(4-methoxyphenylacetylcarbonyl)ethyl alcohol, 2-(3,4-dimethoxyphenylacetylcarbonyl)ethyl alcohol, 2-(2,5-dimethoxyphenylacetylcarbonyl)ethyl alcohol, 2-(2-bromophenylacetylcarbonyl)ethyl alcohol, 2-(2-bromo-4,5-dimethoxyphenylacetylcarbonyl)ethyl alcohol, 2-(4-chlorophenylacetylcarbonyl)ethyl alcohol, 2-(2-chloro-2-phenylacetylcarbonyl)ethyl alcohol, 2-(2-chloro-2,2-diphenylacetylcarbonyl)ethyl alcohol, 2-(4-fluorophenylacetylcarbonyl)ethyl alcohol, 2-(4-methoxyphenyl acetylcarbonyl)ethylalcohol, 2-(phenoxyacetylcarbonyl)ethyl alcohol, 2-(4-fluorophenoxyacetylcarbonyl)ethyl alcohol, 2-(benzyloxyacetylcarbonyl)ethyl alcohol, 2-(2-phenyl -2-chloroacetylcarbonyl)ethylalcohol, 2-(2,2-diphenyl -2-chloroacetylcarbonyl)ethylalcohol and 2-(2-(2,4,5-trichlorophenoxy)acetylcarbonyl)ethyl alcohol;
substituted acetylcarbonylpropyl alcohol compounds such as 3-(phenylacetylcarbonyl)propyl alcohol, 3-(2,2-diphenylacetylcarbonyl)propyl alcohol, 3-(2,2,2-triphenylacetylcarbonyl)propyl alcohol, 3-(4-methoxyphenylacetylcarbonyl)propyl alcohol, 3-(3,4-dimethoxyphenylacetylcarbonyl)propyl alcohol, 3-(2,5-dimethoxyphenylacetylcarbonyl)propyl alcohol, 3-(2-bromophenylacetylcarbonyl)propyl alcohol, 3-(2-bromo-4,5-dimethoxyphenylacetylcarbonyl)propyl alcohol, 3-(4-chlorophenylacetylcarbonyl)propyl alcohol, 3-(2-chloro-2-phenylacetylcarbonyl)propyl alcohol, 3-(2-chloro-2,2-diphenylacetylcarbonyl)propyl alcohol, 3-(4-fluorophenylacetylcarbonyl)propyl alcohol, 3-(4-methoxyphenyl acetylcarbonyl)propylalcohol, 3-(phenoxyacetylcarbonyl)propyll alcohol, 3-(4-fluorophenoxyacetylcarbonyl)propyl alcohol, 3-(benzyloxyacetylcarbonyl)propyl alcohol 3-(2-phenyl -2-chloroacetylcarbonyl)propylalcohol, 3-(2,2-diphenyl -2-chloroacetylcarbonyl)propylalcohol and 3-(2-(2,4,5-trichlorophenoxy)acetylcarbonyl)propyl alcohol;
substituted acetylcarbonylbutyl alcohol compounds such as 4-(phenylacetylcarbonyl)butyl alcohol, 4-(2,2-diphenylacetylcarbonyl)butyl alcohol, 4-(2,2,2-triphenylacetylcarbonyl)butyl alcohol, 4-(4-methoxyphenylacetylcarbonyl)butyl alcohol, 4-(3,4-dimethoxyphenylacetylcarbonyl)butyl alcohol, 4-(2,5-dimethoxyphenylacetylcarbonyl)butyl alcohol, 4-(2-bromophenylacetylcarbonyl)butyl alcohol, 4-(2-bromo-4,5-dimethoxyphenylacetylcarbonyl)butyl alcohol, 4-(4-chlorophenylacetylcarbonyl)butyl alcohol, 4-(2-chloro-2-phenylacetylcarbonyl)butyl alcohol, 4-(2-chloro-2,2-diphenylacetylcarbonyl)butyl alcohol, 4-(4-fluorophenylacetylcarbonyl)butyl alcohol, 4-(4-methoxyphenyl acetylcarbonyl)butylalcohol, 4-(phenoxyacetylcarbonyl)butyl alcohol, 4-(4-fluorophenoxyacetylcarbonyl)butyl alcohol, 4-(benzyloxyacetylcarbonyl)butyl alcohol 4-(2-phenyl -2-chloroacetylcarbonyl)butylalcohol, 4-(2,2-diphenyl -2-chloroacetylcarbonyl)butylalcohol and 4-(2-(2,4,5-trichlorophenoxy)acetylcarbonyl)butyl alcohol;
substituted silyloxycarbonylmethyl alcohol compounds such as trimethylsilyloxycarbonylmethyl alcohol, triethylsilyloxycarbonylmethyl alcohol, tri-n-propylsilyloxycarbonylmethyl alcohol, triisopropylsilyloxycarbonylmethyl alcohol, tri-n-butylsilyloxycarbonylmethyl alcohol, triisobutylsilyloxycarbonylmethyl alcohol, tri-*tert*-butylsilyloxycarbonylmethyl alcohol, tri-s-butylsilyloxycarbonylmethyl alcohol, tri-n-pentylsilyloxycarbonylmethyl alcohol, triisopentylsilyloxycarbonylmethyl alcohol, tri-n-hexylsilyloxycarbonylmethyl alcohol, tri-n-octylsilyloxycarbonylmethyl alcohol, triphenylsilyloxycarbonylmethyl alcohol, tri-p-methylphenylsilyloxycarbonylmethyl alcohol, tribenzylsilyloxycarbonylmethyl alcohol, ethyldimethylsilyloxycarbonylmethyl alcohol, *n*-butyldimethylsilyloxycarbonylmethyl alcohol, diisopropylsilyloxycarbonylmethyl alcohol and t-butyldiphenylsilyloxycarbonylmethyl alcohol;
substituted silyloxycarbonylethyl alcohol compounds such as 2-(trimethylsilyloxycarbonyl)ethyl alcohol, 2-(triethylsilyloxycarbonyl)ethyl alcohol, 2-(tri-n-propylsilyloxycarbonyl)ethyl alcohol, 2-(triisopropylsilyloxycarbonyl)ethyl alcohol, 2-(tri-n-butylsilyloxycarbonyl)ethyl alcohol, 2-(triisobutylsilyloxycarbonyl)ethyl alcohol, 2-(tri-*tert*-butylsilyloxycarbonyl)ethyl alcohol, 2-(tri-s-butylsilyloxycarbonyl)ethyl alcohol, 2-(tri-n-pentylsilyloxycarbonyl)ethyl alcohol, 2-(triisopentylsilyloxycarbonyl)ethyl alcohol, 2-(tri-n-hexylsilyloxycarbonyl)ethyl alcohol, 2-(tri-n-octylsilyloxycarbonyl)ethyl alcohol, 2-(triphenylsilyloxycarbonyl)ethyl alcohol, 2-(tri-p-methylphenylsilyloxycarbonyl)ethyl alcohol, 2-(tribenzylsilyloxycarbonyl)ethyl alcohol, 2-(ethyldimethylsilyloxycarbonyl)ethyl alcohol, 2-(n-butyldimethylsilyloxycarbonyl)ethyl alcohol, 2-(diisopropylsilyloxycarbonyl)ethyl alcohol and 2-(t-butyldiphenylsilyloxycarbonyl)ethyl alcohol;
substituted silyloxycarbonylpropyl alcohol compounds such as 3-(trimethylsilyloxycarbonyl)propyl alcohol, 3-(triethylsilyloxycarbonyl)propyl alcohol, 3-(tri-n-propylsilyloxycarbonyl)propyl alcohol, 3-(triisopropylsilyloxycarbonyl)propyl alcohol, 3-(tri-n-butylsilyloxycarbonyl)propyl alcohol, 3-(triisobutylsilyloxycarbonyl)propyl alcohol, 3-(tri-*tert-*butylsilyloxycarbonyl)propyl alcohol, 3-(tri-s-butylsilyloxycarbonyl)propyl alcohol, 3-(tri-n-pentylsilyloxycarbonyl)propyl alcohol, 3-(triisopentylsilyloxycarbonyl)propyl alcohol, 3-(tri-n-hexylsilyloxycarbonyl)propyl alcohol, 3-(tri-n-octylsilyloxycarbonyl)propyl alcohol, 3-(triphenylsilyloxycarbonyl)propyl alcohol, 3-(tri-p-methylphenylsilyloxycarbonyl)propyl alcohol, 3-(tribenzylsilyloxycarbonyl)propyl alcohol, 3-(ethyldimethylsilyloxycarbonyl)propyl alcohol, 3-(n-butyldimethylsilyloxycarbonyl)propyl alcohol, 3-(diisopropylsilyloxycarbonyl)propyl alcohol and 3-(t-butyldiphenylsilyloxycarbonyl)propyl alcohol;
substituted silyloxycarbonylbutyl alcohol compounds such as 4-(trimethylsilyloxycarbonyl)butyl alcohol, 4-(triethylsilyloxycarbonyl)butyl alcohol, 4-(tri-n-propylsilyloxycarbonyl)butyl alcohol, 4-(triisopropylsilyloxycarbonyl)butyl alcohol, 4-(tri-n-butylsilyloxycarbonyl)butyl alcohol, 4-(triisobutylsilyloxycarbonyl)butyl alcohol, 4-(tri-*tert*-butylsilyloxycarbonyl)butyl alcohol, 4-(tri-s-butylsilyloxycarbonyl)butyl alcohol, 4-(tri-n-pentylsilyloxycarbonyl)butyl alcohol, 4-(triisopentylsilyloxycarbonyl)butyl alcohol, 4-(tri-n-hexylsilyloxycarbonyl)butyl alcohol, 4-(tri-n-octylsilyloxycarbonyl)butyl alcohol, 4-(triphenylsilyloxycarbonyl)butyl alcohol, 4-(tri-p-methylphenylsilyloxycarbonyl)butyl alcohol, 4-(tribenzylsilyloxycarbonyl)butyl alcohol, 4-(ethyldimethylsilyloxycarbonyl)butyl alcohol, 4-(n-butyldimethylsilyloxycarbonyl)butyl alcohol, 4-(diisopropylsilyloxycarbonyl)butyl alcohol and 4-(t-butyldiphenylsilyloxycarbonyl)butyl alcohol;
formyl substituted methyl halogens such as 3-formylphenoxycarbonylmethyl chloride, 4-formylphenoxycarbonylmethyl chloride, 2-formylphenoxycarbonylmethyl chloride, 6-fluoro-2-formylphenoxycarbonylmethyl chloride, 2-formyl-6-methoxyphenoxycarbonylmethyl chloride, 6-ethoxy-2-formylphenoxycarbonylmethyl chloride, 2-formyl-6-hydroxyphenoxycarbonylmethyl chloride, 4-formyl-2-methylphenoxycarbonylmethyl chloride, 4-formyl-3-chlorophenoxycarbonylmethyl chloride4-bromo-, 2-formylphenoxycarbonylmethyl chloride, 2-formyl-4-methoxyphenoxycarbonylmethyl chloride, 4-formyl-2-methoxyphenoxycarbonylmethyl chloride, 2-formyl-4-(trifluoromethoxy) phenoxycarbonylmethyl chloride, 4-formyl-2-nitrophenoxycarbonylmethyl chloride, 4,6-dibromo-2-formylphenoxycarbonylmethyl chloride and 4-formyl-2,5-dimethylphenoxycarbonylmethyl chloride;
formyl substituted ethyl halogens such as 2-(3-formylphenoxycarbonyl)ethyl chloride, 2-(4-formylphenoxycarbonyl)ethyl chloride, 2-(2-formylphenoxycarbonyl)ethyl chloride, 2-(6-fluoro-2-formylphenoxycarbonyl)ethyl chloride, 2-(2-formyl-6-methoxyphenoxycarbonyl)ethyl chloride, 2-(6-ethoxy-2-formylphenoxycarbonyl)ethyl chloride, 2-(2-formyl-6-hydroxyphenoxycarbonyl)ethyl chloride, 2-(4-formyl-2-methylphenoxycarbonyl)ethyl chloride, 2-(4-formyl-3-chlorophenoxycarbonyl)ethyl chloride, 2-(4-bromo-2-formylphenoxycarbonyl)ethyl chloride, 2-(2-formyl-4-methoxyphenoxycarbonyl)ethyl chloride, 2-(4-formyl-2-methoxyphenoxycarbonyl)ethyl chloride, 2-(2-formyl-4-(trifluoromethoxy)phenoxycarbonyl)ethyl chloride, 2-(4-formyl-2-nitrophenoxycarbonyl)ethyl chloride, 2-(4,6-dibromo-2-formylphenoxycarbonyl)ethyl chloride and 2-(4-formyl-2,5-dimethylphenoxycarbonyl)ethyl chloride;
formyl substituted propyl halogens such as 3-(3-formylphenoxycarbonyl)propyl chloride, 3-(4-formylphenoxycarbonyl)propyl chloride, 3-(2-formylphenoxycarbonyl)propyl chloride, 3-(6-fluoro-2-formylphenoxycarbonyl)propyl chloride, 3-(2-formyl-6-methoxyphenoxycarbonyl)propyl chloride, 3-(6-ethoxy-2-formylphenoxycarbonyl)propyl chloride, 3-(2-formyl-6-hydroxyphenoxycarbonyl)propyl chloride, 3-(4-formyl-2-methylphenoxycarbonyl)propyl chloride, 3-(4-formyl-3-chlorophenoxycarbonyl)propyl chloride, 3-(4-bromo-2-formylphenoxycarbonyl)propyl chloride, 3-(2-formyl-4-methoxyphenoxycarbonyl)propyl chloride, 3-(4-formyl-2-methoxyphenoxycarbonyl)propyl chloride, 3-(2-formyl-4-(trifluoromethoxy)phenoxycarbonyl)propyl chloride, 3-(4-formyl-2-nitrophenoxycarbonyl)propyl chloride, 3-(4,6-dibromo-2-formylphenoxycarbonyl)propyl chloride and 3-(4-formyl-2,5-dimethylphenoxycarbonyl)propyl chloride;
formyl substituted butyl halogens such as 4-(3-formylphenoxycarbonyl)butyl chloride, 4-(4-formylphenoxycarbonyl)butyl chloride, 4-(2-formylphenoxycarbonyl)butyl chloride, 4-(6-fluoro-2-formylphenoxycarbonyl)butyl chloride, 4-(2-formyl-6-methoxyphenoxycarbonyl)butyl chloride, 4-(6-ethoxy-2-formylphenoxycarbonyl)butyl chloride, 4-(2-formyl-6-hydroxyphenoxycarbonyl)butyl chloride, 4-(4-formyl-2-methylphenoxycarbonyl)butyl chloride, 4-(4-formyl-3-chlorophenoxycarbonyl)butyl chloride, 4-(4-bromo-2-formylphenoxycarbonyl)butyl chloride, 4-(2-formyl-4-methoxyphenoxycarbonyl)butyl chloride, 4-(4-formyl-2-methoxyphenoxycarbonyl)butyl chloride, 4-(2-formyl-4-(trifluoromethoxy)phenoxycarbonyl)butyl chloride, 4-(4-formyl-2-nitrophenoxycarbonyl)butyl chloride, 4-(4,6-dibromo-2-formylphenoxycarbonyl)butyl chloride and 4-(4-formyl-2,5-dimethylphenoxycarbonyl)butyl chloride;
ester substituted methyl halogens such as 4-methoxycarbonylphenoxycarbonylmethyl chloride, 4-propoxycarbonylphenoxycarbonylmethyl chloride, 4-isopropoxycarbonylphenoxycarbonylmethyl chloride, 2-methoxycarbonylphenoxycarbonylmethyl chloride, 2-nitro-4-methoxycarbonylphenoxycarbonylmethyl chloride, 4-ethoxycarbonyl-2-methoxyphenoxycarbonylmethyl chloride, 5-ethoxycarbonyl-2-hydroxyphenoxycarbonylmethyl chloride, 3-hydroxy-2-methoxycarbonylphenoxycarbonylmethyl chloride, 2-methoxycarbonylmethylphenoxycarbonylmethyl chloride and 4-ethoxycarbonylethyl-2-nitrophenoxycarbonylmethyl chloride;
ester substituted ethyl halogens such as 2-(4-methoxycarbonylphenoxycarbonyl)ethyl chloride, 2-(4-propoxycarbonylphenoxycarbonyl)ethyl chloride, 2-(4-isopropoxycarbonylphenoxycarbonyl)ethyl chloride, 2-(2-methoxycarbonylphenoxycarbonyl)ethyl chloride, 2-(2-nitro-4-methoxycarbonylphenoxycarbonyl)ethyl chloride, 2-(4-ethoxycarbonyl-2-methoxyphenoxycarbonyl)ethyl chloride, 2-(5-ethoxycarbonyl-2-hydroxyphenoxycarbonyl)ethyl chloride, 2-(3-hydroxy-2-methoxycarbonylphenoxycarbonyl)ethyl chloride, 2-(2-methoxycarbonylmethylphenoxycarbonyl)ethyl chloride and 2-(4-ethoxycarbonylethyl-2-nitrophenoxycarbonyl)ethyl chloride;
ester substituted propyl halogens such as 3-(4-methoxycarbonylphenoxycarbonyl)propyl chloride, 3-(4-propoxycarbonylphenoxycarbonyl)propyl chloride, 3-(4-isopropoxycarbonylphenoxycarbonyl)propyl chloride, 3-(2-methoxycarbonylphenoxycarbonyl)propyl chloride, 3-(2-nitro-4-methoxycarbonylphenoxycarbonyl)propyl chloride, 3-(4-ethoxycarbonyl-2-methoxyphenoxycarbonyl)propyl chloride, 3-(5-ethoxycarbonyl-2-hydroxyphenoxycarbonyl)propyl chloride, 3-(3-hydroxy-2-methoxycarbonylphenoxycarbonyl)propyl chloride, 3-(2-methoxycarbonylmethylphenoxycarbonyl)propyl chloride and 3-(4-ethoxycarbonylethyl-2-nitrophenoxycarbonyl)propyl chloride;
ester substituted butyl halogens such as 4-(4-methoxycarbonylphenoxycarbonyl)butyl chloride, 4-(4-propoxycarbonylphenoxycarbonyl)butyl chloride, 4-(4-isopropoxycarbonylphenoxycarbonyl)butyl chloride, 4-(2-methoxycarbonylphenoxycarbonyl)butyl chloride, 4-(2-nitro-4-methoxycarbonylphenoxycarbonyl)butyl chloride, 4-(4-ethoxycarbonyl-2-methoxyphenoxycarbonyl)butyl chloride, 4-(5-ethoxycarbonyl-2-hydroxyphenoxycarbonyl)butyl chloride, 4-(3-hydroxy-2-methoxycarbonylphenoxycarbonyl)butyl chloride, 4-(2-methoxycarbonylmethylphenoxycarbonyl)butyl chloride and 4-(4-ethoxycarbonylethyl-2-nitrophenoxycarbonyl)butyl chloride;
alkoxy substituted methyl halogens such as 2-methoxyphenoxycarbonylmethyl chloride, 4-methoxyphenoxycarbonylmethyl chloride, 4-ethoxyphenoxycarbonylmethyl chloride, 1,4-dimethoxyphenoxycarbonylmethyl chloride, 2,4-dimethoxyphenoxycarbonylmethyl chloride, 2-chloro-5-methoxyphenoxycarbonylmethyl chloride, 3,5-diethoxyphenoxycarbonylmethyl chloride 3-hydroxy- and 2,5-dimethoxyphenoxycarbonylmethyl chloride;
alkoxy substituted ethyl halogens such as 2-(2-methoxyphenoxycarbonyl)ethyl chloride, 2-(4-methoxyphenoxycarbonyl)ethyl chloride, 2-(4-ethoxyphenoxycarbonyl)ethyl chloride, 2-(1,4-dimethoxyphenoxycarbonyl)ethyl chloride, 2-(2,4-dimethoxyphenoxycarbonyl)ethyl chloride, 2-(2-chloro-5-methoxyphenoxycarbonyl)ethyl chloride, 2-(3,5-diethoxyphenoxycarbonyl)ethyl chloride and 2-(3-hydroxy-2,5-dimethoxyphenoxycarbonyl)ethyl chloride;
alkoxy substituted propyl halogens such as 3-(2-methoxyphenoxycarbonyl)propyl chloride, 3-(4-methoxyphenoxycarbonyl)propyl chloride, 3-(4-ethoxyphenoxycarbonyl)propyl chloride, 3-(1,4-dimethoxyphenoxycarbonyl)propyl chloride, 3-(2,4-dimethoxyphenoxycarbonyl)propyl chloride, 3-(2-chloro-5-methoxyphenoxycarbonyl)propyl chloride, 3-(3,5-diethoxyphenoxycarbonyl)propyl chloride and 3-(3-hydroxy-2,5-dimethoxyphenoxycarbonyl)propyl chloride;
alkoxy substituted butyl halogens such as 4-(2-methoxyphenoxycarbonyl)butyl chloride, 4-(4-methoxyphenoxycarbonyl)butyl chloride, 4-(4-ethoxyphenoxycarbonyl)butyl chloride, 4-(1,4-dimethoxyphenoxycarbonyl)butyl chloride, 4-(2,4-dimethoxyphenoxycarbonyl)butyl chloride, 4-(2-chloro-5-methoxyphenoxycarbonyl)butyl chloride, 4-(3,5-diethoxyphenoxycarbonyl)butyl chloride and 4-(3-hydroxy-2,5-dimethoxyphenoxycarbonyl)butyl chloride;
hydroxy substituted methyl halogens such as 2-hydroxyphenoxycarbonylmethyl chloride, 3-hydroxyphenoxycarbonylmethyl chloride, 4-hydroxyphenoxycarbonylmethyl chloride and 3,5-dihydroxyphenoxycarbonylmethyl chloride;
hydroxy substituted ethyl halogens such as 2-(2-hydroxyphenoxycarbonyl)ethyl chloride, 2-(3-hydroxyphenoxycarbonyl)ethyl chloride, 2-(4-hydroxyphenoxycarbonyl)ethyl chloride and 2-(3,5-dihydroxyphenoxycarbonyl)ethyl chloride;
hydroxy substituted propyl halogens such as 3-(2-hydroxyphenoxycarbonyl)propyl chloride, 3-(3-hydroxyphenoxycarbonyl)propyl chloride, 3-(4-hydroxyphenoxycarbonyl)propyl chloride and 3-(3,5-dihydroxyphenoxycarbonyl)propyl chloride;
hydroxy substituted butyl halogens such as 4-(2-hydroxyphenoxycarbonyl)butyl chloride, 4-(3-hydroxyphenoxycarbonyl)butyl chloride, 4-(4-hydroxyphenoxycarbonyl)butyl chloride and 4-(3,5-dihydroxyphenoxycarbonyl)butyl chloride;
halogen substituted compounds such as 4-bromophenoxycarbonylmethyl chloride, 2,4,6-tribromophenoxycarbonylmethyl chloride, 2,4,6-trichlorophenoxycarbonylmethyl chloride, 4-bromo-2,6-dichlorophenoxycarbonylmethyl chloride, 6-iodo-2,4-dichlorophenoxycarbonylmethyl chloride, 4,6-dibromo-2-chlorophenoxycarbonylmethyl chloride;
halogen substituted compounds such as 2-(4-bromophenoxycarbonyl)ethyl chloride, 2-(2,4,6-tribromophenoxycarbonyl)ethyl chloride, 2-(2,4,6-trichlorophenoxycarbonyl)ethyl chloride, 2-(4-bromo-2,6-dichlorophenoxycarbonyl)ethyl chloride, 2-(6-iodo-2,4-dichlorophenoxycarbonyl)ethyl chloride, 2-(4,6-dibromo-2-chlorophenoxycarbonyl)ethyl chloride;
halogen substituted compounds such as 3-(4-bromophenoxycarbonyl)propyl chloride, 3-(2,4,6-tribromophenoxycarbonyl)propyl chloride, 3-(2,4,6-trichlorophenoxycarbonyl)propyl chloride, 3-(4-bromo-2,6-dichlorophenoxycarbonyl)propyl chloride, 3-(6-iodo-2,4-dichlorophenoxycarbonyl)propyl chloride, 3-(4,6-dibromo-2-chlorophenoxycarbonyl)propyl chloride;
halogen substituted compounds such as 4-(4-bromophenoxycarbonyl)butyl chloride, 4-(2,4,6-tribromophenoxycarbonyl)butyl chloride, 4-(2,4,6-trichlorophenoxycarbonyl)butyl chloride, 4-(4-bromo-2,6-dichlorophenoxycarbonyl)butyl chloride, 4-(6-iodo-2,4-dichlorophenoxycarbonyl)butyl chloride, 4-(4,6-dibromo-2-chlorophenoxycarbonyl)butyl chloride;
halogen substituted acetylcarbonyl compounds such as trimethylacetylcarbonylmethyl chloride, methoxyacetylcarbonylmethyl chloride, *tert*-butylacetylcarbonylmethyl chloride, acetoxyacetylcarbonylmethyl chloride, bromoacetylcarbonylmethyl chloride, chloroacetylcarbonylmethyl chloride, cyclopentylacetylcarbonylmethyl chloride, dichloroacetylcarbonylmethyl chloride, tribromoacetylcarbonylmethyl chloride, trichloroacetylcarbonylmethyl chloride and trifluoroacetylcarbonylmethyl chloride,
halogen substituted acetylcarbonyl compounds such as 2-(trimethylacetylcarbonyl)ethyl chloride, methoxyacetylcarbonyl)ethyl chloride, 2-(*tert*-butylacetylcarbonyl)ethyl chloride, 2-(acetoxyacetylcarbonyl)ethyl chloride, 2-(bromoacetylcarbonyl)ethyl chloride, 2-(chloroacetylcarbonyl)ethyl chloride, 2-(cyclopentylacetylcarbonyl)ethyl chloride, 2-(dichloroacetylcarbonyl)ethyl chloride, 2-(tribromoacetylcarbonyl)ethyl chloride, 2-(trichloroacetylcarbonyl)ethyl chloride and 2-(trifluoroacetylcarbonyl)ethyl chloride;
halogen substituted acetylcarbonyl compounds such as 3-(trimethylacetylcarbonyl)propyl chloride, methoxyacetylcarbonyl)propyl chloride, 3-(*tert*-butylacetylcarbonyl)propyl chloride, 3-(acetoxyacetylcarbonyl)propyl chloride, 3-(bromoacetylcarbonyl)propyl chloride, 3-(chloroacetylcarbonyl)propyl chloride, 3-(cyclopentylacetylcarbonyl)propyl chloride, 3-(dichloroacetylcarbonyl)propyl chloride, 3-(tribromoacetylcarbonyl)propyl chloride, 3-(trichloroacetylcarbonyl)propyl chloride and 3-(trifluoroacetylcarbonyl)propyl chloride;
halogen substituted acetyl carbonyl compounds such as 4-(trimethylacetylcarbonyl)butyl chloride, methoxyacetylcarbonyl)butyl chloride, 4-(*tert*-butylacetylcarbonyl)butyl chloride, 4-(acetoxyacetylcarbonyl)butyl chloride, 4-(bromoacetylcarbonyl)butyl chloride, 4-(chloroacetylcarbonyl)butyl chloride, 4-(cyclopentylacetylcarbonyl)butyl chloride, 4-(dichloroacetylcarbonyl)butyl chloride, 4-(tribromoacetylcarbonyl)butyl chloride, 4-(trichloroacetylcarbonyl)butyl chloride and 4-(trifluoroacetylcarbonyl)butyl chloride;
halogen substituted acetylcarbonyl compounds such as phenylacetylcarbonylmethyl chloride, 2,2-diphenylacetylcarbonylmethyl chloride, 2,2,2-triphenylacetylcarbonylmethyl chloride, 4-methoxyphenylacetylcarbonylmethyl chloride, 3,4-dimethoxyphenylacetylcarbonylmethyl chloride, 2,5-dimethoxyphenylacetylcarbonylmethyl chloride, 2-bromophenylacetylcarbonylmethyl chloride, 2-bromo-4,5-dimethoxyphenylacetylcarbonylmethyl chloride, 4-chlorophenylacetylcarbonylmethyl chloride, 2-chloro-2-phenylacetylcarbonylmethyl chloride, 2-chloro-2,2-diphenylacetylcarbonylmethyl chloride, 4-fluorophenylacetylcarbonylmethyl chloride, 4-methoxyphenyl acetylcarbonylmethylchloride, phenoxyacetylcarbonylmethyl chloride, 4-fluorophenoxyacetylcarbonylmethyl chloride, benzyloxyacetylcarbonylmethyl chloride, 2-phenyl -2-chloroacetylcarbonylmethylchloride, 2,2-diphenyl -2-chloroacetylcarbonylmethylchloride and 2-(2,4,5-trichlorophenoxy) acetylcarbonylmethyl chloride;
halogen substituted acetylcarbonyl compounds such as 2-(phenylacetylcarbonyl)ethyl chloride, 2-(2,2-diphenylacetylcarbonyl)ethyl chloride, 2-(2,2,2-triphenylacetylcarbonyl)ethyl chloride, 2-(4-methoxyphenylacetylcarbonyl)ethyl chloride, 2-(3,4-dimethoxyphenylacetylcarbonyl)ethyl chloride, 2-(2,5-dimethoxyphenylacetylcarbonyl)ethyl chloride, 2-(2-bromophenylacetylcarbonyl)ethyl chloride, 2-(2-bromo-4,5-dimethoxyphenylacetylcarbonyl)ethyl chloride, 2-(4-chlorophenylacetylcarbonyl)ethyl chloride, 2-(2-chloro-2-phenylacetylcarbonyl)ethyl chloride, 2-(2-chloro-2,2-diphenylacetylcarbonyl)ethyl chloride, 2-(4-fluorophenylacetylcarbonyl)ethyl chloride, 2-(4-methoxyphenyl acetylcarbonyl)ethylchloride, 2-(phenoxyacetylcarbonyl)ethyl chloride, 2-(4-fluorophenoxyacetylcarbonyl)ethyl chloride, 2-(benzyloxyacetylcarbonyl)ethyl chloride, 2-(2-phenyl -2-chloroacetylcarbonyl)ethylchloride, 2-(2,2-diphenyl -2-chloroacetylcarbonyl)ethylchloride and 2-(2-(2,4,5-trichlorophenoxy) acetylcarbonyl)ethyl chloride;
halogen substituted acetylcarbonyl compounds such as 3-(phenylacetylcarbonyl)propyl chloride, 3-(2,2-diphenylacetylcarbonyl)propyl chloride, 3-(2,2,2-triphenylacetylcarbonyl)propyl chloride, 3-(4-methoxyphenylacetylcarbonyl)propyl chloride, 3-(3,4-dimethoxyphenylacetylcarbonyl)propyl chloride, 3-(2,5-dimethoxyphenylacetylcarbonyl)propyl chloride, 3-(2-bromophenylacetylcarbonyl)propyl chloride, 3-(2-bromo-4,5-dimethoxyphenylacetylcarbonyl)propyl chloride, 3-(4-chlorophenylacetylcarbonyl)propyl chloride, 3-(2-chloro-2-phenylacetylcarbonyl)propyl chloride, 3-(2-chloro-2,2-diphenylacetylcarbonyl)propyl chloride, 3-(4-fluorophenylacetylcarbonyl)propyl chloride, 3-(4-methoxyphenyl acetylcarbonyl)propylchloride, 3-(phenoxyacetylcarbonyl)propyl chloride, 3-(4-fluorophenoxyacetylcarbonyl)propyl chloride, 3-(benzyloxyacetylcarbonyl)propyl chloride 3-(2-phenyl -2-chloroacetylcarbonyl)propylchloride, 3-(2,2-diphenyl -2-chloroacetylcarbonyl)propylchloride and 3-(2-(2,4,5-trichlorophenoxy) acetylcarbonyl)propyl chloride;
halogen substituted acetylcarbonyl compounds such as 4-(phenylacetylcarbonyl)butyl chloride, 4-(2,2-diphenylacetylcarbonyl)butyl chloride, 4-(2,2,2-triphenyl acetylcarbonyl)butyl chloride, 4-(4-methoxyphenylacetylcarbonyl)butyl chloride, 4-(3,4-dimethoxyphenylacetylcarbonyl)butyl chloride, 4-(2,5-dimethoxyphenylacetylcarbonyl)butyl chloride, 4-(2-bromophenylacetylcarbonyl)butyl chloride, 4-(2-bromo-4,5-dimethoxyphenylacetylcarbonyl)butyl chloride, 4-(4-chlorophenylacetylcarbonyl)butyl chloride, 4-(2-chloro-2-phenylacetylcarbonyl)butyl chloride, 4-(2-chloro-2,2-diphenylchloroacetylcarbonyl)butyl chloride, 4-(4-fluorophenylacetylcarbonyl)butyl chloride, 4-(4-methoxyphenyl acetylcarbonyl)butylchloride, 4-(phenoxyacetylcarbonyl)butyl chloride, 4-(4-fluorophenoxyacetylcarbonyl)butyl chloride, 4-(benzyloxyacetylcarbonyl)butyl chloride, 4-(2-phenyl -2-chloroacetylcarbonyl)butylchloride, 4-(2,2-diphenyl -2-chloroacetylcarbonyl)butylchloride and 4-(2-(2,4,5-trichlorophenoxy) acetylcarbonyl)butyl chloride;
silyl substituted methyl halogens such as trimethylsilyloxycarbonylmethyl chloride, triethylsilyloxycarbonylmethyl chloride, tri-n-propylsilyloxycarbonylmethyl chloride, triisopropylsilyloxycarbonylmethyl chloride, tri-n-butylsilyloxycarbonylmethyl chloride, triisobutylsilyloxycarbonylmethyl chloride, tri-*tert-*butylsilyloxycarbonylmethyl chloride, tri-s-butylsilyloxycarbonylmethyl chloride, tri-n-pentylsilyloxycarbonylmethyl chloride, triisopentylsilyloxycarbonylmethyl chloride, tri-n-hexylsilyloxycarbonylmethyl chloride, tri-n-octylsilyloxycarbonylmethyl chloride, triphenylsilyloxycarbonylmethyl chloride, tri-p-methylphenylsilyloxycarbonylmethyl chloride, tribenzylsilyloxycarbonylmethyl chloride, ethyldimethylsilyloxycarbonylmethyl chloride, n-butyldimethylsilyloxycarbonylmethyl chloride, diisopropylsilyloxycarbonylmethyl chloride and t-butyldiphenylsilyloxycarbonylmethyl chloride;
silyl substituted ethyl halogens such as 2-(trimethylsilyloxycarbonyl)ethyl chloride, 2-(triethylsilyloxycarbonyl)ethyl chloride, 2-(tri-n-propylsilyloxycarbonyl)ethyl chloride, 2-(triisopropylsilyloxycarbonyl)ethyl chloride, 2-(tri-n-butylsilyloxycarbonyl)ethyl chloride, 2-(triisobutylsilyloxycarbonyl)ethyl chloride, 2-(tri-*tert*-butylsilyloxycarbonyl)ethyl chloride, 2-(tri-s-butylsilyloxycarbonyl)ethyl chloride, 2-(tri-n-pentylsilyloxycarbonyl)ethyl chloride, 2-(triisopentylsilyloxycarbonyl)ethyl chloride, 2-(tri-n-hexylsilyloxycarbonyl)ethyl chloride, 2-(tri-n-octylsilyloxycarbonyl)ethyl chloride, 2-(triphenylsilyloxycarbonyl)ethyl chloride, 2-(tri-p-methylphenylsilyloxycarbonyl)ethyl chloride, 2-(tribenzylsilyloxycarbonyl)ethyl chloride, 2-(ethyldimethylsilyloxycarbonyl)ethyl chloride, 2-(n-butyldimethylsilyloxycarbonyl)ethyl chloride, 2-(diisopropylsilyloxycarbonyl)ethyl chloride and 2-(t-butyldiphenylsilyloxycarbonyl)ethyl chloride;
silyl substituted propyl halogens such as 3-(trimethylsilyloxycarbonyl)propyl chloride, 3-(triethylsilyloxycarbonyl)propyl chloride, 3-(tri-n-propylsilyloxycarbonyl)propyl chloride, 3-(triisopropylsilyloxycarbonyl)propyl chloride, 3-(tri-n-butylsilyloxycarbonyl)propyl chloride, 3-(triisobutylsilyloxycarbonyl)propyl chloride, 3-(tri-*tert*-butylsilyloxycarbonyl)propyl chloride, 3-(tri-s-butylsilyloxycarbonyl)propyl chloride, 3-(tri-n-pentylsilyloxycarbonyl)propyl chloride, 3-(triisopentylsilyloxycarbonyl)propyl chloride, 3-(tri-n-hexylsilyloxycarbonyl)propyl chloride, 3-(tri-n-octylsilyloxycarbonyl)propyl chloride, 3-(triphenylsilyloxycarbonyl)propyl chloride, 3-(tri-p-methylphenylsilyloxycarbonyl)propyl chloride, 3-(tribenzylsilyloxycarbonyl)propyl chloride, 3-(ethyldimethylsilyloxycarbonyl)propyl chloride, 3-(n-butyldimethylsilyloxycarbonyl)propyl chloride, 3-(diisopropylsilyloxycarbonyl)propyl chloride and 3-(t-butyldiphenylsilyloxycarbonyl)propyl chloride;
silyl substituted butyl halogens such as 4-(trimethylsilyloxycarbonyl)butyl chloride, 4-(triethylsilyloxycarbonyl)butyl chloride, 4-(tri-n-propylsilyloxycarbonyl)butyl chloride, 4-(triisopropylsilyloxycarbonyl)butyl chloride, 4-(tri-n-butylsilyloxycarbonyl)butyl chloride, 4-(triisobutylsilyloxycarbonyl)butyl chloride, 4-(tri-*tert*-butylsilyloxycarbonyl)butyl chloride, 4-(tri-s-butylsilyloxycarbonyl)butyl chloride, 4-(tri-n-pentylsilyloxycarbonyl)butyl chloride, 4-(triisopentylsilyloxycarbonyl)butyl chloride, 4-(tri-n-hexylsilyloxycarbonyl)butyl chloride, 4-(tri-n-octylsilyloxycarbonyl)butyl chloride, 4-(triphenylsilyloxycarbonyl)butyl chloride, 4-(tri-p-methylphenylsilyloxycarbonyl)butyl chloride, 4-(tribenzylsilyloxycarbonyl)butyl chloride, 4-(ethyldimethylsilyloxycarbonyl)butyl chloride, 4-(n-butyldimethylsilyloxycarbonyl)butyl chloride, 4-(diisopropylsilyloxycarbonyl)butyl chloride and 4-(t-butyldiphenylsilyloxycarbonyl)butyl chloride.

The copolymer according to the present invention may be prepared by polymerising at least one monomer of the structure defined by I, II or IV, and at least one other vinyl polymerisable monomer by using a suitable polymerisation initiator in a suitable polymerisation method.

Suitable polymerisation methods include radical or ionic polymerisation in e.g. solution polymerisation, bulk polymerisation, semibatch polymerisation, coordination polymerisation, suspension polymerisation or emulsion polymerisation.

Polymerisation in a suitable solvent comprises the use of e.g. water; aromatic hydrocarbons such as xylene, white spirit, toluene; aliphatic hydrocarbons such as hexane and heptane; chlorinated hydrocarbons such as dichloromethane, trichloroethane, tetrachloroethane, fluorinated hydrocarbons such as difluoromethane, difluoroethane, trifluoroethane, tetrafluoroethane; fluorochloro aliphatic and aromatic hydrocarbons such as chlorofluoromethane, chlorodifluoroethane, parachlorobenzotrifluoride; ketones such as acetone, methyl ethyl ketone, methyl isobutyl ketone, cyclohexanone; esters such as methyl acetate, ethyl acetate, butyl acetate, *tert*-butyl acetate, ethylene glycol methyl ether acetate; ethers such as ethylene glycol dimethyl ether, diethylene glycol dimethyl ether, dibutyl ether, dioxane, tetrahydrofuran, alcohols such as methanol, ethanol, propanol, isopropanol, butanol, benzyl alcohol; ether alcohols such as butoxyethanol, propylene glycol monomethyl ether, amides such as N-methylpyrrolidone, dimethylformamide; or solvents such as such as dimethylsulfoxide; optionally mixtures of the above mentioned solvents.

Suitable radical initiators include peroxides such as benzoyl peroxide, di-*tert*-butyl peroxide, didecanoyl peroxide and dilauryl peroxide, *tert*-butyl peroxy-2-ethylhexaneoate, *tert*-butyl peroxypivalate, *tert*-butyl peroxydiethylacetate, *tert-*amyl peroxy-2-ethylhexaneoate and 2,5-dimethyl-2,5-di(2-ethylhexanoylperoxy)hexane and azo compounds such as 2,2'-azobis(isobutyronitrile) and 2,2'-azobis(2-methylbutyronitrile), usually in concentrations of 0.01 to 5 % by weight of the employed monomer mixture.

The copolymer of the present invention may also be prepared by polymerising at least one monomer defined by the structure I, II or IV and at least one other vinyl polymerisable monomer by using a suitable polymerisation initiator and a suitable chain transfer agent. By using chain transfer agent, a branched or star-shaped structure in which the polymer molecules have three or more polymer chains from the branching point, is obtained.

Suitable chain transfer agents include multifunctional mercaptans such as pentaerythritol tetra(3-mercaptopropionate), trimethylolpropane tri(mercaptopropionate), pentaerythritol tetra(thioglycolate), pentaerythritol tri(thioglycolate) or others as described in WO9603465.

The copolymer of the present invention which is prepared from at least one monomer defined by the structure I, II or IV, has preferably a weight average molecular weight (M_{w}) from 1000 to 200000 g/mole.

A typical antifouling paint according to the invention consists of binders and one or more other components. These binders may be the polymer described above, optionally mixtures of one or more polymers of the type described above, and other binders.

A typical antifouling paint according to the invention may also consist of the polymer described above mixed with other polymers as described in NO 2002 0846.

A typical antifouling paint according to the invention may also consist of the polymer described above in a mixture with other polymers as described in EP1127925.

It will be understood by those familiar with such formulations, that the binder phase may also contain other cobinders such as rosin, plasticisers and others as exemplified below.

Examples of cobinders comprise rosin, rosin derivatives and metal salts of rosin such as tall oil rosin, gum rosin, wood rosin, hydrogenated rosin, fumaric acid gum rosin ester, maleic acid rosin ester, polymerised rosin, dimerised rosin, zinc rosinate, copper rosinate, calcium rosinate, potassium rosinate or others as described in WO9744401 and W09615198;
other cobinders such as castor oil and derivatives of castor oil, linseed oil and derivatives of linseed oil, soybean oil and derivatives of soybean oil, coconut oil and derivatives of coconut oil;
vinyl chloride and copolymers of vinyl chloride;
styrene copolymers such as styrene/(meth)acrylate copolymers; saturated polyesters such as polyvinylacetate; alkyds and modified alkyds; hydrocarbon resins such as petroleum fraction condensates.

Examples of plasticisers include chlorinated paraffins; phthalates such as butyl phthalate and benzyl butyl phthalate, dioctyl phthalate, dinonyl phthalate and diisodecyl phthalate;
phosphate esters such as tricresyl phosphate, nonyl phenyl phosphate, octyloxy poly(ethyleneoxy) ethyl phosphate, tributoxy ethyl phosphate, isooctyl phosphate and 2-ethylhexyl diphenyl phosphate;
sulphonamides such as H-ethyl-para-toluenesulphonamide and alkyl-para-toluenesulphonamide ;
adipates such as bis(2-ethylhexyl)adipate, diisobutyl adipate and
dioctyl adipate; and others such as phosphoric acid triethyl ester, butylstearate, sorbitan trioleate and epoxydised soya (bean) oil.

The other components included in the paint may be biologically active substances, pigments and fillers, surfactants, wetting agents and dispersing agents, drying agents, activators, foam inhibitors, stabilisers, antioxidants, corrosion inhibitors, coalescent agents, thickening agents and antisettling agents, fibres and solvents.

The total binder composition will provide the paint with the necessary self-polishing and broad mechanical properties. In addition it is necessary to use different compounds to control the adherence and growth of fouling organisms. These biologically active compounds may generally be divided into two groups, viz. inorganic and organic compounds.

Inorganic biologically active compounds include different copper compounds such as oxides, salts, metals and alloys, either used alone or several in combination. Specific examples are copper oxide, copper thiocyanate, metallic copper and metallic copper-nickel. This category also includes barium metaborate.

The organic biologically active compounds include both purely organic and organometallic substances that are extensively used in the antifouling technology today or that are evaluated for this technology. These are generally used in combination with inorganic compounds to provide improved effect, but may also be used in paints that do not contain these. The most commonly used compounds of this type today are available under the commercial names Copper Omadine, Zinc Omadine/Zinc-Pyrion, Sea-Nine 211, Irgarol 1051, Preventol A4, Preventol A5, Preventol A6/Diuron, Zineb and PK. These and others are listed in the list from the European Council of the Paint, Printing Ink and Artists Colours Industry (CEPE)'s of antifouling compounds that were available on the European market in 1998 (Utilisation of more "Environmentally Friendly" Antifouling Products, EC project No96/559/3040/DEB/E2, Final Report, July 1999) and these are hereby included as reference.

Organometallic compounds include organocopper, zinc and manganese compounds.

Examples of organocopper compounds include copper pyrithione, oxine copper, copper nonylphenolsulphonate, copper naphthenate, copper acetate, copper bis(ethylenediamine)bisdodecylbenzensulphonate and copper bis(pentachlorophenolate).

Examples of organozinc compounds include zinc pyrithione, zinc dimethyldithiocarbamate, zinc ethylene bis(dithiocarbamate) and manganese ethylene bis(dithiocarbamate)polymer complexed with zinc salt. An example of organomanganese compound is manganese ethylene bis(dithiocarbamate)polymer. Examples of heterocyclic nitrogenous compounds include 2-methylthio-4-*tert*-butylamino-6-cyclopropylaminotriazine, 2,3,5,6-tetrachloro-4-(methylsulphonyl)pyridine and pyridine triphenylboran.

Examples of heterocyclic sulphur containing compounds includes 4,5-dichloro-2-octyl-3(2H)-isothiazolone, 1,2-benzisothiazoline-3-one and 2-(thiocyanomethylthio)benzothiazole.

Examples of urea derivatives include 3-(3,4-dichlorophenyl )-1,1-dimethylurea.

Examples of amides and imides of carboxylic acids, sulphonic and sulphenic acids include N-(dichlorofluoromethylthio)phthalimide, N,N-dimethyl-N-phenyl (dichlorofluoromethylthio)sulphamide and 1,1-dichloro-N-((dimethylamino)sulphonyl)-1-fluoro-N-(4-methylphenyl)methane sulphenamide.

Examples of salts or esters of carboxylic acids include 2-((3-iodo-2-propynyl)oxo)ethanolphenyl carbamate and 3-iodo-2-propynyl N-butylcarbamate.

Examples of amines include dehydroabiethylamines and coconutdimethylamine.

Examples of substituted benzene include 2,4,5,6-tetrachloro-1,3-benzenedicarbonitrile and 1-((diiodomethyl)sulphonyl)-4-methylbenzene.

Other examples may be tetraalkylphosphonium halogenides and guanidine derivatives.

When the organic and organometallic active compounds are used in combination with inorganic active compounds such as copper oxide, copper thiocyanate and metallic copper, the amount of organic and organometallic compounds become typically lower than when these are used alone.

These organic and organometallic active compounds may also be used in combination with other organic and organometallic active compounds in the absence of inorganic active compounds.

In many antifouling paints the biologically active compounds represent the majority of the pigments. In addition different compounds may be included. These compounds are designated either as pigments or as fillers depending on their refractive index.

An important property of the pigments or the fillers is the degree of water solubility. This applies particularly when considering the selfpolishing antifouling paints based on an hydrolysing binder. Pigments or fillers may be divided in those that are sensitive to water or soluble in water and those that are not sensitive to water or insoluble in water. The first category includes pigments or fillers that are biologically active as mentioned previously. Examples of these include copper oxide and copper thiocyanate. Other pigments or fillers of this category include zinc oxide, silver chloride, silver oxide, calcium fluoride, calcium hydrogenphosphate, calcium phosphate, calcium silicate, iron carbonate, iron carbonyl, magnesium hydroxide, magnesium dihydroxide, magnesium carbonate, manganese carbonate, zinc chromate, zinc carbonate, zinc sulphide. In self-polishing systems, as described herein, such substances may be used to adjust the polishing rate of the paint. These components may be used alone or in combination.

The water insoluble pigments or fillers in this group are comprehensive, and when they are used in self-polishing systems, as described in this patent, they may reduce the polishing rate of the paint. Examples of these include talc, dolomites, calcium carbonate, mica, barium sulphate, kaolin, silica, perlite, magnesium oxide, calcite and quartz powder. Other water insoluble pigments include titanium dioxide, iron oxide, graphite and carbon black. These are generally used in combination with water soluble components.

Examples of surfactants include derivatives of propylene oxide and ethylene oxide such as alkyl phenol ethylene oxide condensates; ethoxylated monoethanol amides of unsaturated fatty acids such as ethoxylated monoethanol amides of linolenic acids; and others such as sodium dodecyl sulphate, alkylphenol ethoxylates and soya lecithin.

Examples of wetting agents and dispersing agents include those that are described in detail in M. Ash and I. Ash., Handbook of Paint and Coating Raw Materials, Vol. 1, 1996, Gower Publ. Ltd., Great Britain, p. 821-823, 849-851.

Examples of drying substances include metal octoates and metal naphthenates;

Examples of activators include salicylic acid and benzyl alcohol;

Examples of foam inhibitors include silicone oils and aromatic or aliphatic mineral oils;

Examples of stabilisers to prevent degradation both due to light and heat, include HALS compounds such as 2-hydroxy-4-methoxybenzophenone, 2-(5-chloro-(2H)-benzotriazole-2-yl)-4-methyl-6-(*tert*-butyl)phenol and 2,4-di-tert-butyl-6-(5-chlorobenzotriazole-2-yl)phenol; stabilisers to prevent degradation due to moisture include molecular sieves and water absorbers;

Examples of antioxidants include butylated hydroxyanisol, butylated hydroxy toluene, propyl gallate, tocopherols, 2,5-di-*tert*-butyl-hydroquinone, L-ascorbic acid;

Examples of corrosion inhibitors include aminocarboxylates and metal salts of these, calcium silica phosphate, ammonium benzoate, barium-, calcium-, zinc and magnesium salts of alkyl naphthalene, sulphonic acids, zinc salts of cyanuric acid and 5-nitroisophthalic acids, zinc salts of dodecylnaphthalene sulphonic acid, zinc- and amine complexes of tolylpropionic acid;

Examples of coalescing agents include glycols, 2-butoxyethanol, 2,2,4-trimethyl-1,3-pentandiol monoisobutyrate, diisobutyl esters of adipic-, glutaric-and succinic acid;

Examples of thickening agents and antisettling agents include colloidal silica, hydrated aluminium silicate, aluminium tristearate, aluminium monostearate, xanthan gum, salicylic acid, crysolite, fumed silica, unsaturated and hydrogenated castor oil, organomodified clay, polyhydroxy carboxylic acid amides, modified urea and polyamide waxes.

Examples of fibres include natural and synthetic inorganic fibres such as silicone-containing fibres, metal fibres, oxide fibres, mineral fibres; natural and synthetic organic fibres such as cellulose fibres, rubber fibres, acrylic fibres; or others as described in WO0077102.

Examples of solvents includes water; aromatic hydrocarbons such as xylene, white spirit, Solvesso, toluene; aliphatic hydrocarbons such as hexane and heptane; chlorinated hydrocarbons such as dichloromethane, trichloroethane, tetrachloroethane, fluorinated hydrocarbons such as difluoromethane, difluoroethane, trifluoroethane, tetrafluoroethane; fluorochloro aliphatic and aromatic hydrocarbons such as chlorofluoromethane, chlorodifluoroethane, parachlorobenzotrifluoride; ketones such as acetone, methyl ethyl ketone, methyl isobutyl ketone, cyclohexanone; esters such as methyl acetate, ethyl acetate, butyl acetate, *tert*-butyl acetate, ethylene glycol methyl ether acetate; ethers such as ethylene glycol dimethyl ether, diethylene glycol dimethyl ether, dibutyl ether, dioxane, tetrahydrofuran, alcohols such as methanol, ethanol, propanol, isopropanol, butanol, benzyl alcohol; ether alcohols such as butoxyethanol, propylene glycol monomethyl ether, amides such as N-methylpyrrolidone, dimethylformamide or solvents such as dimethyl sulfoxide; optionally mixtures of the above mentioned solvents.

The present invention is described in more detail in the following examples.

### Example 1

### General synthesis instructions for the preparation of intermediates

To a solution of the starting compound with an OH group (100 mmole) in acetone (100 ml) cooled to 20°C, Jones' reagent (150 mmole CrO₃ in 13 ml H₂SO₄) is slowly added with stirring. After completed addition stirring is continued for further 10 hours before acetone is decanted off. The precipitate is washed with ether, and the ether is removed by means of vacuum. The aqueous phase is made basic with aqueous sodium carbonate solution, washed with diethyl ether and acidified with hydrochloric acid before extraction with ethyl acetate. The organic phase is washed with water and dried with magnesium sulphate before evaporating off the solvent by means of vacuum. The crude product may be purified by distillation or recrystallisation.

### Example 2

### General synthesis instructions for the preparation of intermediates

To a solution of the starting compounds with phenolic OH-groups (100 mmole) and with chloro substituted acid chlorides (100 mmole) in dichloromethane (100 ml), triethylamine (100 mmole) is added by means of a dropping funnel with stirring. After stirring for further 5-8 hours the precipitated salt is removed by filtration, and the organic solution is washed with water. The organic phase is dried with magnesium sulphate before the solvent is evaporated under vacuum. The crude product may be purified by distillation or recrystallisation.

### Example 3

### General synthesis instructions for the preparation of monomers

The acid functional starting compound (1 mole) is dissolved in ethyl acetate together with triethylamine (1 mole). To the mixture the desired chlorosilane compound (1 mole) is added by means of a dropping funnel with stirring. The reaction is continued at 20-80°C for 3-8 hours before the precipitated salt is removed by filtration, and the organic phase is washed once with a saturated sodium carbonate solution and twice with water before drying on magnesium sulphate, and the solvent is evaporated off with vacuum. The crude product may be purified by distillation.

### Example 4

### General synthesis instructions for the preparation of monomers

The acid functional starting compound (1 mole) is dissolved in dichloromethane together with the desired chlorosilane compound (1mole). To the mixture the triethylamine (1 mole) is added by means of a dropping funnel with stirring. The stirring is continued at room temperature for 5-8 hours before the precipitated salt is removed by filtration, and the organic phase is washed once with a saturated sodium carbonate solution and twice with water before drying on magnesium sulphate, and the solvent is removed by means of vacuum. The crude product may be purified by distillation or recrystallisation.

### Example 5

### General synthesis instructions for the preparation of monomers

To a solution of chlorinated starting compound (100 mmole) and sodium methacrylate (150 mmole) in acetonitrile, trioctylmethylamine (5 mole-%) is added and refluxed for 6-8 hours over night. The organic phase is washed with water before drying on magnesium sulphate, and the solvent is removed by means of vacuum. The crude product may be purified by distillation or recrystallisation.

### Example 6

### General synthesis instructions for the preparation of monomer

To a solution of chlorinated starting compound (100 mmole) and potassium methacrylate (150mmole) in acetonitrile, crown ether (18-crown-6, 5 mole-%) is added and refluxed for 4-8 hours. The organic phase is washed with water before drying on magnesium sulphate, and the solvent is evaporated off by means of vacuum. The crude product may be purified by distillation or recrystallisation.

### Example 7

### General synthesis instructions for the preparation of monomer

The desired acid functional compound (100 mmole) is dissolved in dichloromethane together with the desired acetyl chloride (100 mmole). To the mixture there is added triethylamine (100 mmole) by means of a dropping funnel under stirring. The stirring is continued over night at 20-50°C, before precipitated salt is filtered off, and the organic phase is washed with water, dried on magnesium sulphate, and the solvent is evaporated off by means of vacuum. The crude product may be purified by distillation or recrystallisation.

### Example 8

### General synthesis instructions for the preparation of polymers

Solvent is charged to a five-necked flask equipped with stirrer, thermometer, adding funnel and cooler, and is heated to the desired temperature, typically 60-140°C. The desired or a mixture of the desired monomers corresponding to the desired composition is added together with the desired polymerisation initiator dropwise over three hours at the reaction temperature. The polymerisation is continued for further 3 hours before cooling. The copolymer may, if desired, be purified by precipitation in hexane or water.

### Example 9

### Polymer- analogue reaction;

### Synthesis of copolymer: Methyl methacrylate / triisopropylsilyloxycarbonylmethyl methacrylate

Methyl methacrylate (400 mmole) and carboxymethyl methacrylate (100 mmole) are copolymerised according to example 8. After finished polymerisation the polymer is cooled to room temperature, and first triisopropylsilyl chloride (150 mmole) and then triethylamine (150 mmole) are added under stirring. The stirring is continued for further 12 hours at room temperature before precipitating salt is filtered off. The copolymer may be purified by precipitation in hexane or water.

A summary of synthesised intermediates and monomers is given in table 1 and table 2 respectively.

### Determination of solids in the polymers

The solid in the polymers were determined by weighing a 0.6 ± 0.1 gram sample before and after exposure at 150°C for 30 minutes.

### Determination of the molecular weight distribution in the polymers

The molecular weight distribution in the polymers was determined by using gel permeation chromatography (GPC). Tetrahydrofuran (THF) was used as solvent and polystyrene as reference.

A summary of the polymers with the composition (given in mole %) and the measured properties are given in table 3.

**Table 1 Synthesis of intermediates**

| **Intermediate No.** | **Intermediate** | **Starting compound** | **Synthesis according to example number** |
|---|---|---|---|
| I1 | Carboxymethyl methacrylate | 2-hydroxyethylmethacrylate | 1 |
| I2 | 4-methoxyphenoxycarbonylmethyl chloride | 4-methoxyphenol and chloroacetic acid chloride | 2 |
| I3 | 4-methoxyphenoxycarbonylpropyl chloride | 4-methoxyphenol and 4-chlorobutanoic acid chloride | 2 |
| I4 | 2-formylphenoxycarbonylmethyl chloride | 2-hydroxybenzaldehyde and chloroacetic acid chloride | 2 |
| I5 | 2,3-dimethylphenoxycarbonylmethyl chloride | 4-methoxyphenol and chloroacetic acid chloride | 2 |
| I6 | 2-methylacetylphenoxycarbonylmethyl chloride | methyl-4-hydroxyphenyl acetate and chloroacetic acid chloride | 2 |
| I7 | Triisopropylsilylcarbonylbutyl chloride | triisopropylchlorosilane and 4-chlorobutanoic acid | 2 |

**Table 2 Synthesis of monomers**

| **Monomer No.** | **Monomer** | **Starting compound** | **Synthesis according to example number** |
|---|---|---|---|
| M1 | 4-methoxyphenoxycarbonylmethyl methacrylate Formula I (Y = H, X = CH₃, n =1, R¹ = Ph(OMe)) | 4-methoxyphenoxycarbonylmethyl chloride and sodium methacrylate | 5 |
| M2 | 4-methoxyphenoxycarbonylmethyl methacrylate Formula I (Y = H, X = CH₃, n = 1, R¹ = Ph(OMe)) | 4-methoxyphenoxycarbonylmethyl chloride and potassium methacrylate | 6 |
| M3 | 4-methoxyphenoxycarbonylpropyl methacrylate Formula I (Y = H, X = CH₃, n = 3, R¹ = Ph(OMe)) | 4-methoxyphenoxycarbonylpropyl chloride and sodium methacrylate | 5 |
| M4 | 2-formylphenoxycarbonyllmethyl methacrylate Formula I (Y = H, X = CH₃, n =1, R¹ = Ph(CHO)) | 2-formylphenoxycarbonylmethyl chloride and sodium methacrylate | 5 |
| M5 | 2,3-dimethylphenoxycarbonyllmethyl methacrylate Formula I (Y = H, X = CH₃, n =1, R¹ = Ph(Me)₂) | 2,3-dimethylphenoxycarbonylmethyl chloride and sodium methacrylate | 5 |
| M6 | 2-methylacetylphenoxycarbonylmethyl methacrylate Formula I (Y = H, X = CH3, n = 1, R¹ = Ph(CH₂COOMe)) | 2-methylacetylphenoxycarbonylmethyl chloride and sodium methacrylate | 5 |
| M7 | triisopropylsilyloxycarbonylmethyl methacrylate Formula I (Y = H, X = CH₃, n = 1, R¹ = Si(iPr)₃) | carboxymethyl methacrylate and triisopropylchlorosilane | 3 |
| M8 | triisopropylsilyloxycarbonylmethyl methacrylate Formula I (Y = H, X = CH₃, n = 1, R¹ = Si(iPr)₃) | carboxymethyl methacrylate and triisopropylchlorosilane | 4 |
| M9 | triisopropylsilyloxycarbonylethyl acrylate Formula I (Y = X = H, n = 2, R¹ = Si(iPr)₃) | carboxyethyl acrylate and triisopropylchlorosilane | 4 |
| M10 | tributylsilyloxycarbonylmethyl methacrylate Formula I (Y = H, X = CH₃, n =1, R¹ = Si(Bu)₃) | carboxymethyl methacrylate and tributylchlorosilane | 4 |
| M11 | *tert*-butyldiphenylsilyloxycarbonylmethyl methacrylate Formula I (Y = H, X = CH₃, n = 1, R¹ = Si(tBu)(Ph)₂) | carboxymethyl methacrylate and *tert*-butyldiphenylchlorosilane | 4 |
| M12 | triisopropylsilyloxycarbonylbutyl methacrylate Formula I (Y = H, X = CH₃, n = 4, R¹ = Si(iPr)₃) | triisopropylsilylcarbonylbutyl chloride and sodium methacrylate | 5 |
| M13 | dimethyl-*tert*-butylsilyloxycarbonylmethyl methacrylate Formula I (Y = H, X = CH₃, n = 4, R¹ = Si(*t*-Bu)(Me)₂ | carboxymethyl methacrylate and dimethyl-*tert*-butylchlorosilane | 4 |
| M14 | trimethylacetoxycarbonylmethyl methacrylate Formula I (Y = H, X = CH₃, n =1, R¹ = C(O)CMe₃) | carboxymethyl methacrylate and trimethylacetyl chloride | 7 |
| M15 | benzoyloxycarbonylmethyl methacrylate Formula I (Y = H, X = CH₃, n =1, R¹ = C(O)Ph) | carboxymethyl methacrylate and phenylacetyl chloride | 7 |

**Table 3. Synthesis of polymers**

| **Polymer** | | **P1** | **P2** | **P3** | **P4** | **P5** | **P6** | **P7** | **P8** | **P9** | | | |
|---|---|---|---|---|---|---|---|---|---|---|---|---|---|
| Monomers (mole-%) | M1 | 20 | | | | | | | | | | | |
| | M2 | | 40 | | | | | | | | | | |
| | M3 | | | 25 | 25 | | | | | | | | |
| | M4 | | | | | | | | 15 | 10 | | | |
| | M5 | | | | | 25 | 25 | | | | | | |
| | M6 | | | | | | | 20 | | | | | |
| | Methoxyethyl methacrylate | 10 | 10 | 5 | 10 | 5 | 10 | 10 | 10 | 10 | | | |
| | 2-vinylpyrrolidone | | | 5 | 5 | 5 | 5 | | | 5 | | | |
| | Methyl methacrylate | 70 | 50 | 65 | 60 | 60 | 60 | 70 | 75 | 75 | | | |
| Solvent (w-%) | Xylene | 50 | 50 | 50 | 50 | 50 | 50 | 50 | 50 | 50 | | | |
| Initiator (mole%) | 2,2'-azobis(isobutyronitrile) | 1.0 | 1.0 | 1.0 | 1.0 | 1.0 | 1.0 | 1.0 | 1.0 | 1.0 | | | |
| Molecular weight (Mw, x1000) | | 30 | 25 | 44 | 30 | 54 | 20 | 31 | 30 | 65 | | | |
| Synthesis according to example number | | 8 | 8 | 8 | 8 | 8 | 8 | 8 | 8 | 8 | | | |

| **Polymer** | | **P10** | **P11** | **P12** | **P13** | **P14** | **P15** | **P16** | **P17** | **P18** | **P19** | **P20** | **P21** |
|---|---|---|---|---|---|---|---|---|---|---|---|---|---|
| Monomers (mole-%) | I1 | | | 20 | | | | | | | | | |
| | M7 | 15 | | | | | | | | | | | |
| | M8 | | 15 | | | | | | | | | | |
| | M9 | | | | 20 | 30 | | | | | | | |
| | M10 | | | | | | 12,5 | 12,5 | | | | | |
| | M11 | | | | | | | | 15 | 20 | | | |
| | M12 | | | | | | | | | | 30 | | |
| | M13 | | | | | | | | | | | 15 | 20 |
| | Methoxyethyl methacrylate | 5 | 10 | | 10 | 10 | 5 | 10 | 10 | 10 | 10 | 5 | 5 |
| | Methyl methacrylate | 80 | 75 | 80 | 70 | 60 | 82.5 | 77.5 | 75 | 70 | 60 | 80 | 75 |
| Solvent (w-%) | Xylene | 50 | 50 | 50 | 50 | 50 | 50 | 50 | 50 | 50 | 50 | 50 | 50 |
| Initiator (mole%) | 2,2'-azobis(isobutyronitrile) | 1.0 | 1.0 | 1.0 | 1.0 | 1.0 | 1.0 | 1.0 | 1.0 | 1.0 | 1.0 | 1.0 | 1.0 |
| Molecular weight (Mw, x1000) | | 19 | 13 | - | 14 | 16 | 21 | 22 | 18 | 21 | 17 | 26 | 27 |
| *Synthesis according to Example number* | | 8 | 8 | 9 | 8 | 8 | 8 | 8 | 8 | 8 | 8 | 8 | 8 |

| **Polymer** | | **P22** | **P23** | **P24** | **P25** | | | | | | | | |
|---|---|---|---|---|---|---|---|---|---|---|---|---|---|
| Monomers (mole-%) | M14 | 5 | 2,5 | | | | | | | | | | |
| | M15 | | | 5 | | | | | | | | | |
| | Methoxyethyl methacrylate | 10 | 10 | 10 | | | | | | | | | |
| | n-Butyl methacrylate | | | | 100 | | | | | | | | |
| | Methyl methacrylate | 85 | 87,5 | 85 | | | | | | | | | |
| Solvent (w-%) | Xylene | 50 | 50 | | | | | | | | | | |
| | | | | 0 | 0 | | | | | | | | |
| Initiator (mole%) | 2,2'-azobis(isobutyronitrile) | 2.0 | 2.0 | 2.0 | 2.5 | | | | | | | | |
| Molecular weight (Mw, x1000) | | 7 | 7 | 8 | 53 | | | | | | | | |
| Synthesis according to Example number | | 8 | 8 | 8 | 8 | | | | | | | | |

### Example 10

### General preparation of antifouling paint

A polymer according to the invention, a mixture of polymers according to the invention or polymers according to the invention together with other polymers or binders are mixed together with solvent in a "dissolver". The pigments are added under stirring, and the paint is ground to a fineness < 30 µm. The thickening agent (BYK-410) and any biologically active compounds are added after grinding.

Table 4 presents a summary of paint formulations with amounts given I weight %.

**Table 4. Paint formulations**

| **Example/** Ingredient | **MF1** | **MF2** | **MF3** | **MF4** | **MF5** | **MF6** | **MF7** | **MF8** | **MF9** |
|---|---|---|---|---|---|---|---|---|---|
| Polymer P1 | 56.4 | | | | | | | | |
| Polymer P2 | | 56.4 | | | | | | | |
| Polymer P3 | | | 56.4 | | | | | | |
| Polymer P4 | | | | 56.4 | | | | | |
| Polymer P5 | | | | | 56.4 | | | | |
| Polymer P6 | | | | | | 56.4 | | | |
| Polymer P7 | | | | | | | 56.4 | | |
| Polymer P8 | | | | | | | | 56.4 | |
| Polymer P9 | | | | | | | | | 56.4 |
| Copper thiocyanate | 37.5 | 37.5 | 37.5 | 37.5 | 37.5 | 37.5 | 37.5 | 37.5 | 37.5 |
| Iron oxide | | | | | | | | | |
| Dolomite | | | | | | | | | |
| Zinc oxide | | | | | | | | | |
| Copper oxide | | | | | | | | | |
| BIK-410 | 0.5 | 0.5 | 0.5 | 0.5 | 0.5 | 0.5 | 0.5 | 0.5 | 0.5 |
| Xylene | 5.6 | 5.6 | 5.6 | 5.6 | 5.6 | 5.6 | 5.6 | 5.6 | 5.6 |

| **Example/** Ingredient | **MF10** | **MF11** | **MF12** | **MF13** | **MF14** | **MF15** | **MF16** | **MF17** | |
|---|---|---|---|---|---|---|---|---|---|
| Polymer P10 | 33.3 | | | | | | | | |
| Polymer P11 | | 33.3 | | | | | | | |
| Polymer P12 | | | 33.3 | | | | | | |
| Polymer P13 | | | | 33.3 | | | | | |
| Polymer P14 | | | | | 33.3 | | | | |
| Polymer P15 | | | | | | 33.3 | | | |
| Polymer P16 | | | | | | | 33.3 | | |
| Polymer P17 | | | | | | | | 33.3 | |
| Copper thiocyanate | | | | | | | | | |
| Iron oxide | 2.6 | 2.6 | 2.6 | 2.6 | 2.6 | 2.6 | 2.6 | 2.6 | |
| Dolomite | 1.7 | 1.7 | 1.7 | 1.7 | 1.7 | 1.7 | 1.7 | 1.7 | |
| Zinc oxide | 28.8 | 28.8 | 28.8 | 28.8 | 28.8 | 28.8 | 28.8 | 28.8 | |
| Copper oxide | 29.8 | 29.8 | 29.8 | 29.8 | 29.8 | 29.8 | 29.8 | 29.8 | |
| BYK-410 | 0.5 | 0.5 | 0.5 | 0.5 | 0.5 | 0.5 | 0.5 | 0.5 | |
| Xylene | 3.3 | 3.3 | 3.3 | 3.3 | 3.3 | 3.3 | 3.3 | 3.3 | |

| **Example/** Ingredient | **MF18** | **MF19** | **MF20** | **MF21** | **MF22** | **MF23** | **MF24** | **MF25** | |
|---|---|---|---|---|---|---|---|---|---|
| Polymer P18 | 33.3 | | | | | | | | |
| Polymer P19 | | 33.3 | | | | | | | |
| Polymer P20 | | | 33.3 | | | | | | |
| Polymer P21 | | | | 33.3 | | | | | |
| Polymer P22 | | | | | 56.4 | | | | |
| Polymer P23 | | | | | | 56.4 | | | |
| Polymer P24 | | | | | | | 56.4 | | |
| Polymer P25 | | | | | | | | 56.4 | |
| Copper thiocyanate | | | | | 37.5 | 37.5 | 37.5 | 37.5 | |
| Iron oxide | 2.6 | 2.6 | 2.6 | 2.6 | | | | | |
| Dolomite | 1.7 | 1.7 | 1.7 | 1.7 | | | | | |
| Zinc oxide | 28.8 | 28.8 | 28.8 | 28.8 | | | | | |
| Copper oxide | 29.8 | 29.8 | 29.8 | 29.8 | | | | | |
| BYK-410 | 0.5 | 0.5 | 0.5 | 0.5 | 0.5 | 0.5 | 0.5 | 0.5 | |
| Xylene | 3.3 | 3.3 | 3.3 | 3.3 | 5.6 | 5.6 | 5.6 | 5.6 | |

### Determination of polishing rates in sea water

The polishing rate is determined by measuring the reduction in film thickness of a paint formulation over time. For this test round PVC panels are used. The panels are degreased, and the paint formulations are applied as radial stripes on the panel. After application the films are dried for at least 24 hours. The thickness of the dry films are measured by means of a suitable electronic film thickness gauge. The PVC panels are mounted on a shaft which rotates in a container in which seawater is flowing through. Natural seawater which has been filtered and temperature-adjusted to 25±2°C, is used. The PVC panels are taken out at regular intervals for measuring the film thickness. The panels are then rinsed with fresh water and allowed to dry at room temperature over night. The film thickness is measured, and the panels are placed back in seawater and rotated.

The polishing results for the antifouling paints are given in table 7. The table illustrates that the antifouling paints according to the invention will provide a satisfactory polishing in seawater, which is the basis for the antifouling properties.

**Table 7. Polishing of paint films on a spinning disk in seawater.**

| **Paint formulation** | **Polishing rate (µm/year)** |
|---|---|
| MF1 | 30 |
| MF2 | 31 |
| MF3 | 26 |
| MF4 | 29 |
| MF5 | 25 |
| MF6 | 26 |
| MF7 | 38 |
| MF8 | 16 |
| MF9 | 25 |
| MF10 | 44 |
| MF11 | 36 |
| MF12 | 60 |
| MF13 | 99 |
| MF 14 | 109 |
| MF15 | 42 |
| MF16 | 45 |
| MF17 | 9 |
| MF18 | 16 |
| MF19 | 55 |
| MF20 | >100 |
| MF21 | >100 |
| MF22 | 18 |
| MF23 | 23 |
| MF24 | 6 |
| MF25 | 0 |

### Determination of antifouling performance in seawater

The antifouling performance of formulations with polymers according to the invention against marine organisms was tested by static exposure tests in the outer Oslofjord (Sandefjord, Norway).

For this test test panels of polyvinyl chloride, PVC, (150 x 75 x 3 mm) which in advance had been treated with a commercial primer, were used. Said antifouling paints were applied to the panels with a thickness corresponding to a dry film of 100-150 µm. After the antifouling paint had been applied, the panels were dried for at least 48 hours before they were mounted in a frame and submerged in seawater at a depth of 1 metre for four months. After the exposure in seawater had been terminated, the paints were evaluated with respect to their antifouling performance, i.e. how much slime, algae and animals were found on the test panels. The antifouling performance is rated from 0 to 100, where 100 means no fouling on the test panel, while 0 means that the test panel has been completely fouled.

The results from the determination of the antifouling performance in seawater are given in table 8. The table illustrates that the test panels, where the antifouling paints according to the invention had been applied, exhibited an antifouling performance similar to the reference paint with a commercial polymer and were not fouled by animals and algae during this period. The test panels with a reference paint (MF25) without the compounds of the invention demonstrated considerable fouling of algae and mussel.

**Table 8. Results from testing of antifouling performance by static testing in seawater in Sandefjord, Norway.**

| **Paint formulation** | **Antifouling performance** |
|---|---|
| MF1 | 97 |
| MF2 | 97 |
| MF3 | 95 |
| MF4 | 95 |
| MF5 | 95 |
| MF6 | 95 |
| MF7 | 95 |
| MF8 | 95 |
| MF9 | 95 |
| MF10 | 100 |
| MF11 | 100 |
| MF12 | 100 |
| MF13 | 100 |
| MF 14 | 100 |
| MF15 | 100 |
| MF16 | 100 |
| MF17 | 100 |
| MF18 | 100 |
| MF19 | 100 |
| MF20 | 100 |
| MF21 | 100 |
| MF22 | 95 |
| MF23 | 97 |
| MF24 | 97 |
| MF25 | 20 |
| Commercial product | 100 |

## Claims

1. Water insoluble, water erodable, film forming polymer which includes repeating units corresponding to the monomers A and B:
A) monomers of the formula 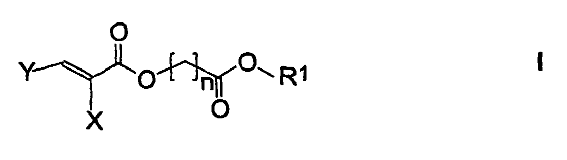 wherein
n is an integer from 1 to 4,
X is H or CH₃,
Y is H or an optionally esterified COOH group,
R¹ is Ph(R²)ₘ, -C(O)R³ or -Si(R⁴)₃,
m is an integer from 1 to 3,
R², which are the same or different, are selected from -C(O)H, C(O)R⁵, COOR⁶,
CH₂COOR⁷, C₁₋₄ alkyl, C₁₋₄ alkanoyl, halogen, nitro, OH and OR⁸,
R³ is selected from substituted or unsubstituted alkyl, aryl, aralkyl and heterocyclyl,
R⁴, which are the same or different, are selected from substituted or unsubstituted C₁₋₂₀ alkyl, C₅₋₂₀ aryl, C₁₋₂₀ alkoxy and C₅₋₂₀ aryloxy, and
R⁵, R⁶, R⁷ and R⁸ are independently selected from substituted or unsubstituted C₁₋₂₀ alkyl and C₅₋₂₀ aryl; and
B) other vinyl polymerisable monomers.

2. Polymer according to claim 1 wherein units of monomers A are present in an amount of 2-95 mole-%, and units of monomer B are present in an amount of 5-98 mole-%.

3. Polymer according to claim 1 wherein units of monomers A are present in an amount of 5-80 mole-%, and units of monomer B are present in an amount of 20-95 mole-%.

4. Polymer according to claim 1 wherein units of monomers A are present in an amount of 10-70 mole-%, and units of monomer B are present in an amount of 30-90 mole-%.

5. Polymer according to any of claims 1-4 wherein R¹ is Si(R⁴)₃.

6. Polymer according to claim 5 wherein the R⁴ groups are the same or different and are independently selected from substituted or unsubstituted C₁₋₁₂ alkyl, C₅₋₁₂ aryl, C₁₋₁₂ alkoxy and C₅₋₁₂ aryloxy.

7. Polymer according to claim 6 wherein the R⁴ groups are the same or different and are independently selected from substituted or unsubstituted C₁₋₈ alkyl, C₅₋₈ aryl, C₁₋₈ alkoxy and C₅₋₈ aryloxy.

8. Polymer according to claim 5 wherein Si(R⁴)₃ is selected from the group consisting of trimethylsilyl, triethylsilyl, tri-n-propylsilyl, triisopropylsilyl, tri-n-butylsilyl, tri-*tert*-butylsilyl, triisobutylsityl, tri-n-pentylsilyl, tri-n-hexylsilyl, triphenylsilyl, tribenzylsilyl, ethyldimethylsilyl, *n-*butyldimethylsilyl, diisopropyl-n-butylsilyl, dicyclohexylphenylsilyl, t-butyldiphenylsilyl, t-butyldimethylsilyl, dimethylphenylsilyl, biphenyldimethylsilyl, biphenyldiisopropylsilyl, diphenylmethylsilyl, dimethylhexylsilyl.

9. Polymer according to any of claims 1-4 wherein R¹ is Ph(R²)m.

10. Polymer according to claim 9, wherein R² is selected from C(O)R⁵, COOR⁶, CH₂COOR⁷ and OR⁸.

11. Polymer according to claim 10, wherein R⁵, R⁶, R⁷ and R⁸ are independently selected from unsubstituted or substituted C₁₋₁₂ alkyl and C₅₋₁₂ aryl.

12. Polymer according to claim 11, wherein R⁵, R⁶, R⁷ and R⁸ are independently selected from unsubstituted or substituted C₁₋₈ alkyl and C₅₋₁₂ aryl.

13. Polymer according to any of claims 11 and 12, wherein R⁵, R⁶, R⁷ and/or R⁸ are substituted with halogen.

14. Polymer according to claim 9, wherein the R² groups are the same or different and are independently selected from formyl, C₁₋₄ alkoxy and halogen.

15. Polymer according to claim 14, wherein the R² groups are the same or different and are independently selected from formyl, methoxy and bromo.

16. Polymer according to any of claims 1-4, wherein R¹ is -C(O)R³.

17. Polymer according to claim 16, wherein R³ is phenyl or C₁₋₆ alkyl, which may be substituted with phenoxy or phenyl, wherein said phenyl-, alkyl- and phenoxy group may be substituted with halogen, C₁₋₄ alkoxy, C₄₋₆ cycloalkyl or C₁₋₄ alkanoyloxy.

18. Polymer according to claim 17, wherein the R³ group is phenyl, benzyl, *tert*-butyl or *tert*-amyl.

19. Monomers of the formula 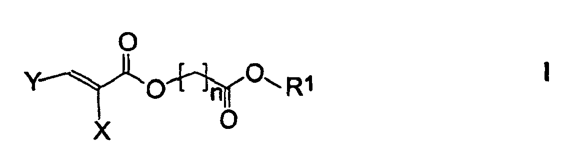 wherein
n is an integer from 1 to 4,
X is H or CH₃,
Y is H or an optionally esterified COOH group,
R¹ is Ph(R²)ₘ, -C(O)R³ or -Si(R⁴)₃,
m is an integer from 1 to 3,
R², which are the same or different, are selected from -C(O)H, C(O)R⁵, COOR⁶, CH₂COOR⁷, C₁₋₄ alkyl, C₁₋₄ alkanoyl, halogen, nitro, OH and OR⁸,
R³ is selected from substituted or unsubstituted alkyl, aryl, aralkyl and heterocyclyl,
R⁴, which are the same or different, are selected from substituted or unsubstituted C₁₋₂₀ alkyl, C₅₋₂₀ aryl, C₁₋₂₀ alkoxy and C₅₋₂₀ aryloxy, with the exception that R4 may not be unsubstituted C1-alkyl, and
R⁵, R⁶, F⁷ and R⁸ are independently selected from substituted or unsubstituted C₁₋₂₀ alkyl and C₅₋₂₀ aryl.

20. Monomer according to claim 19, wherein R¹ is -Si(R⁴)₃, in which R⁴ is as defined in any of claims 6-8.

21. Monomer according to claim 19, wherein R¹ is Ph(R²)ₘ, in which R² is as defined in any of claims 10-15.

22. Monomer according to claim 19, wherein R¹ is -C(O)R³, in which R³ is as defined in any of claims 17 and 18.

23. Process for preparing polymers by homopolymerisation or copolymerisation of polymerisable monomers,
**characterised by** polymerisation of a mixture of
A) 2-95 mole-% of one or more monomers of the formula 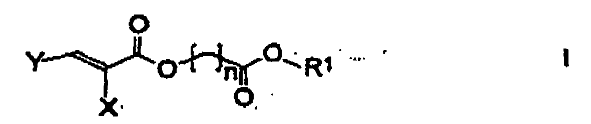 wherein
n is an integer from 1 to 4,
X is H or CH₃,
Y is H or an optionally esterified COOH group,
R¹ is Ph(R²)ₘ, -C(O)R³ or -Si(R⁴)₃,
m is an integer from 1 to 3,
R², which are the same or different, are selected from -C(O)H, C(O)R⁵, COOR⁶,
CH₂COOR⁷, C₁₋₄ alkyl, C₁₋₄ alkanoyl, halogen, nitro, OH and OR⁸,
R³ is selected from substituted or unsubstituted alkyl, aryl, aralkyl and heterocyclyl,
R⁴, which are the same or different, are selected from substituted or unsubstituted C₁₋₂₀ alkyl, C₅₋₂₀ aryl, C₁₋₂₀ alkoxy and C₅₋₂₀ aryloxy, and
R⁵, R⁶, R⁷ and R⁸ are independently selected from substituted or unsubstituted C₁₋₂₀ alkyl and C₅₋₂₀ aryl; and
B) 5-98 mole-% of other vinyl-polymerisable monomers.
in the presence of radical- or ion-forming initiators and inert solvents.

24. Process for preparing polymers by homopolymerisation or copolymerisation of polymerisable monomers,
**characterised by** polymerisation of a mixture of
C) 2-95 mole-% of one or more monomers of the formula 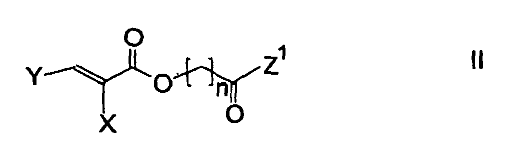 and
D) 5-98 mole-% of other vinyl polymerisable monomers,
in the presence of radical or ion-forming initiators and inert solvents,
and then, in a polymer analogous reaction, reacting the obtained copolymers with one or more compounds of the general formula
R¹-Z² III
wherein
n is an integer from 1 to 4,
X is H or CH₃,
Y is H or an optionally esterified COOH group,
Z¹ is Cl or OH,
Z² are groups which are reactive with Z¹, such as OH or Cl,
R¹ is Ph(R²)ₘ, -C(O)R³ or -Si(R⁴)₃,
m is an integer from 1 to 3,
R², which are the same or different, are selected from -C(O)H, C(O)R⁵, COOR⁶, CH₂COOR⁷, C₁₋₄ alkyl, C₁₋₄ alkanoyl, halogen, nitro, OH and OR⁸,
R³ is selected from substituted or unsubstituted alkyl, aryl, aralkyl and heterocyclyl,
R⁴, which are the same or different, are selected from substituted or unsubstituted C₁₋₂₀ alkyl, C₅₋₂₀ aryl, C₁₋₂₀ alkoxy and C₅₋₂₀ aryloxy, and
R⁵, R⁶, R⁷ and R⁸ are independently selected from substituted or unsubstituted C₁₋₂₀ alkyl and C₅₋₂₀ aryl.

25. Process for the preparation of polymers for the preparation of polymers by homopolymerisation or copolymerisation of polymerisable monomers,
**characterised by** polymerisation of a mixture of
E) 2-95 mole-% of one or more monomers of the formula 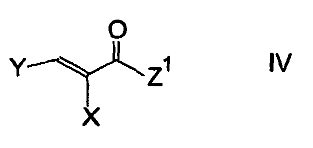 and
F) 5-98 mole-% of other vinyl polymerisable monomers,
in the presence of radical- or ion-forming initiators,
and in a polymer-analogous reaction, reacting the obtained copolymers with one or more compounds of the general formula 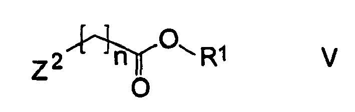
wherein
n is an integer from 1 to 4,
X is H or CH₃,
Y is H or an optionally esterified COOH group,
Z¹ is Cl or OH,
Z² are groups which are reactive with Z¹, such as OH or Cl,
R¹ is Ph(R²)ₘ, -C(O)R³ or -Si(R⁴)₃,
m is an integer from 1 to 3,
R², which are the same or different, are selected from -C(O)H, C(O)R⁵, COOR⁶, CH₂COOR⁷, C₁₋₄ alkyl, C₁₋₄ alkanoyl, halogen, nitro, OH and OR⁸,
R³ is selected from substituted or unsubstituted alkyl, aryl, aralkyl and heterocyclyl,
R⁴, which are the same or different, are selected from substituted or unsubstituted C₁₋₂₀ alkyl, C₅₋₂₀ aryl, C₁₋₂₀ alkoxy and C₅₋₂₀ aryloxy, and
R⁵, R⁶, R⁷ and R⁸ are independently selected from substituted or unsubstituted C₁₋₂₀ alkyl and C₅₋₂₀ aryl.

26. Antifouling paint containing a polymer according to any of claims 1-18, and in addition a biocide and optionally other components that are suitable in a marine paint.

27. The use of a polymer according to any of claims 1-18 as a component in a marine paint to obtain a coating having selfpolishing properties in water.

28. The use of monomers of the general formula 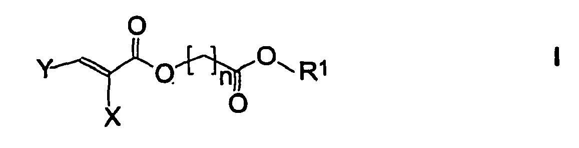 wherein
n is an integer from 1 to 4,
X is H or CH₃,
Y is H or an optionally esterified COOH group,
R¹ is Ph(R²)ₘ, -C(O)R³ or -Si(R⁴)₃,
m is an integer from 1 to 3,
R², which are the same or different, are selected from -C(O)H, C(O)R⁵, COOR⁶, CH₂COOR⁷, C₁₋₄ alkyl, C₁₋₄ alkanoyl, halogen, nitro, OH and OR⁸,
R³ is selected from substituted or unsubstituted alkyl, aryl, aralkyl and heterocyclyl,
R⁴, which are the same or different, are selected from substituted or unsubstituted C₁₋₂₀ alkyl, C₅₋₂₀ aryl, C₁₋₂₀ alkoxy and C₅₋₂₀ aryloxy, and
R⁵, R⁶, R⁷ and R⁸ are independently selected from substituted or unsubstituted C₁₋₂₀ alkyl and C₅₋₂₀ aryl;
for the preparation of polymers for antifouling paint.

29. Marine construction provided with a selfpolishing antifouling coating of the paint according to claim 26.

## Patentansprüche

1. Wasserunlösliches, mit Wasser erodierbares, Film-bildendes Polymer, welches sich wiederholende Einheiten umfasst, die den Monomeren A und B entsprechen:
A) Monomere der Formel 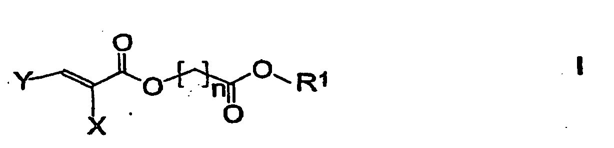 wobei
n eine ganze Zahl von 1 bis 4 ist,
X H oder CH₃ ist,
Y H oder eine gegebenenfalls veresterte COOH-Gruppe ist,
R¹ Ph(R²)ₘ, -C(O)R³ oder -Si(R⁴)₃ ist,
m eine ganze Zahl von 1 bis 3 ist,
R², welche gleich oder unterschiedlich sind, ausgewählt sind aus -C(O)H, C(O)R⁵, COOR⁶,
CH₂COOR⁷, C₁₋₄-Alkyl, C₁₋₄-Alkanoyl, Halogen, Nitro, OH und OR⁸,
R³ ausgewählt ist aus substituiertem oder unsubstituiertem Alkyl, Aryl, Aralkyl und Heterocyclyl,
R⁴, welche gleich oder unterschiedlich sind, ausgewählt sind aus substituiertem oder unsubstituiertem C₁₋₂₀-Alkyl, C₅₋₂₀-Aryl, C₁₋₂₀-Alkoxy und C₅₋₂₀-Aryloxy und
R⁵, R⁶, R⁷ und R⁸ unabhängig voneinander ausgewählt sind aus substituiertem oder unsubstituiertem C₁₋₂₀-Alkyl und C₅₋₂₀-Aryl; und
B) weitere polymerisierbare Vinyl-Monomere.

2. Polymer nach Anspruch 1, wobei Einheiten von Monomeren A in einer Menge von 2-95 Mol-% vorliegen und Einheiten von Monomer B in einer Menge von 5-98 Mol-% vorliegen.

3. Polymer nach Anspruch 1, wobei Einheiten von Monomeren A in einer Menge von 5-80 Mol-% vorliegen und Einheiten von Monomer B in einer Menge von 20-95 Mol-% vorliegen.

4. Polymer nach Anspruch 1, wobei Einheiten von Monomeren A in einer Menge von 10-70 Mol-% vorliegen und Einheiten von Monomer B in einer Menge von 30-90 Mol-% vorliegen.

5. Polymer nach einem der Ansprüche 1-4, wobei R¹ Si(R⁴)₃ ist.

6. Polymer nach Anspruch 5, wobei die R⁴-Gruppen gleich oder unterschiedlich sind und unabhängig voneinander ausgewählt sind aus substituiertem oder unsubstituiertem C₁₋₁₂-Alkyl, C₅₋₁₂-Aryl, C₁₋₁₂Alkoxy und C₅₋₁₂-Aryloxy.

7. Polymer nach Anspruch 6, wobei die R⁴-Gruppen gleich oder unterschiedlich sind und unabhängig voneinander voneinander ausgewählt sind aus substituiertem oder unsubstituiertem C₁₋₈-Alkyl, C₅₋₈-Aryl, C₁₋₈-Alkoxy und C₅₋₈-Aryloxy.

8. Polymer nach Anspruch 5, wobei Si(R⁴)₃, ausgewählt ist aus der Gruppe bestehend aus Trimethylsilyl, Triethylsilyl, Tri-n-propylsilyl, Triisopropylsilyl, Tri-n-butylsilyl, Tri-*tert*-butylsilyl, Triisobutylsilyl, Tri-n-pentylsilyl, Tri-n-hexylsilyl, Triphenylsilyl, Tribenzylsilyl, Ethyldimethylsilyl, n-Butyldimethylsilyl, Diisopropyl-n-butylsilyl, Dicyclohexylphenylsilyl, t-Butyldiphenylsilyl, t-Butyldimethylsilyl, Dimethylphenylsilyl, Biphenyldimethylsilyl, Biphenyldiisopropylsilyl, Diphenylmethylsilyl, Dimethylhexylsilyl.

9. Polymer nach einem der Ansprüche 1-4, wobei R¹ Ph(R²)ₘ ist.

10. Polymer nach Anspruch 9, wobei R² ausgewählt ist aus C(O)R⁵, COOR⁶, CH₂COOR⁷ und OR⁸.

11. Polymer nach Anspruch 10, wobei R⁵, R⁶, R⁷ und R⁸ unabhängig voneinander ausgewählt sind aus unsubstituiertem oder substituiertem C₁₋₁₂ -Alkyl und C₅₋₁₂-Aryl.

12. Polymer nach Anspruch 11, wobei R⁵, R⁶, R⁷ und R⁸ unabhängig voneinander ausgewählt sind aus unsubstituiertem oder substituiertem C₁₋₈-Alkyl und C₅₋₁₂-Aryl.

13. Polymer nach einem der Ansprüche 11 und 12, wobei R⁵, R⁶, R⁷ und/oder R⁸ mit Halogen substituiert sind.

14. Polymer nach Anspruch 9, wobei die R²-Gruppen gleich oder unterschiedlich sind und unabhängig voneinander ausgewählt sind aus Formyl, C₁₋₄-Alkoxy und Halogen.

15. Polymer nach Anspruch 14, wobei die R²-Gruppen gleich oder unterschiedlich sind und unabhängig voneinander ausgewählt sind aus Formyl, Methoxy und Brom.

16. Polymer nach einem der Ansprüche 1-4, wobei R¹ -C(O)R³ ist.

17. Polymer nach Anspruch 16, wobei R³ Phenyl oder C₁₋₆-Alkyl ist, welche mit Phenoxy oder Phenyl substituiert sein können, wobei die Phenyl-, Alkyl-und Phenoxy-Gruppe mit Halogen, C₁₋₄-Alkoxy, C₄₋₆-Cycloalkyl oder C₁₋₄-Alkanoyloxy substituiert sein kann.

18. Polymer nach Anspruch 17, wobei die R³-Gruppe Phenyl, Benzyl, *tert*-Butyl oder *tert*-Amyl ist.

19. Monomere der Formel 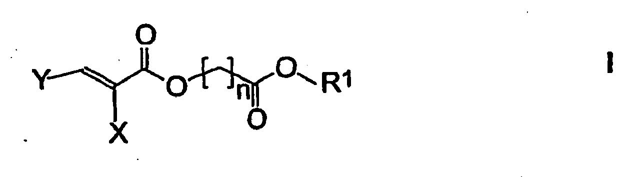 wobei
n eine ganze Zahl von 1 bis 4 ist,
X H oder CH₃ ist,
Y H oder eine gegebenenfalls veresterte COOH-Gruppe ist,
R¹ Ph(R²)ₘ, -C(O)R³ oder -Si(R⁴)₃ ist,
m eine ganze Zahl von 1 bis 3 ist,
R², welche gleich oder unterschiedlich sind, ausgewählt sind aus -C(O)H, C (O)R⁵, COOR⁶, CH₂COOR⁷, C₁₋₄-Alkyl, C₁₋₄-Alkanoyl, Halogen, Nitro, OH und OR⁸,
R³ ausgewählt ist aus substituiertem oder unsubstituiertem Alkyl, Aryl, Aralkyl und Heterocyclyl,
R⁴, welche gleich oder unterschiedlich sind, ausgewählt sind aus substituiertem oder unsubstituiertem C₁₋₂₀-Alkyl, C₅₋₂₀-Aryl, C₁₋₂₀-Alkoxy und C₅₋₂₀-Aryloxy, mit der Ausnahme, dass R⁴ nicht unsubstituiertes C₁-Alkyl sein kann, und .
R⁵, R⁸, R⁷ und R⁸ unabhängig voneinander ausgewählt sind aus substituiertem oder unsubstituiertem C₁₋₂₀-Alkyl und C₅₋₂₀-Aryl.

20. Monomer nach Anspruch 19, wobei R¹ -Si(R⁴)₃ ist, in welchem R⁴ wie in einem der Ansprüche 6-8 definiert ist.

21. Monomer nach Anspruch 19, wobei R¹ Ph(R²)ₘ ist, in welchem R² wie in einem der Ansprüche 10-15 definiert ist. TR[müsste es hier nicht heißen "... in welchem R² ...?

22. Monomer nach Anspruch 19, wobei R¹ -C(O)R³ ist, in welchem R³ wie in einem der Ansprüche 17 und 18 definiert ist.

23. Verfahren zur Herstellung von Polymeren mittels Homopolymerisation oder Copolymerisation von polymerisierbaren Monomeren,
**gekennzeichnet durch** Polymerisation eines Gemisches von
A) 2-95 Mol-% eines oder mehrerer Monomere der Formel 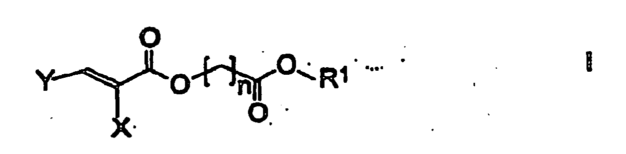 wobei
n eine ganze Zahl von 1 bis 4 ist,
X H oder CH₃ ist,
Y H oder eine gegebenenfalls veresterte COOH-Gruppe ist,
R¹ Ph(R²)ₘ, -C(O)R³ oder -Si(R⁴)₃ ist,
m eine ganze Zahl von 1 bis 3 ist,
R², welche gleich oder unterschiedlich sind, ausgewählt sind aus -C(O)H, C (O)R⁵, COOR⁶, CH₂COOR⁷, C₁₋₄-Alkyl, C₁₋₄-Alkanoyl, Halogen, Nitro, OH und OR⁸,
R³ ausgewählt ist aus substituiertem oder unsubstituiertem Alkyl, Aryl, Aralkyl und Heterocyclyl,
R⁴, welche gleich oder unterschiedlich sind, ausgewählt sind aus substituiertem oder unsubstituiertem C₁₋₂₀-Alkyl, C₅₋₂₀-Aryl, C₁₋₂₀-Alkoxy und C₅₋₂₀-Aryloxy, und
R⁵, R⁶, R⁷. und R⁸ unabhängig voneinander ausgewählt sind aus substituiertem oder unsubstituiertem C₁₋₂₀-Alkyl und C₅₋₂₀-Aryl; und
B) 5-98 Mol-% weiterer polymerisierbarer Vinyl-Monomere.
in Gegenwart von Radikal- oder Ionen-bildenden Initiatoren und inerten Lösungsmitteln.

24. Verfahren zur Herstellung von Polymeren mittels Homopolymerisation oder Copolymerisation von polymerisierbaren Monomeren,
**gekennzeichnet durch** Polymerisation eines Gemisches von
C) 2-95 Mol-% eines oder mehrerer Monomere der Formel 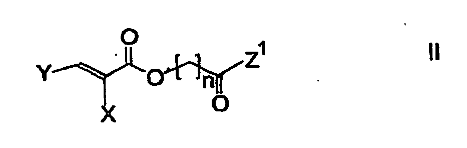 und
D) 5-98 Mol-% weiterer polymerisierbarer Vinyl-Monomere,
in Gegenwart von Radikal- oder Ionen-bildenden Initiatoren und inerten Lösungsmitteln,
und anschließend, in einer polymeranalogen Reaktion, Umsetzen der erhaltenen Copolymere mit einer oder mehreren Verbindungen der allgemeinen Formel
**R¹-Z²** **III**
wobei
n eine ganze Zahl von 1 bis 4 ist,
X H oder CH₃ ist,
Y H oder eine gegebenenfalls veresterte COOH-Gruppe ist,
Z¹ Cl oder OH ist,
**Z²** Gruppen sind, welche mit Z¹ reaktionsfähig sind, wie zum Beispiel OH oder Cl,
R¹ Ph(R²)ₘ, -C(O)R³ oder -Si(R⁴)₃ ist,
m eine ganze Zahl von 1 bis 3 ist,
R², welche gleich oder unterschiedlich sind, ausgewählt sind aus -C(O)H, C(O)R⁵, COOR⁸,
CH₂COOR⁷, C₁₋₄-Alkyl, C₁₋₄-Alkanoyl, Halogen, Nitro, OH und OR⁸,
R³ ausgewählt ist aus substituiertem oder unsubstituiertem Alkyl, Aryl, Aralkyl und Heterocyclyl,
R⁴, welche gleich oder unterschiedlich sind, ausgewählt sind aus substituiertem oder unsubstituiertem C₁₋₂₀-Alkyl, C₅₋₂₀-Aryl, C₁₋₂₀-Alkoxy und C₅₋₂₀-Aryloxy, und
R⁵, R⁶, R⁷ und R⁸ unabhängig voneinander ausgewählt sind aus substituiertem oder unsubstituiertem C₁₋₂₀-Alkyl und C₅₋₂₀-Aryl.

25. Verfahren zur Herstellung von Polymeren mittels Homopolymerisation oder Copolymerisation von polymerisierbaren Monomeren,
**gekennzeichnet durch** Polymerisation eines Gemisches von
E) 2-95 Mol-% eines oder mehrerer Monomere der Formel IV 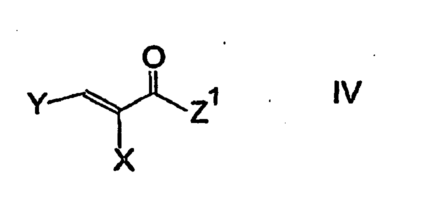 und
F) 5-98 Mol-% weiterer polymerisierbarer Vinyl-Monomere,
in Gegenwart von Radikal- oder Ionen-bildenden Initiatoren,
und in einer polymeranalogen Reaktion Umsetzen der erhaltenen Copolymere mit einer oder mehrerer Verbindungen der allgemeinen Formel 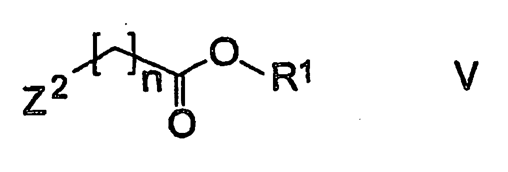
wobei
n eine ganze Zahl von 1 bis 4 ist,
X H oder CH₃ ist,
Y H oder eine gegebenenfalls veresterte COOH-Gruppe ist,
Z¹ Cl oder OH ist,
Z² Gruppen sind, welche mit Z¹ reaktionsfähig sind, wie zum Beispiel OH oder Cl,
R¹ Ph(R²)ₘ, -C(O)R³ oder -Si(R⁴)₃ ist,
m eine ganze Zahl von 1 bis 3 ist,
R², welche gleich oder unterschiedlich sind, ausgewählt sind aus -C(O)H,
C(O)R⁵, COOR⁸, CH₂COOR⁷, C₁₋₄-Alkyl, C₁₋₄-Alkanoyl, Halogen, Nitro, OH und OR⁸,
R³ ausgewählt ist aus substituiertem oder unsubstituiertem Alkyl, Aryl, Aralkyl und Heterocyclyl,
R⁴, welche gleich oder unterschiedlich sind, ausgewählt sind aus substituiertem oder unsubstituiertem C₁₋₂₀-Alkyl, C₅₋₂₀-Aryl, C₁₋₂₀-Alkoxy und C₅₋₂₀-Aryloxy, und
R⁵, R⁶, R⁷ und R⁸ unabhängig voneinander ausgewählt sind aus substituiertem oder unsubstituiertem C₁₋₂₀-Alkyl und C₅₋₂₀-Aryl.

26. Antifouling-Farbe, die ein Polymer nach einem der Ansprüche 1-18 enthält, sowie zusätzlich ein Biozid und gegebenenfalls weitere Komponenten, die für eine Schiffsfarbe geeignet sind.

27. Verwendung eines Polymers nach einem der Ansprüche 1-18 als Komponente in einer Schiffsfarbe, um eine Beschichtung zu erhalten, die in Wasser selbstpolierende Eigenschaften aufweist.

28. Verwendung von Monomeren der allgemeinen Formel 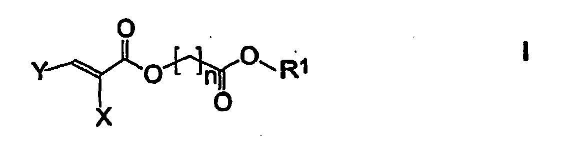 wobei
n eine ganze Zahl von 1 bis 4 ist,
X H oder CH₃ ist,
Y H oder eine gegebenenfalls veresterte COOH-Gruppe ist,
R¹ Ph(R²)ₘ, -C(O)R³ oder -Si(R⁴)₃ ist,
m eine ganze Zahl von 1 bis 3 ist,
R², welche gleich oder unterschiedlich sind, ausgewählt sind aus -C(O)H,
C(O)R⁵, COOR⁶, CH₂COOR⁷, C₁₋₄-Alkyl, C₁₋₄-Alkanoyl, Halogen, Nitro, OH und OR⁸,
R³ ausgewählt ist aus substituiertem oder unsubstituiertem Alkyl, Aryl, Aralkyl und Heterocyclyl,
R⁴, welche gleich oder unterschiedlich sind, ausgewählt sind aus substituiertem oder unsubstituiertem C₁₋₂₀-Alkyl, C₅₋₂₀-Aryl, C₁₋₂₀-Alkoxy und C₅₋₂₀-Aryloxy, und
R⁵, R⁶, R⁷ und R⁸ unabhängig voneinander ausgewählt sind aus substituiertem oder unsubstituiertem C₁₋₂₀-Alkyl und C₅₋₂₀-Aryl;
zur Herstellung von Polymeren für Antifouling-Farben.

29. Marine Konstruktion, welche mit einer selbstpolierenden Antifouling-Beschichtung der Farbe nach Anspruch 26 ausgestattet ist.

## Revendications

1. Polymère filmogène insoluble dans l'eau et érodable par l'eau, qui comprend des motifs répétés correspondant aux monomères A et B :
A) monomères de formule 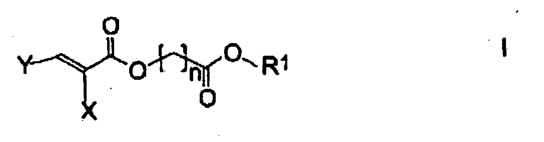 dans laquelle
n représente un nombre entier de 1 à 4,
X représente H ou un groupe CH₃,
Y représente H ou un groupe COOH facultativement estérifié,
R¹ représente un groupe Ph(R²)ₘ, -C(O)R³ ou -Si(R⁴)₃,
m représente un nombre entier de 1 à 3,
les groupes R², qui sont identiques ou différents, sont choisis entre des groupes -C(O)H, C(O)R⁵, COOR⁶, CH₂COOR⁷, alkyle en C₁ à C₄, alcanoyle en C₁ à C₄, halogéno, nitro, OH et OR⁸,
R³ est choisi entre des groupes alkyle, aryle, aralkyle et hétérocyclyle, substitués ou non substitués,
les groupes R⁴, qui sont identiques ou différents, sont choisis entre des groupes alkyle en C₁ à C₂₀, aryle en C₅ à C₂₀, alkoxy en C₁ à C₂₀ et aryloxy en C₅ à C₂₀, substitués ou non substitués, et
R⁵, R⁶, R⁷ et R⁸ sont choisis indépendamment entre des groupes alkyle en C₁ à C₂₀ et aryle en C₅ à C₂₀ substitués ou non substitués ; et
B) d'autres monomères polymérisables vinyliques.

2. Polymère suivant la revendication 1, dans lequel les motifs de monomères A sont présents en une quantité de 2 à 95 % en moles et les motifs du monomère B sont présents en une quantité de 5 à 98 % en moles.

3. Polymère suivant la revendication 1, dans lequel les motifs de monomères A sont présents en une quantité de 5 à 80 % en moles et les motifs du monomère B sont présents en une quantité de 20 à 95 % en moles.

4. Polymère suivant la revendication 1, dans lequel les motifs de monomères A sont présents en une quantité de 10 à 70 % en moles et les motifs du monomère B sont présents en une quantité de 30 à 90 % en moles.

5. Polymère suivant l'une quelconque des revendications 1 à 4, dans lequel R¹ représente un groupe Si(R⁴)₃.

6. Polymère suivant la revendication 5, dans lequel les groupes R⁴ sont identiques ou différents et sont choisis indépendamment entre des groupes alkyle en C₁ à C₁₂, aryle en C₅ à C₁₂, alkoxy en C₁ à C₁₂ et aryloxy en C₅ à C₁₂, substitués ou non substitués.

7. Polymère suivant la revendication 6, dans lequel les groupes R⁴ sont identiques ou différents et sont choisis indépendamment entre des groupes alkyle en C₁ à C₈, aryle en C₅ à C₈, alkoxy en C₁ à C₈ et aryloxy en C₅ à C₈, substitués ou non substitués.

8. Polymère suivant la revendication 5, dans lequel Si(R⁴)₃ est choisi dans le groupe consistant en des groupes triméthylsilyle, triéthylsilyle, tri-n-propylsilyle, triisopropylsilyle, tri-n-butylsilyle, tri-tertiobutylsilyle, triisobutylsilyle, tri-n-pentylsilyle, tri-n-hexylsilyle, triphénylsilyle, tribenzylsilyle, éthyldiméthylsilyle, n-butyldiméthylsilyle, diisopropyl-n-butylsilyle, dicyclohexylphénylsilyle, tertiobutyldiphénylsilyle, tertiobutyldiméthylsilyle, diméthylphénylsilyle, biphényldiméthylsilyle, biphényldiisopropylsilyle, diphénylméthylsilyle, diméthylhexylsilyle.

9. Polymère suivant l'une quelconque des revendications 1 à 4, dans lequel R¹ représente un groupe Ph(R²)ₘ.

10. Polymère suivant la revendication 9, dans lequel R² est choisi entre des groupes C(O)R⁵, COOR⁶, CH₂COOR⁷ et OR⁸.

11. Polymère suivant la revendication 10, dans lequel R⁵, R⁶, R⁷ et R⁸ sont choisis indépendamment entre des groupes alkyle en C₁ à C₁₂ et aryle en C₅ à C₁₂, non substitués ou substitués.

12. Polymère suivant la revendication 11, dans lequel R⁵, R⁶, R⁷ et R⁸ sont choisis indépendamment entre des groupes alkyle en C₁ à C₈ et aryle en C₅ à C₁₂, non substitués ou substitués.

13. Polymère suivant l'une quelconque des revendications 11 et 12, dans lequel R⁵, R⁶, R⁷ et/ou R⁸ sont substitués avec des substituants halogéno.

14. Polymère suivant la revendication 9, dans lequel les groupes R² sont identiques ou différents et sont choisis indépendamment entre des groupes formyle, alkoxy en C₁ à C₄ et halogéno.

15. Polymère suivant la revendication 14, dans lequel les groupes R² sont identiques ou différents et sont choisis indépendamment entre des groupes formyle, méthoxy et bromo.

16. Polymère suivant l'une quelconque des revendications 1 à 4, dans lequel R¹ représente un groupe -C(O)R³.

17. Polymère suivant la revendication 16, dans lequel R³ représente un groupe phényle ou alkyle en C₁ à C₆, qui peut être substitué avec un substituant phénoxy ou phényle, dans lequel lesdits groupes phényle, alkyle et phénoxy peuvent être substitués avec des substituants halogéno, alkoxy en C₁ à C₄, cycloalkyle en C₄ à C₆ ou alcanoyloxy en C₁ à C₄.

18. Polymère suivant la revendication 17, dans lequel le groupe R³ est un groupe phényle, benzyle ou tertioamyle.

19. Monomères de formule 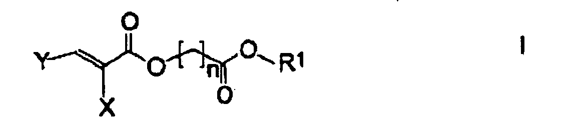 dans laquelle
n représente un nombre entier de 1 à 4,
X représente H ou un groupe CH₃,
Y représente H ou un groupe COOH facultativement estérifié,
R¹ représente un groupe Ph(R²)ₘ, -C(O)R³ ou -Si(R⁴)₃,
m représente un nombre entier de 1 à 3,
les groupes R², qui sont identiques ou différents, sont choisis entre des groupes -C(O)H, C(O)R⁵, COOR⁶, CH₂COOR⁷, alkyle en C₁ à C₄, alcanoyle en C₁ à C₄, halogéno, nitro, OH et OR⁸,
R³ est choisi entre des groupes alkyle, aryle, aralkyle et hétérocyclyle, substitués ou non substitués,
les groupes R⁴, qui sont identiques ou différents, sont choisis entre des groupes alkyle en C₁ à C₂₀, aryle en C₅ à C₂₀, alkoxy en C₁ à C₂₀ et aryloxy en C₅ à C₂₀, substitués ou non substitués, sauf que R⁴ ne peut pas représenter un groupe alkyle en C₁ non substitué, et
R⁵, R⁶, R⁷ et R⁸ sont choisis indépendamment entre des groupes alkyle en C₁ à C₂₀ et aryle en C₅ à C₂₀ substitués ou non substitués

20. Monomère suivant la revendication 19, dans lequel R¹ représente un groupe -Si(R⁴)₃, dans lequel R⁴ est tel que défini dans l'une quelconque des revendications 6 à 8.

21. Monomère suivant la revendication 19, dans lequel R¹ représente un groupe -Ph(R²)ₘ, et dans lequel R² est tel que défini dans l'une quelconque des revendications 10 à 15.

22. Monomère suivant la revendication 19, dans lequel R¹ représente un groupe -C(O)R³, dans lequel R³ est tel que défini dans l'une quelconque des revendications 17 et 18.

23. Procédé pour la préparation de polymères par homopolymérisation ou copolymérisation de monomères polymérisables, **caractérisé par** la polymérisation d'un mélange
A) de 2 à 95 % en moles d'un ou plusieurs monomères de formule 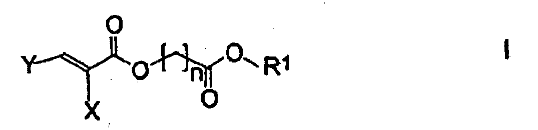 dans laquelle
n représente un nombre entier de 1 à 4,
X représente H ou un groupe CH₃,
Y représente H ou un groupe COOH facultativement estérifié,
R¹ représente un groupe Ph(R²)ₘ, -C(O)R³ ou -Si(R⁴)₃,
m représente un nombre entier de 1 à 3,
les groupes R², qui sont identiques ou différents, sont choisis entre des groupes -C(O)H, C(O)R⁵, COOR⁶, CH₂COOR⁷, alkyle en C₁ à C₄, alcanoyle en C₁ à C₄, halogéno, nitro, OH et OR⁸,
R³ est choisi entre des groupes alkyle, aryle, aralkyle et hétérocyclyle, substitués ou non substitués,
les groupes R⁴, qui sont identiques ou différents, sont choisis entre des groupes alkyle en C₁ à C₂₀, aryle en C₅ à C₂₀, alkoxy en C₁ à C₂₀ et aryloxy en C₅ à C₂₀, substitués ou non substitués, et
R⁵, R⁶, R⁷ et R⁸ sont choisis indépendamment entre des groupes alkyle en C₁ à C₂₀ et aryle en C₅ à C₂₀ substitués ou non substitués ; et
B) de 5 à 98 % en moles d'autres monomères à polymérisation vinylique,
en présence d'initiateurs radicalaires ou de formation d'ions et de solvants inertes.

24. Procédé pour la préparation de polymères par homopolymérisation ou copolymérisation de monomères polymérisables, **caractérisé par** la polymérisation d'un mélange
C) de 2 à 95 % en moles d'un ou plusieurs monomères de formule 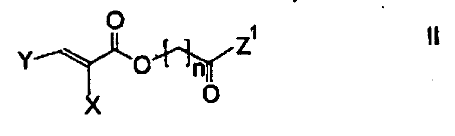 et
D) de 5 à 98 % en moles d'autres monomères à polymérisation vinylique,
en présence d'initiateurs radicalaires ou de formation d'ions et de solvants inertes,
et ensuite, dans une réaction analogue à la formation de polymère, la réaction des copolymères obtenus avec un ou plusieurs composés de formule générale
**R¹-Z²** **III**
dans laquelle
n représente un nombre entier de 1 à 4,
X représente H ou un groupe CH₃,
Y représente H ou un groupe COOH facultativement estérifié,
Z¹ représente un groupe Cl ou OH,
les groupes Z² sont des groupes qui sont réactifs avec Z¹, tels que des groupes OH ou Cl,
R¹ représente un groupe Ph(R²)ₘ, -C(O)R³ ou -Si(R⁴)₃,
m représente un nombre entier de 1 à 3,
les groupes R², qui sont identiques ou différents, sont choisis entre des groupes -C(O)H, C(O)R⁵, COOR⁶, CH₂COOR⁷, alkyle en C₁ à C₄, alcanoyle en C₁ à C₄, halogéno, nitro, OH et OR⁸,
R³ est choisi entre des groupes alkyle, aryle, aralkyle et hétérocyclyle, substitués ou non substitués,
les groupes R⁴, qui sont identiques ou différents, sont choisis entre des groupes alkyle en C₁ à C₂₀, aryle en C₅ à C₂₀, alkoxy en C₁ à C₂₀ et aryloxy en C₅ à C₂₀, substitués ou non substitués, et
R⁵, R⁶, R⁷ et R⁸ sont choisis indépendamment entre des groupes alkyle en C₁ à C₂₀ et aryle en C₅ à C₂₀ substitués ou non substitués.

25. procédé pour la préparation de polymères par homopolymérisation ou copolymérisation de monomères polymérisables, **caractérisé par** la polymérisation d'un mélange
E) de 2 à 95 % en moles d'un ou plusieurs monomères de formule 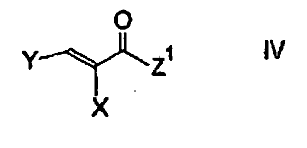 et
F) de 5 à 98 % en moles d'autres monomères à polymérisation vinylique
en présence d'initiateurs radicalaires ou de formation d'ions,
et, dans une réaction analogue à la formation d'un polymère, la réaction des copolymères obtenus avec un ou plusieurs composés de formule générale 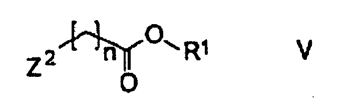
dans laquelle
n représente un nombre entier de 1 à 4,
X représente H ou un groupe CH₃,
Y représente H ou un groupe COOH facultativement estérifié,
Z¹ représente un groupe Cl ou OH,
les groupes Z² sont des groupes qui sont réactifs avec Z¹, tels que des groupes OH ou Cl,
R¹ représente un groupe Ph(R²)ₘ, -C(O)R³ ou -Si(R⁴)₃,
m représente un nombre entier de 1 à 3,
les groupes R², qui sont identiques ou différents, sont choisis entre des groupes -C(O)H, C(O)R⁵, COOR⁶, CH₂COOR⁷, alkyle en C₁ à C₄, alcanoyle en C₁ à C₄, halogéno, nitro, OH et OR⁸,
R³ est choisi entre des groupes alkyle, aryle, aralkyle et hétérocyclyle, substitués ou non substitués,
les groupes R⁴, qui sont identiques ou différents, sont choisis entre des groupes alkyle en C₁ à C₂₀, aryle en C₅ à C₂₀, alkoxy en C₁ à C₂₀ et aryloxy en C₅ à C₂₀, substitués ou non substitués, et
R⁵, R⁶, R⁷ et R⁸ sont choisis indépendamment entre des groupes alkyle en C₁ à C₂₀ et aryle en C₅ à C₂₀ substitués ou non substitués.

26. Peinture anti-salissures, contenant un polymère suivant l'une quelconque des revendications 1 à 18 et, en outre, un biocide et facultativement d'autres constituants qui conviennent dans une peinture marine.

27. Utilisation d'un polymère suivant l'une quelconque des revendications 1 à 18 comme constituant dans une peinture marine pour obtenir un revêtement ayant des propriétés d'auto-polissage dans l'eau.

28. Utilisation de monomères de formule générale 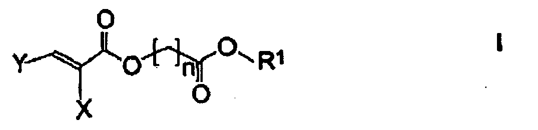 dans laquelle
n représente un nombre entier de 1 à 4,
X représente H ou un groupe CH₃,
Y représente H ou un groupe COOH facultativement estérifié,
R¹ représente un groupe Ph(R²)ₘ, -C(O)R³ ou -Si(R⁴)₃,
m représente un nombre entier de 1 à 3,
les groupes R², qui sont identiques ou différents, sont choisis entre des groupes -C(O)H, C(O)R⁵, COOR⁶, CH₂COOR⁷, alkyle en C₁ à C₄, alcanoyle en C₁ à C₄, halogéno, nitro, OH et OR⁸,
R³ est choisi entre des groupes alkyle, aryle, aralkyle et hétérocyclyle, substitués ou non substitués,
les groupes R⁴, qui sont identiques ou différents, sont choisis entre des groupes alkyle en C₁ à C₂₀, aryle en C₅ à C₂₀, alkoxy en C₁ à C₂₀ et aryloxy en C₅ à C₂₀, substitués ou non substitués, et
R⁵, R⁶, R⁷ et R⁸ sont choisis indépendamment entre des groupes alkyle en C₁ à C₂₀ et aryle en C₅ à C₂₀ substitués ou non substitués ;
pour la préparation de polymères pour une peinture anti-salissures.

29. Construction marine munie d'un revêtement anti-salissures, doué d'auto-polissage, de la peinture suivant la revendication 26.
